# EUROPEAN PATENT APPLICATION

(11) **EP 4 056 563 A1**
(43) Date of publication of application: **14.09.2022**
(21) Application number: 20885361.4
(22) Date of filing: 06.11.2020
(51) Int. Cl.: C07D 401/14, C07D 471/20, C07D 487/04, C07D 491/113, C07D 513/10, C07D 519/00, A61K 31/438, A61K 31/496, A61K 31/506, A61K 31/519, A61P 35/00

(54) **COMPOUND AS SHP2 INHIBITOR AND USE THEREOF**

(30) Priority: 08.11.2019 CN 201911089365
(71) Applicant: Nanjing Sanhome Pharmaceutical Co., Ltd., Nanjing Jiangsu 210038 (CN)
(72) Inventor: ZHAO, Liwen, Nanjing, Jiangsu 210038 (CN); WANG, Xiaowei, Nanjing, Jiangsu 210038 (CN); QUAN, Xu, Nanjing, Jiangsu 210038 (CN); YANG, Zhishuai, Nanjing, Jiangsu 210038 (CN); WANG, Yazhou, Nanjing, Jiangsu 210038 (CN); XU, Tao, Nanjing, Jiangsu 210038 (CN); WANG, Mingxiao, Nanjing, Jiangsu 210038 (CN); LIANG, Cheng, Nanjing, Jiangsu 210038 (CN); GUO, Jing, Nanjing, Jiangsu 210038 (CN); TANG, Zhichao, Nanjing, Jiangsu 210038 (CN); WANG, Chunmeng, Nanjing, Jiangsu 210038 (CN); LUO, Haizhou, Nanjing, Jiangsu 210038 (CN); LI, Xue, Nanjing, Jiangsu 210038 (CN); XU, Zheng, Nanjing, Jiangsu 210038 (CN); SUN, Tianwen, Nanjing, Jiangsu 210038 (CN)
(74) Representative: ABG Intellectual Property Law, S.L.
(86) International application number: PCT/CN2020/126916
(87) International publication number: WO 2021/088945

(57) **Abstract**

The present invention falls within the field of medical chemistry and relates to a class of compounds as SHP2 inhibitors and the use thereof. Specifically, the present invention provides a compound represented by formula (I), or an isomer, a pharmaceutically acceptable salt, a solvate, a crystal or a prodrug thereof, a preparation method therefor, a pharmaceutical composition comprising the compounds, and the use of the compounds or the composition for treating a disease mediated by SHP2.

## Description

### FIELD

The present disclosure belongs to the field of medicinal chemistry, and specifically relates to a compound as SHP2 inhibitor or an isomer, a pharmaceutically acceptable salt, a solvate, a crystal or a prodrug thereof, their preparation method, a pharmaceutical composition comprising these compounds, and use of these compounds or composition for the treatment of an SHP2 mediated disease.

### BACKGROUND

SHP2 phosphatase is a non-receptor PTP (protein tyrosine phosphatase) encoded by the PTPN11 gene, and contains two N-terminal SRC (sarcoma gene) homology domains (SH2), a PTP domain and a C-terminal tail. X-ray diffraction results indicates that SHP2 interacts with the PTP domain through the N-terminal SH2 and blocks the entry of ATP into the catalytic site, and the kinase is in an autoinhibited configuration. Some small peptides or proteins that can bind to the SH2 domain can activate the phosphorylation of the enzyme and promote the development of cancer. In cells, the function of SHP2 is related to receptor tyrosine kinases downstream of the cytoplasm, including RAS-ERK, PI3K-AKT and JAK-STAT. First, SHP2 can bind to RAS and dephosphorylate RAS, thereby increasing the effect of effector protein RAF and activating the RAS/ERK/MAPK signaling pathway to promote proliferation. Second, SHP2 is involved in the PD-1/PD-L1 signaling pathway and promotes immune escape. The PD-1/SHP2/STAT1/T-bet signaling axis mediates the immunosuppressive effect of PD-1 on Th1 cells. Therefore, inhibition of PD-1 or SHP2 can restore Th1 immune function and T cell activation, and relieve immunosuppression in the tumor microenvironment.

SHP2 is associated with the occurrence of various diseases, such as Noonan syndrome, breast cancer, melanoma, gastric cancer, esophageal cancer, lung cancer, colon cancer, head cancer, neuroblastoma, head and neck squamous cell carcinoma, anaplastic large cell lymphoma and glioblastoma.

Inhibitors targeting the catalytic site generally have poor selectivity and druggability. In recent years, researchers have found that inhibiting the activity of SHP2 through the allosteric site can improve the activity and selectivity, and drug research has also made some progress. However, there is still a need to develop better SHP2 inhibitors in order to obtain drugs with superior activity and better pharmacokinetic properties for the treatment of SHP2-mediated related diseases.

### SUMMARY

An object of the present disclosure is to provide a class of compounds with SHP2 inhibitory activity represented by general formula (I) or an isomer, a pharmaceutically acceptable salt, a solvate, a crystal or a prodrug thereof, 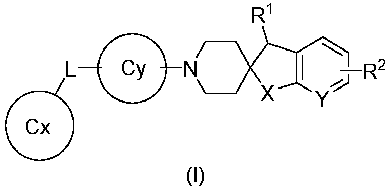 wherein,
L is absent or selected from -NH-, -S- and -O-;
X is selected from CH₂, NH, O and S;
Y is selected from CH and N;
R¹ and R² are independently selected from hydrogen, halogen, hydroxyl, alkyl, haloalkyl, hydroxyalkyl, alkoxy, haloalkoxy, hydroxyalkoxy, nitro, carboxyl, cyano, amino, monoalkylamino, alkylacylamino, alkylacyl, aminoacyl, alkylaminoacyl, bisalkylamino and cycloalkyl;
ring Cx is selected from aryl, heteroaryl, cycloalkyl and heterocyclyl, and the aryl, heteroaryl, cycloalkyl or heterocyclyl is optionally substituted with one or more R³, wherein R³ is selected from halogen, hydroxy, alkyl, haloalkyl, hydroxyalkyl, alkoxy, haloalkoxy, hydroxyalkoxy, nitro, carboxyl, cyano, amino, monoalkylamino, alkylacylamino, alkylacyl, alkylsulfonyl, aminoacyl, alkylaminoacyl, bisalkylamino, alkenyl, alkynyl, cycloalkyl, heterocyclyl, substituted heterocyclyl, cycloalkylamino, cycloalkylacyl, heterocyclylacyl, substituted heterocyclylacyl, cycloalkylaminoacyl, cycloalkylacylamino, alkylsulfonyl, alkylaminosulfonyl, alkylsulfonamido, cycloalkylsulfonamido, bisalkylphosphoryl and oxo group; and
ring Cy is selected from 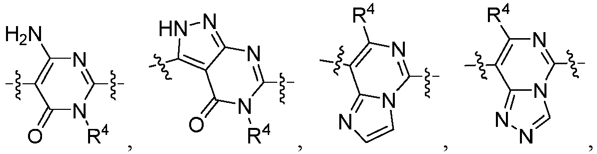 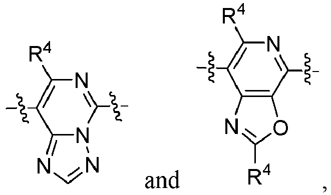 wherein R⁴ is selected from hydrogen, halogen, hydroxy, alkyl, haloalkyl, hydroxyalkyl, alkoxy, haloalkoxy, hydroxyalkoxy, nitro, carboxyl, cyano, amino, monoalkylamino, alkylacylamino, alkylacyl, aminoacyl, alkylaminoacyl, bisalkylamino and cycloalkyl.

Another object of the present disclosure is to provide a method for preparing the compound represented by general formula (I) or an isomer, a pharmaceutically acceptable salt, a solvate, a crystal or a prodrug thereof of the present disclosure.

Yet another object of the present disclosure is to provide a composition comprising the compound represented by general formula (I) or an isomer, a pharmaceutically acceptable salt, a solvate, a crystal or a prodrug thereof of the present disclosure and a pharmaceutically acceptable carrier, as well as a composition comprising the compound represented by general formula (I) or an isomer, a pharmaceutically acceptable salt, a solvate, a crystal or a prodrug thereof of the present disclosure and one or more additional drugs.

Still another object of the present disclosure is to provide a method for treating an SHP2-mediated disease using the compound represented by general formula (I) or an isomer, a pharmaceutically acceptable salt, a solvate, a crystal or a prodrug thereof of the present disclosure, as well as use of the compound represented by general formula (I) or an isomer, a pharmaceutically acceptable salt, a solvate, a crystal or a prodrug thereof of the present disclosure in the manufacture of a medicament for treating an SHP2-mediated disease.

For the above-mentioned purposes, the present disclosure provides the following technical solutions:

In the first aspect, the present disclosure provides a compound represented by general formula (I) or an isomer, a pharmaceutically acceptable salt, a solvate, a crystal or a prodrug thereof, 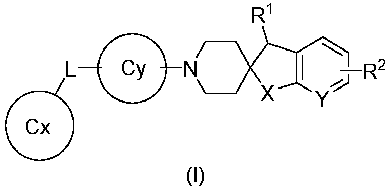 wherein,
L is absent or selected from -NH-, -S- and -O-;
X is selected from CH₂, NH, O and S;
Y is selected from CH and N;
R¹ and R² are independently selected from hydrogen, halogen, hydroxyl, alkyl, haloalkyl, hydroxyalkyl, alkoxy, haloalkoxy, hydroxyalkoxy, nitro, carboxyl, cyano, amino, monoalkylamino, alkylacylamino, alkylacyl, aminoacyl, alkylaminoacyl, bisalkylamino and cycloalkyl;
ring Cx is selected from aryl, heteroaryl, cycloalkyl and heterocyclyl, and the aryl, heteroaryl, cycloalkyl or heterocyclyl is optionally substituted with one or more R³, wherein R³ is selected from halogen, hydroxy, alkyl, haloalkyl, hydroxyalkyl, alkoxy, haloalkoxy, hydroxyalkoxy, nitro, carboxyl, cyano, amino, monoalkylamino, alkylacylamino, alkylacyl, alkylsulfonyl, aminoacyl, alkylaminoacyl, bisalkylamino, alkenyl, alkynyl, cycloalkyl, heterocyclyl, substituted heterocyclyl, cycloalkylamino, cycloalkylacyl, heterocyclylacyl, substituted heterocyclylacyl, cycloalkylaminoacyl, cycloalkylacylamino, alkylsulfonyl, alkylaminosulfonyl, alkylsulfonamido, cycloalkylsulfonamido, bisalkylphosphoryl and oxo group; and
ring Cy is selected from 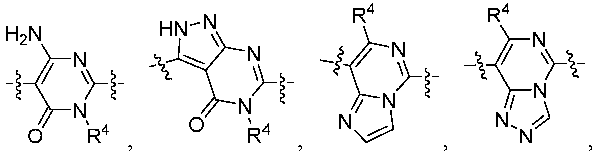 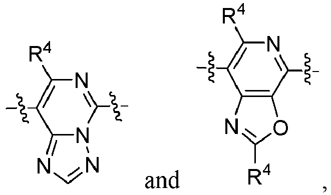 wherein R⁴ is selected from hydrogen, halogen, hydroxy, alkyl, haloalkyl, hydroxyalkyl, alkoxy, haloalkoxy, hydroxyalkoxy, nitro, carboxyl, cyano, amino, monoalkylamino, alkylacylamino, alkylacyl, aminoacyl, alkylaminoacyl, bisalkylamino and cycloalkyl.

In some preferred embodiments, the compound of the present disclosure is a compound represented by general formula (I) or an isomer, a pharmaceutically acceptable salt, a solvate, a crystal or a prodrug thereof, wherein:

Cx is selected from C₆₋₁₂ aryl, C₅₋₁₂ heteroaryl, C₃₋₆ cycloalkyl and C₃₋₆ heterocyclyl, and the aryl, heteroaryl, cycloalkyl or heterocyclyl is substituted with one or more R³, wherein R³ is selected from halogen, hydroxy, alkyl, haloalkyl, hydroxyalkyl, alkoxy, haloalkoxy, hydroxyalkoxy, nitro, carboxyl, cyano, amino, monoalkylamino, alkylacylamino, alkylacyl, alkylsulfonyl, aminoacyl, alkylaminoacyl, bisalkylamino, alkenyl, alkynyl, cycloalkyl, heterocyclyl, substituted heterocyclyl, cycloalkylamino, cycloalkylacyl, heterocyclylacyl, substituted heterocyclylacyl, cycloalkylaminoacyl, cycloalkylacylamino, alkylsulfonyl, alkylsulfonamido, bisalkylphosphoryl and oxo group.

Further preferably, Cx is selected from C₆₋₈ aryl, C₅₋₁₀ heteroaryl, C₃₋₆ cycloalkyl and C₃₋₆ heterocyclyl, and the aryl, heteroaryl, cycloalkyl or heterocyclyl is substituted with one or more R³, wherein R³ is selected from halogen, hydroxy, alkyl, haloalkyl, hydroxyalkyl, alkoxy, haloalkoxy, hydroxyalkoxy, nitro, carboxyl, cyano, amino, monoalkylamino, alkylacylamino, alkylacyl, alkylsulfonyl, aminoacyl, alkylaminoacyl, bisalkylamino, alkenyl, alkynyl, cycloalkyl, heterocyclyl, substituted heterocyclyl, cycloalkylamino, cycloalkylacyl, heterocyclylacyl, substituted heterocyclylacyl, cycloalkylaminoacyl, cycloalkylacylamino, alkylsulfonyl, alkylaminosulfonyl, alkylsulfonamido, cycloalkylsulfonamido, bisalkylphosphoryl and oxo group;

More preferably, Cx is selected from phenyl, pyridyl, pyrimidinyl, pyrazinyl, imidazolyl, oxazolyl, thiazolyl, isoxazolyl, isothiazolyl, pyrazolophenyl, pyrazolopyridyl, oxazolophenyl, oxazolopyridyl, dihydrooxazolophenyl, dihydrooxazolopyridyl, imidazophenyl, pyrrolyl, furyl, thienyl, pyrazolyl, isoxazolyl, isothiazolyl, oxadiazolyl, triazolyl, indolyl, isoindolyl, dihydrobenzopyrazolyl, dihydrobenzoxazolyl, dihydrobenzothiazolyl, dihydrobenzisoxazolyl, dihydrobenzisothiazolyl, tetrahydroquinolinyl, tetrahydroisoquinolinyl, dihydroquinolinyl, dihydroisoquinolinyl, tetrahydroquinazolinyl, dihydroquinazolinyl, dihydrobenzoxazinyl, dihydrobenzothiazinyl, benzodioxenyl, benzomorpholinyl, benzoxathianyl, tetrahydropyrrolopyrazolyl, tetrahydropiperidinopyrazolyl, dihydropiperidinopyrazolyl, pyrazolodihydropyrazolyl, benzopyrazolyl, benzimidazolyl, benzofuranyl, benzopyranyl, benzothienyl, benzoxazolyl, benzothiazolyl, benzisoxazolyl, benzisothiazolyl, quinolinyl, isoquinolinyl, quinazolinyl, aziridinyl, azetidinyl, tetrahydropyrrolyl, dihydropyrrolyl, optionally substituted with one or more R³;

In some preferred embodiments, the compound of the present disclosure is a compound represented by general formula (I) or an isomer, a pharmaceutically acceptable salt, a solvate, a crystal or a prodrug thereof, wherein:

R³ is selected from halogen, hydroxy, alkyl, haloalkyl, hydroxyalkyl, alkoxy, haloalkoxy, hydroxyalkoxy, nitro, carboxyl, cyano, amino, monoalkylamino, alkylacylamino, alkylacyl, alkylsulfonyl, aminoacyl, alkylaminoacyl, bisalkylamino, alkenyl, alkynyl, cycloalkyl, heterocyclyl, substituted heterocyclyl, cycloalkylamino, cycloalkylacyl, heterocyclylacyl, substituted heterocyclylacyl, cycloalkylaminoacyl, cycloalkylacylamino, alkylsulfonyl, alkylaminosulfonyl, alkylsulfonamido, cycloalkylsulfonamido, bisalkylphosphoryl and oxo group;

Further preferably, R³ is selected from halogen, hydroxy, C₁₋₆ alkyl, halogenated C₁₋₆ alkyl, hydroxy C₁₋₆ alkyl, C₁₋₆ alkoxy, halogenated C₁₋₆ alkoxy, hydroxy C₁₋₆ alkoxyl, nitro, carboxyl, cyano, amino, mono-C₁₋₆ alkylamino, C₁₋₆ alkylacylamino, C₁₋₆ alkylacyl, C₁₋₆ alkylsulfonyl, aminoacyl, C₁₋₆ alkylaminoacyl, bis-C₁₋₆ alkylamino, C₂₋₁₀ alkenyl, C₂₋₁₀ alkynyl, C₃₋₈ cycloalkyl, C₃₋₈ heterocyclyl, substituted C₃₋₈ heterocyclyl, C₃₋₈ cycloalkylamino, C₃₋₈ cycloalkylacyl, C₃₋₈ heterocyclylacyl, substituted C₃₋₈ heterocyclylacyl, C₃₋₈ cycloalkylaminoacyl, C₃₋₈ cycloalkylacylamino, C₁₋₆ alkylsulfonyl, C₁₋₆ alkylaminosulfonyl, C₁₋₆ alkylsulfonamido, C₃₋₈ cycloalkylsulfonamido, bis-C₁₋₆ alkylphosphoryl and oxo group;

More preferably, R³ is selected from halogen, hydroxy, C₁₋₃ alkyl, halogenated C₁₋₃ alkyl, hydroxy C₁₋₃ alkyl, C₁₋₃ alkoxy, halogenated C₁₋₃ alkoxy, hydroxy C₁₋₃ alkoxyl, nitro, carboxyl, cyano, amino, mono-C₁₋₃ alkylamino, C₁₋₃ alkylacylamino, C₁₋₃ alkylacyl, aminoacyl, C₁₋₃ alkylaminoacyl, bis-C₁₋₃ alkylamino, C₂₋₆ alkenyl, C₂₋₆ alkynyl, C₃₋₆ cycloalkyl, C₃₋₆ cycloalkyl, C₃₋₆ heterocyclyl, substituted C₃₋₆ heterocyclyl, C₃₋₆ cycloalkylamino, C₃₋₆ cycloalkylacyl, C₃₋₆ heterocyclylacyl, substituted C₃₋₆ heterocyclylacyl, C₃₋₆ cycloalkylaminoacyl, C₃₋₆ cycloalkylacylamino, C₁₋₃ alkylsulfonyl, C₁₋₃ alkylaminosulfonyl, C₁₋₃ alkylsulfonamido, C₃₋₆ cycloalkylsulfonamido, bis-C₁₋₃ alkylphosphoryl and oxo group;

Still more preferably, R³ is selected from hydrogen, fluorine, chlorine, bromine, iodine, hydroxyl, methyl, ethyl, propyl, isopropyl, cyclopropyl, cyclobutyl, cyclopentyl, cyclohexyl, difluoromethyl, trifluoromethyl, trifluoroethyl, hydroxymethyl, hydroxyethyl, hydroxypropyl, 2-hydroxypropyl, methoxy, ethoxy, propoxy, isopropoxy, trifluoromethoxy, hydroxymethoxy, hydroxyethoxy, hydroxypropoxy, nitro, carboxyl, cyano, amino, methylamino, dimethylamino, ethylamino, diethylamino, methylethylamino, methylacylamino, ethylacylamino, vinylacylamino, methylacyl, ethylacyl, vinylacyl, aminoacyl, methylaminoacyl, ethylaminoacyl, vinyl, ethynyl, C₃₋₆ cycloalkyl, substituted C₃₋₆ heterocyclyl, C₃₋₆ cycloalkylamino, C₃₋₆ cycloalkylacyl, C₃₋₆ heterocyclylacyl, substituted C₃₋₆ heterocyclylacyl, C₃₋₆ cycloalkylaminoacyl, C₃₋₆ cycloalkylacylamino, C₁₋₃ alkylsulfonyl, C₁₋₃ alkylaminosulfonyl, C₁₋₃ alkylsulfonamido, C₃₋₆ cycloalkylsulfonamido, bis-C₁₋₃ alkyl phosphoryl and oxo group.

In some preferred embodiments, the compound of the present disclosure is a compound represented by general formula (I) or an isomer, a pharmaceutically acceptable salt, a solvate, a crystal or a prodrug thereof, wherein:

R¹ and R² are independently selected from hydrogen, halogen, hydroxy, C₁₋₆ alkyl, halogenated C₁₋₆ alkyl, hydroxy C₁₋₆ alkyl, C₁₋₆ alkoxy, halogenated C₁₋₆ alkoxy, hydroxy C₁₋₆ alkoxy, nitro, carboxyl, cyano, amino, mono-C₁₋₆ alkylamino, C₁₋₆ alkylacylamino, C₁₋₆ alkylacyl, aminoacyl, C₁₋₆ alkylaminoacyl, bis-C₁₋₆ alkylamino and C₃₋₁₂ cycloalkyl;

Further preferably, R¹ and R² are each independently selected from hydrogen, halogen, hydroxyl, C₁₋₃ alkyl, halogenated C₁₋₃ alkyl, hydroxy C₁₋₃ alkyl, C₁₋₃ alkoxy, halogenated C₁₋₃ alkoxy, hydroxy C₁₋₃ alkoxy, nitro, carboxyl, cyano, amino, mono-C₁₋₃ alkylamino, C₁₋₃ alkylacylamino, C₁₋₃ alkylacyl, aminoacyl, C₁₋₃ alkylaminoacyl, bis-C₁₋₃ alkylamino and C₃₋₈ cycloalkyl;

More preferably, R¹ and R² are each independently selected from hydrogen, halogen, hydroxyl, methyl, ethyl, propyl, isopropyl, halogenated C₁₋₃ alkyl, hydroxy C₁₋₃ alkyl, C₁₋₃ alkoxy, halogenated C₁₋₃ alkoxy, hydroxy C₁₋₃ alkoxy, nitro, carboxyl, cyano, amino, mono-C₁₋₃ alkylamino, C₁₋₃ alkylacylamino, C₁₋₃ alkylacyl, aminoacyl, C₁₋₃ alkylaminoacyl, bis-C₁₋₃ alkylamino and C₃₋₈ cycloalkyl.

In some preferred embodiments, the compound of the present disclosure is a compound represented by general formula (I) or an isomer, a pharmaceutically acceptable salt, a solvate, a crystal or a prodrug thereof, wherein R⁴ is selected from hydrogen, halogen, hydroxyl, C₁₋₆ alkyl, halogenated C₁₋₆ alkyl, hydroxy C₁₋₆ alkyl, C₁₋₆ alkoxy, halogenated C₁₋₆ alkoxy, hydroxy C₁₋₆ alkoxy, nitro, carboxyl, cyano, amino, mono-C₁₋₆ alkylamino, C₁₋₆ alkylacylamino, C₁₋₆ alkylacyl, aminoacyl, C₁₋₆ alkylaminoacyl, bis-C₁₋₆ alkylamino and C₃₋₈ cycloalkyl.

In some preferred embodiments, the compound of the present disclosure is a compound represented by general formula (Ia), (Ib), (Ic), (Id), (Ie) or (If) or an isomer, a pharmaceutically acceptable salt, a solvate, a crystal or a prodrug thereof, 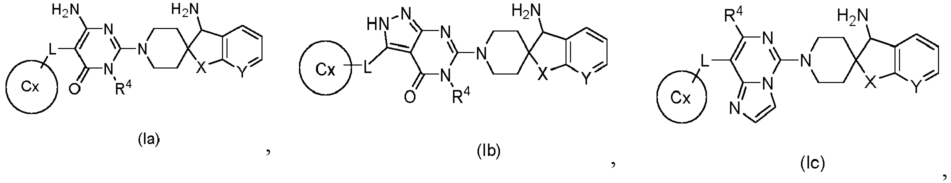 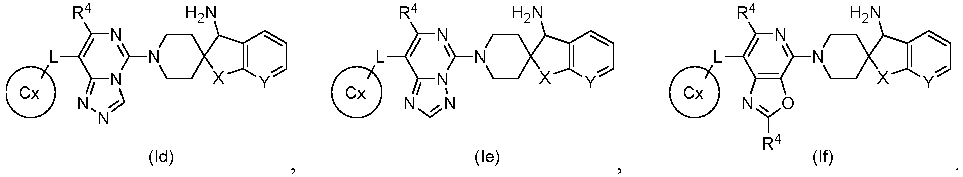 wherein, Cx, L, R⁴, X and Y have the definitions set forth above for general formula (I).

In some preferred embodiments, the compound of the present disclosure is a compound represented by general formula (I), (Ia), (Ib), (Ic), (Id), (Ie) or (If) or an isomer, a pharmaceutically acceptable salt, a solvate, a crystal or a prodrug thereof, wherein:

Cx is selected from 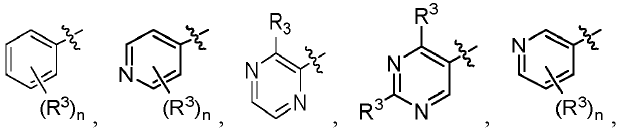 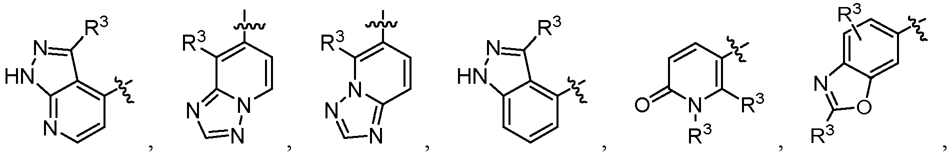 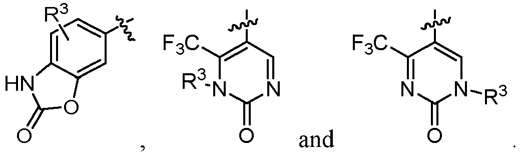 wherein n is 1, 2 or 3, and R³ has the definition set forth above for general formula (I).

In some preferred embodiments, the compound of the present disclosure is a compound represented by general formula (I), (Ia), (Ib), (Ic), (Id), (Ie) or (If) or an isomer, a pharmaceutically acceptable salt, a solvate, a crystal or a prodrug thereof, wherein:

Cx is selected from

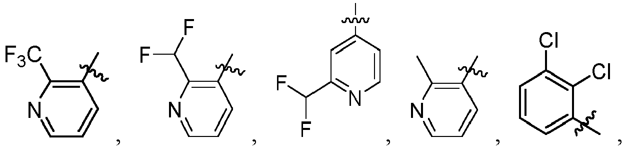

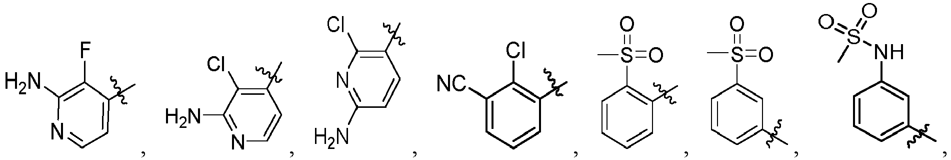

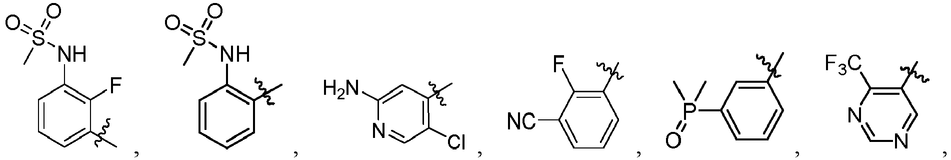

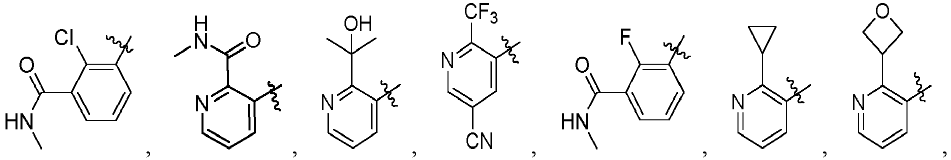

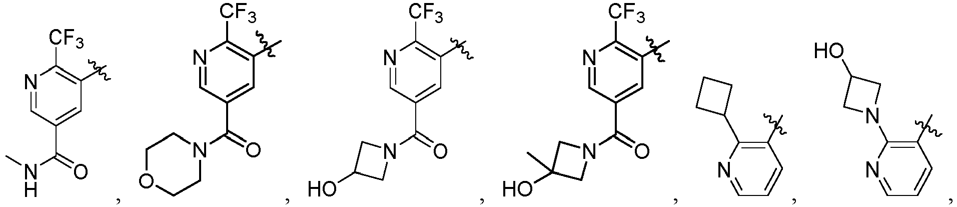

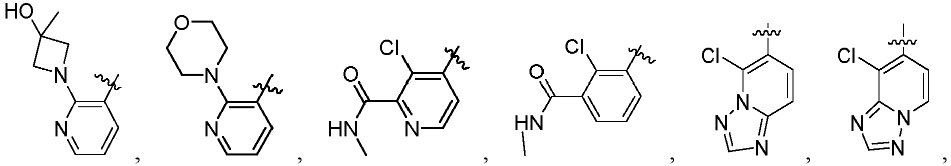

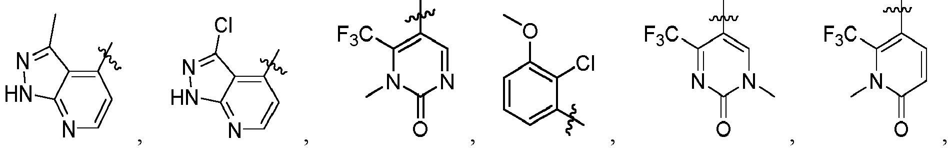

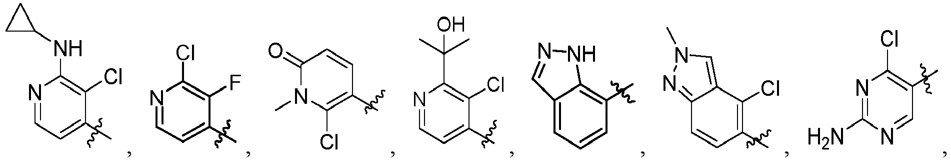

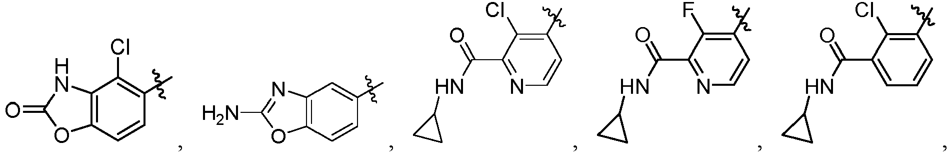

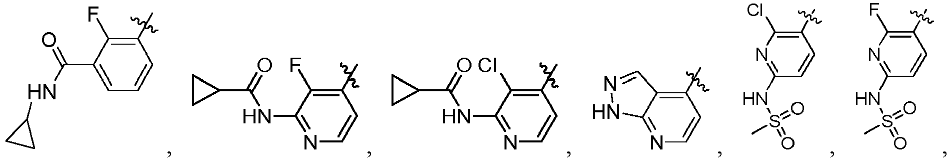

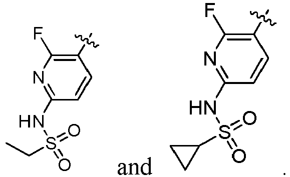

In some preferred embodiments, the compound of the present disclosure is a compound represented by general formula (I), (Ia), (Ib), (Ic), (Id), (Ie) or (If) or an isomer, a pharmaceutically acceptable salt, a solvate, a crystal or a prodrug thereof, wherein L is absent.

In some preferred embodiments, the compound of the present disclosure is a compound represented by general formula (I), (Ia), (Ib), (Ic), (Id), (Ie) or (If) or an isomer, a pharmaceutically acceptable salt, a solvate, a crystal or a prodrug thereof, wherein L is -S-.

In some preferred embodiments, the compound of the present disclosure is a compound represented by general formula (I), (Ia), (Ib), (Ic), (Id), (Ie) or (If) or an isomer, a pharmaceutically acceptable salt, a solvate, a crystal or a prodrug thereof, wherein X is CH₂ or O.

In some preferred embodiments, the compound of the present disclosure is a compound represented by general formula (I), (Ia), (Ib), (Ic), (Id), (Ie) or (If) or an isomer, a pharmaceutically acceptable salt, a solvate, a crystal or a prodrug thereof, wherein R⁴ is selected from hydrogen, methyl and ethyl.

The present disclosure provides the following specific compounds or an isomer, a pharmaceutically acceptable salt, a solvate, a crystal or a prodrug thereof:

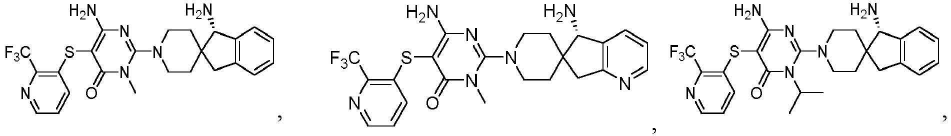

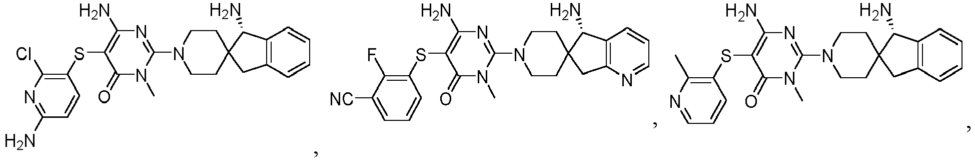

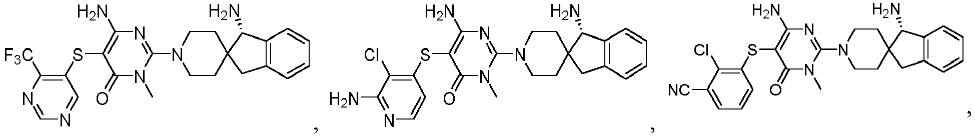

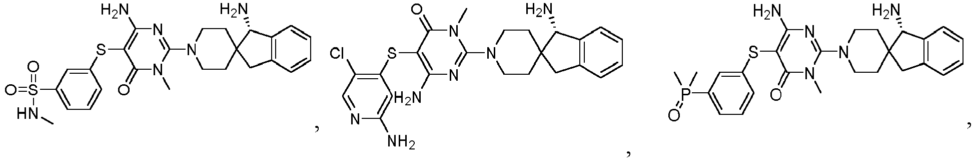

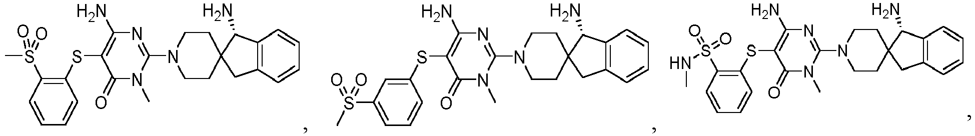

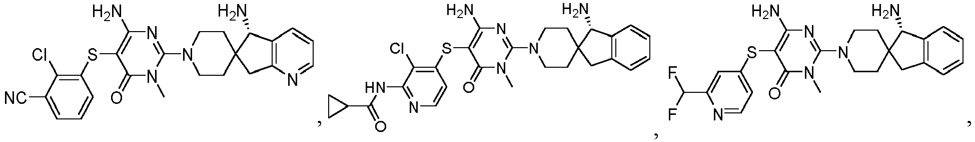

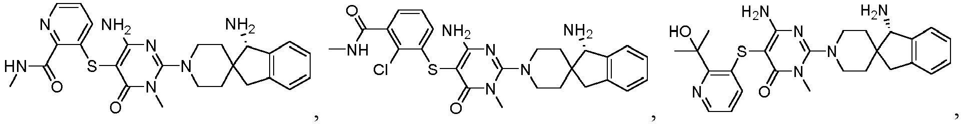

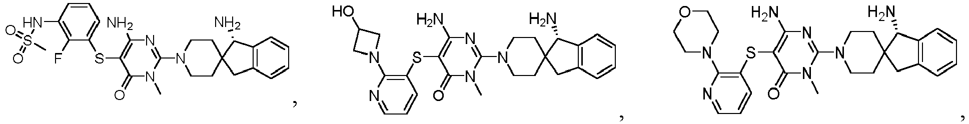

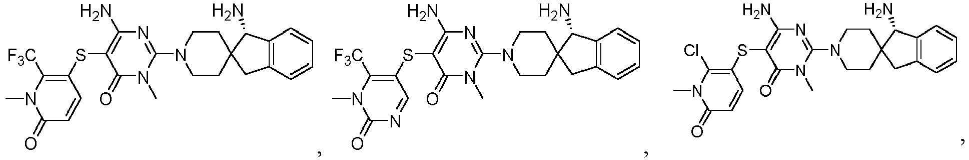

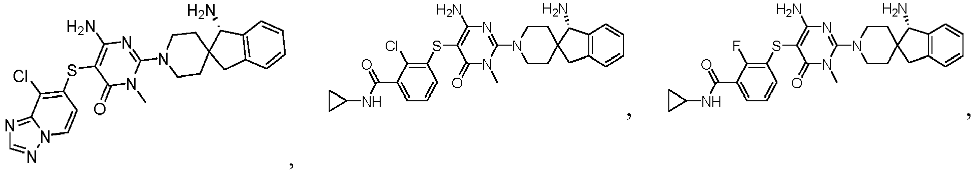

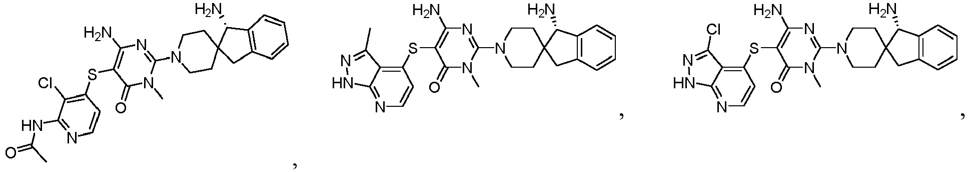

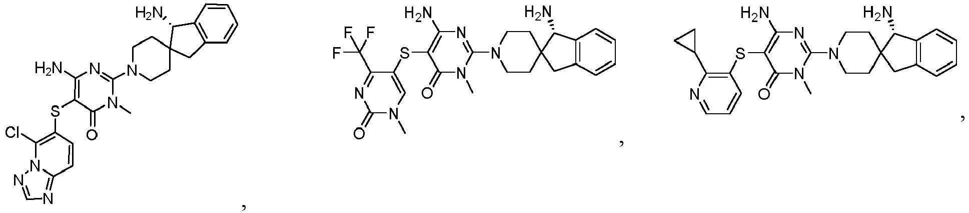

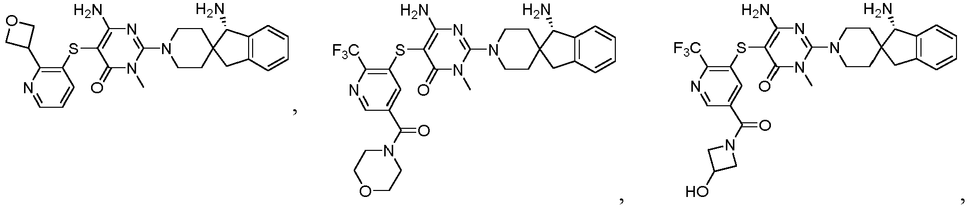

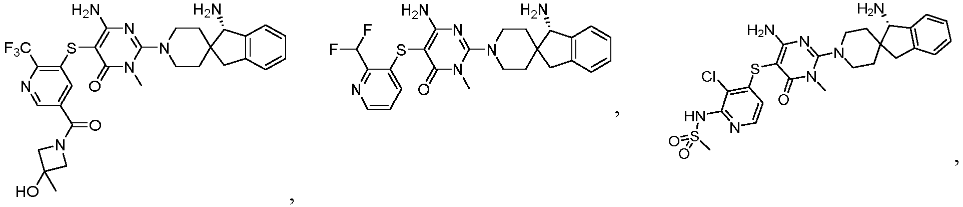

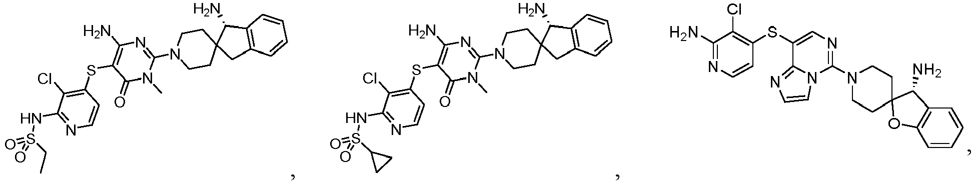

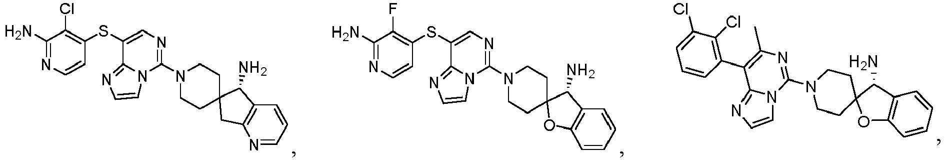

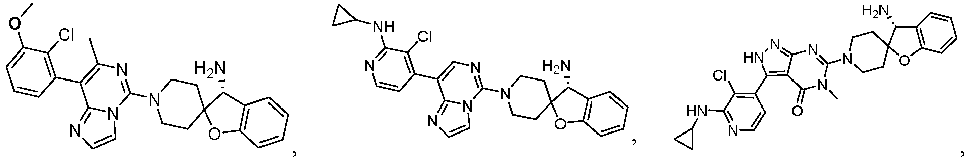

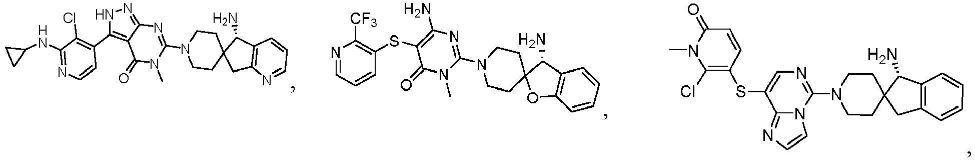

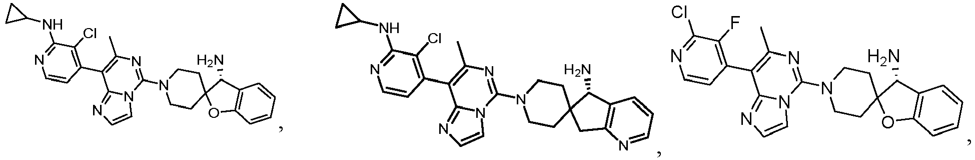

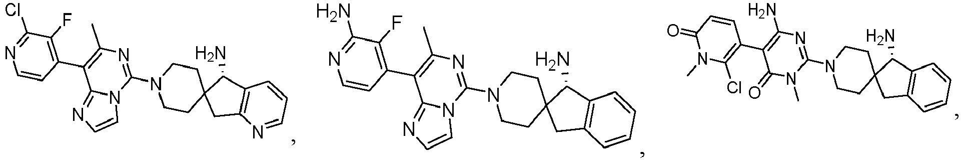

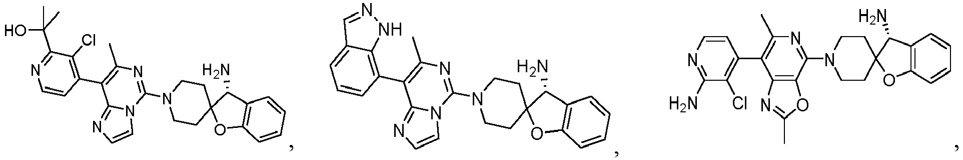

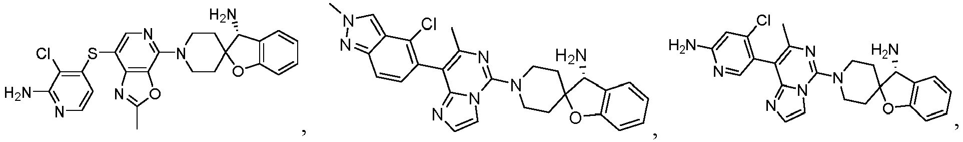

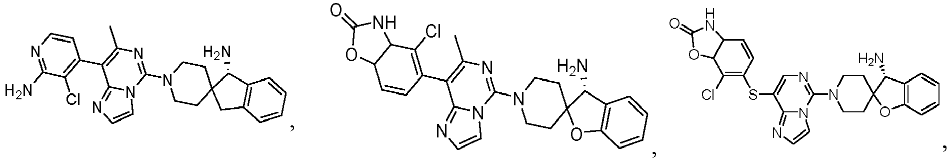

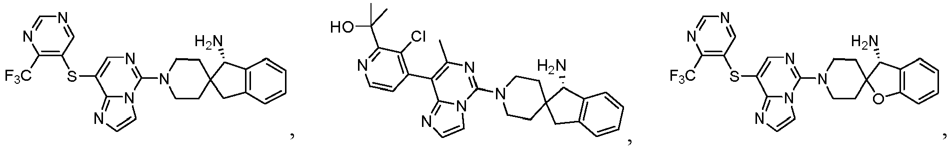

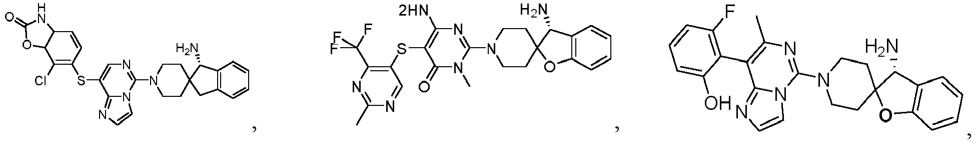

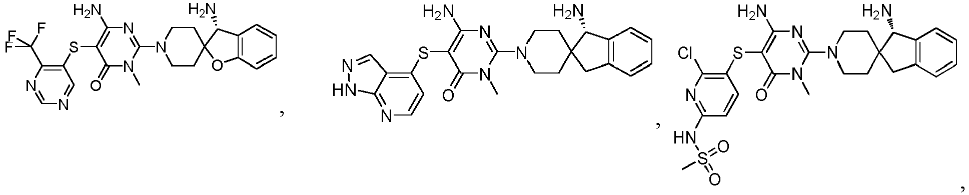

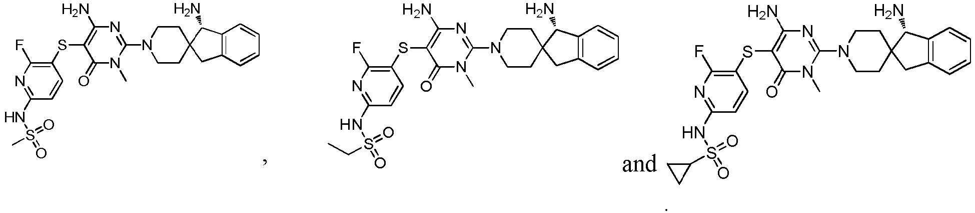

In another aspect, the present disclosure provides a method for preparing the compound represented by general formula (I) of the present disclosure, comprising steps of: 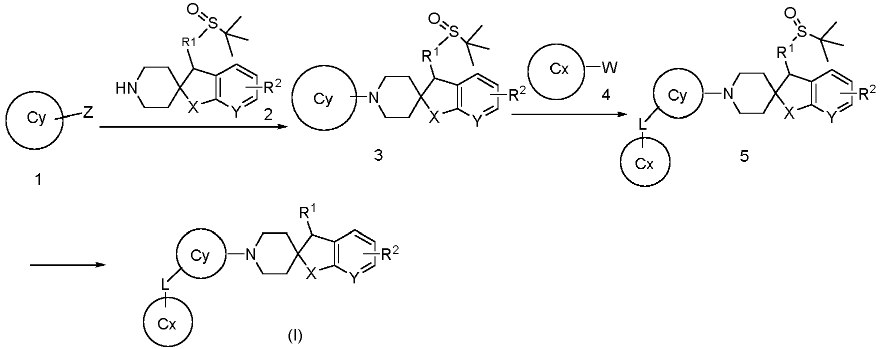 or 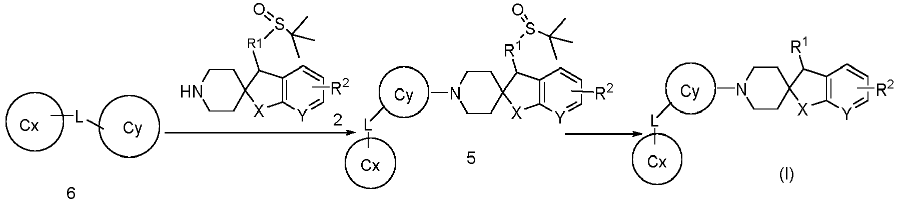
1) A compound of formula (1) reacts with a compound of formula (2) to generate a compound of formula (3);
2) A compound of formula (3) reacts with a compound of formula (4) to obtain a compound of formula (5); or
3) A compound of formula (6) reacts with a compound of formula (2) to obtain a compound of formula (5)
4) A compound of formula (5) is reacted under acidic conditions to obtain a compound of formula (I);
wherein, R¹, R², Cx, Cy, L, X and Y have the definitions set forth for the general formula (I); Z is sulfydryl; W is a halogen; the compound of formula 1, the compound of formula 2, the compound of formula 4 and the compound of formula 6 are commercially available compounds or can be synthesized by other technical means commonly used by those skilled in the art.

In a third aspect, the present disclosure provides a pharmaceutical composition comprising the compound or an isomer, a pharmaceutically acceptable salt, a solvate, a crystal or a prodrug thereof of the present disclosure.

In some embodiments, the present disclosure provides the compound or an isomer, a pharmaceutically acceptable salt, a solvate, a crystal or a prodrug thereof of the present disclosure and a pharmaceutical composition comprising the compound or an isomer, a pharmaceutically acceptable salt, a solvate, a crystal or a prodrug thereof of the present disclosure for use in the treatment of an SHP2 mediated disease.

In some embodiments, the present disclosure provides a pharmaceutical composition comprising the compound or an isomer, a pharmaceutically acceptable salt, a solvate, a crystal or a prodrug thereof of the present, and a pharmaceutically acceptable carrier.

The compound or an isomer, a pharmaceutically acceptable salt, a solvate, a crystal or a prodrug thereof of the present can be mixed with a pharmaceutically acceptable carrier, diluent or excipient to prepare a pharmaceutical preparation suitable for oral or parenteral administration. Routes of administration include, but are not limited to, intradermal, intramuscular, intraperitoneal, intravenous, subcutaneous, intranasal, and oral routes. The preparation may be administered by any route, such as by infusion or bolus injection, by route of epithelium or mucocutaneous (e.g., oral mucosa or rectum, etc.) absorption. Administration can be systemic or local. Examples of preparations for oral administration include solid or liquid dosage forms, specifically, tablets, pills, granules, powders, capsules, syrups, emulsions, suspensions and the like. The preparation can be prepared by methods known in the art and comprises carriers, diluents or excipients conventionally used in the art of pharmaceutical preparations.

In the fourth aspect, the present disclosure provides a method for treating an SHP2-mediated disease using the compound represented by formula (I), (Ia), (Ib), (Ic), (Id), (Ie) or (If) or an isomer thereof, a pharmaceutically acceptable salt, a solvate, a crystal or a prodrug thereof of the present disclosure or a pharmaceutical composition comprising the same, as well as use of the compound or the pharmaceutical composition in the manufacture of a medicament for treating an SHP2-mediated disease.

In some preferred embodiments, the present disclosure provides a method for treating an SHP2-mediated disease using the compound represented by formula (I), (Ia), (Ib), (Ic), (Id), (Ie) or (If) or an isomer thereof, a pharmaceutically acceptable salt, a solvate, a crystal or a prodrug thereof of the present disclosure or a pharmaceutical composition comprising the same, as well as use of the compound or the pharmaceutical composition in the manufacture of a medicament for treating an SHP2-mediated disease, wherein the SHP2-mediated disease includes, but is not limited to, proliferative diseases, metabolic diseases or hematological diseases. In some embodiments, the SHP2-mediated disease described herein is cancer.

In some embodiments, the SHP2-mediated disease described herein includes, but is not limited to: acoustic neuroma, adenocarcinoma, adrenal cancer, anal cancer, angiosarcoma (such as lymphangiosarcoma, lymphatic endothelial sarcoma, angiosarcoma), adnexal carcinoma, benign monoclonal gammopathy, gallbladder cancer (such as cholangiocarcinoma), bladder cancer, breast cancer (such as breast adenocarcinoma, papillary breast carcinoma, breast cancer, medullary breast carcinoma, triple negative breast cancer), brain cancer (such as meningioma; glioma, such as astrocytoma, oligodendroglioma; medulloblastoma), bronchial carcinoma, carcinoid tumor, cervical cancer (such as cervical adenocarcinoma), choriocarcinoma, chordoma, craniopharyngioma, colorectal cancer (such as colon cancer, rectal cancer, colorectal adenocarcinoma), epithelial cancer, ependymoma, endothelial sarcoma (such as Kaposi's sarcoma, multiple idiopathic hemorrhagic sarcoma), endometrial cancer (such as uterine cancer, uterine sarcoma), esophageal cancer (such as esophageal adenocarcinoma, Barrett's adenocarinoma), Ewing sarcoma, eye cancer (such as intraocular melanoma, retinoblastoma), familial eosinophilia, gallbladder cancer, gastric cancer (such as gastric adenocarcinoma), gastrointestinal stromal tumor (GIST), head and neck cancer (such as head and neck squamous cell carcinoma, oral cancer (such as oral squamous cell carcinoma (OSCC), throat cancer (such as laryngeal cancer, pharyngeal cancer, nasopharyngeal cancer, oropharyngeal carcinoma)), hematopoietic cancer (such as leukemia, such as acute lymphoblastic leukemia (ALL) (such as B-cell ALL, T-cell ALL), acute myeloid leukemia (AML) (such as B-cell AML, T-cell AML), chronic myelogenous leukemia (CML) (such as B-cell CML, T-cell CML), and chronic lymphocytic leukemia (CLL) (such as B-cell CLL, T-cell CLL)); lymphoma, such as Hodgkin lymphoma (HL) (such as B-cell HL, T-cell HL) and non-Hodgkin lymphoma (NHL) (such as B-cell NHL, such as diffuse large cell lymphoma (DLCL) (such as diffuse large B-cell lymphoma (DLBCL)), follicular lymphoma, chronic lymphocytic leukemia/small lymphocytic lymphoma (CLL/SLL), mantle cell lymphoma (MCL), marginal zone B-cell lymphoma (such as mucosa-associated lymphoid tissue (MALT) lymphoma, nodular marginal zone B-cell lymphoma, splenic marginal zone B-cell lymphoma), primary mediastinal B-cell lymphoma tumor, Burkitt lymphoma, lymphoplasmacytic lymphoma (i.e., "Waldenström's macroglobulinemia"), hairy cell leukemia (HCL), immunoblastic large cell lymphoma, precursor B-lymphoblastic lymphoma, and primary central nervous system (CNS) lymphoma; and T-cell NHL, such as precursor T-lymphoblastic lymphoma/leukemia, peripheral T-cell lymphoma (PTCL) (such as cutaneous T-cell lymphoma (CTCL) (such as mycosis fungiodes, Sezary syndrome), angioimmunoblastic T-cell lymphoma, extranodal natural killer T-cell lymphoma, enteropathy type T-cell lymphoma, subcutaneous panniculitis-like T-cell lymphoma, anaplastic large cell lymphoma); a combination of one or more of the leukemias/lymphomas described above; and multiple myeloma (MM), heavy chain disease (such as α chain disease, γ chain disease, µ chain disease), hemangioblastoma, inflammatory myofibroblastic tumor, immune cell amyloidosis, kidney cancer (such as nephroblastoma also known as Wilms' tumor, renal cell carcinoma), liver cancer (such as hepatocellular carcinoma (HCC), malignant hepatocellular tumor), lung cancer (such as bronchial carcinoma, small cell lung cancer (SCLC), non-small cell lung cancer (NSCLC), lung adenocarcinoma), leiomyosarcoma (LMS), mastocytosis (such as systemic mastocytosis), myelodysplastic syndrome (MDS), mesothelioma, myeloproliferative disease (MPD) (such as polycythemia vera (PV), essential thrombocythemia (ET), agnogenic myeloid metaplasia (AMM) also known as myelofibrosis (MF), chronic idiopathic myelofibrosis, chronic myelogenous leukemia (CML), chronic neutrophilic leukemia (CNL), hypereosinophilic syndrome (HES)), neuroblastoma, neurofibromatosis (such as type 1 or 2 multiple neurofibromatosis (NF), schwannomatosis), neuroendocrine carcinoma (such as gastroenteropancreatic neuroendocrine tumor (GEP-NET), carcinoid tumors), osteosarcoma, ovarian cancer (such as cystadenocarcinoma, ovarian embryonal carcinoma, ovarian adenocarcinoma, ovarian clear cell carcinoma, ovarian serous cystadenocarcinoma), papillary adenocarcinoma, pancreatic cancer (such as pancreatic adenocarcinoma, intraductal papillary mucinous neoplasm (IPMN), pancreatic islet cell tumor), penile cancer (such as Paget's disease of the penis and scrotum), pineal tumor, primary neuroectodermal tumor (PNT), prostate cancer (such as prostate adenocarcinoma), rectal cancer, rhabdomyosarcoma, salivary duct cancer, skin cancer (such as squamous cell carcinoma (SCC), keratoacanthoma (KA), melanoma, basal cell carcinoma (BCC)), small intestinal cancer (such as adnexal carcinoma), soft tissue sarcoma (such as malignant fibrous histiocytoma (MFH), liposarcoma, malignant peripheral nerve sheath tumor (MPNST), chondrosarcoma, fibrosarcoma, myxosarcoma), sebaceous carcinoma, sweat gland carcinoma, synovial tumor, testicular cancer (such as seminoma, testicular embryonal carcinoma), thyroid cancer (such as thyroid papillary cancer, papillary thyroid cancer (PTC), medullary thyroid cancer), urethral cancer, vaginal cancer, and vulvar cancer (such as Paget's disease of the vulva), medulloblastoma, adenoid cystic carcinoma, melanoma, glioblastoma.

In some preferred embodiments, the present disclosure provides a method for treating an SHP2-mediated disease using the compound represented by formula I or an isomer thereof, a pharmaceutically acceptable salt, a solvate, a crystal or a prodrug thereof of the present disclosure or a pharmaceutical composition comprising the same, as well as use of the compound or the pharmaceutical composition in the manufacture of a medicament for treating an SHP2-mediated disease, wherein the SHP2-mediated disease includes, but is not limited to, non-small cell lung cancer, breast cancer, esophagus cancer, bladder cancer, lung cancer, hematopoietic cancer, lymphoma, medulloblastoma, medulloblastoma, rectal adenocarcinoma, colon cancer, gastric cancer, pancreatic cancer, liver cancer, adenoid cystic carcinoma, prostate cancer, lung cancer, head and neck squamous cell carcinoma, brain cancer, hepatocellular carcinoma, melanoma, oligodendroglioma, glioblastoma, testicular cancer, ovarian clear cell carcinoma, ovarian serous cystadenocarcinoma, thyroid cancer, multiple myeloma (AML), renal cell carcinoma, mantle cell lymphoma, triple negative breast cancer, hemoglobinopathies, diabetes and obesity.

### Definition of Terms

Unless stated to the contrary, the terms used in the specification and claims have the following meanings.

"Hydrogen", "carbon" and "oxygen" in the compounds of the present disclosure include all isotopes thereof. Isotopes are understood to include those atoms having the same atomic number but different mass numbers. For example, isotopes of hydrogen include protium, tritium and deuterium, isotopes of carbon include ¹²C, ¹³C and ¹⁴C, and isotopes of oxygen include ¹⁶O and ¹⁸O, and the like.

"Isomer" used herein refers to molecules with the same atomic composition and connection but different three-dimensional spatial arrangements, including but not limited to diastereomers, enantiomers, cis-trans isomers, and their mixtures, such as racemic mixtures. Many organic compounds exist in optically active forms, that is, they have the ability to rotate the plane of plane-polarized light. When describing an optically active compound, the prefixes D, L or R, S are used to denote the absolute configuration of the chiral center of the molecule. The prefixes D, L or (+), (-) are used to designate the compound's sign of plane-polarized light rotation, among which (-) or L means the compound is levorotatory, and the prefix (+) or D means the compound is dextrorotatory. The chemical structures of these stereoisomers are the same, but their steric structures are different. A specific stereoisomer may be an enantiomer, and a mixture of isomers is often referred to as an enantiomeric mixture. A 50:50 mixture of enantiomers is called a racemic mixture or racemate, which can result in no stereoselectivity or stereospecificity during chemical reactions. The terms "racemic mixture" and "racemate" refer to an equimolar mixture of two enantiomers, devoid of optical activity.

Depending on the choice of starting materials and process, the compounds of the present disclosure may be present as one of the possible isomers or as mixtures thereof, such as racemates and mixtures of diastereomers (depending on the number of asymmetric carbon atoms). Optically active (R)- or (S)-isomers can be prepared using chiral synthons or chiral reagents, or resolved using conventional techniques.

The resulting mixtures of any stereoisomers may be separated into pure or substantially pure geometric isomers, enantiomers, diastereomers on the basis of differences in the physicochemical properties of the components, for example, by chromatography and/or fractional crystallization.

As used herein, "halogen" means fluorine, chlorine, bromine and iodine. As used herein, "halogenated" or "halo" means substitution with fluorine, chlorine, bromine or iodine.

As used herein, "alkyl" refers to a saturated straight or branched aliphatic hydrocarbon group, preferably a straight or branched group containing 1 to 6 carbon atoms, more preferably a straight or branched group containing 1 to 3 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, and the like. Alkyl may be substituted or unsubstituted, and when substituted, the substituent can be at any available point of attachment.

As used herein, "carbonyl" and "acyl" both refer to -C(O)-.

As used herein, "sulfonyl" refers to -S(O)₂-.

As used herein, "sulfonamido" refers to -S(O)₂NH-.

As used herein, "phosphoryl" refers to -P(O)-.

As used herein, "haloalkyl" refers to alkyl substituted with at least one halogen.

As used herein, "hydroxyalkyl" refers to alkyl substituted with at least one hydroxy group.

As used herein, "alkoxy" refers to -O-alkyl. Non-limiting examples of alkoxyl include: methoxy, ethoxy, propoxy, n-propoxy, isopropoxy, isobutoxy, sec-butoxy, and the like. Alkoxyl may be optionally substituted or unsubstituted, and when substituted, the substituent can be at any available point of attachment.

As used herein, "cycloalkyl" refers to a cyclic saturated hydrocarbon group. Suitable cycloalkyl may be substituted or unsubstituted monocyclic, bicyclic or tricyclic saturated hydrocarbon groups having 3 to 12 carbon atoms, such as cyclopropyl, cyclobutyl, cyclopentyl, cyclohexyl.

As used herein, "heterocyclyl" refers to a group of a 3- to 12-membered non-aromatic ring system having 1 to 4 ring heteroatoms ("3- to 12-membered heterocyclyl"), wherein each heteroatom is independently selected from nitrogen, oxygen, sulfur, boron, phosphorus and silicon. In a heterocyclyl group containing one or more nitrogen atoms, the point of attachment may be a carbon or nitrogen atom, as long as the valence permits. Heterocyclyl groups may be either monocyclic ("monocyclic heterocyclyl") or fused, bridged, or spiro ring systems (e.g., bicyclic systems (also known as "bicyclic heterocyclyl")), and may be saturated or partially unsaturated. Suitable heterocyclyl groups include, but are not limited to, piperidinyl, azetidinyl, aziridinyl, tetrahydropyrrolyl, piperazinyl, dihydroquinazolinyl, oxiranyl, oxetanyl, tetrahydrofuranyl, tetrahydropyranyl, 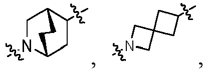 and the like. Each example of heterocyclyl may be optionally substituted or unsubstituted, and when substituted, the substituent can be at any available point of attachment.

As used herein, "aryl" refers to an aromatic system that may contain a single ring or a fused polycyclic ring, preferably an aromatic system containing a single ring or a fused bicyclic ring, which contains 6 to 12 carbon atoms, preferably about 6 to about 10 carbon atoms. Suitable aryl groups include, but are not limited to, phenyl, naphthyl, anthracenyl, fluorenyl, indanyl. Aryl may be optionally substituted or unsubstituted, and when substituted, the substituent can be at any available point of attachment.

As used herein, "heteroaryl" refers to an aryl group having at least one carbon atom substituted by a heteroatom, preferably composed of 5-12 atoms (5- to 12-membered heteroaryl), more preferably composed of 5-10 atoms (5- to 10-membered heteroaryl), wherein the heteroatom is O, S or N. The heteroaryl groups include, but are not limited to, imidazolyl, pyrrolyl, furyl, thienyl, pyrazolyl, oxazolyl, thiazolyl, isoxazolyl, isothiazolyl, oxadiazolyl, triazolyl, tetrazolyl, indolyl, pyridyl, pyrimidinyl, pyridazinyl, pyrazinyl, triazinyl, isoindolyl, benzopyrazolyl, benzimidazolyl, benzofuranyl, benzopyranyl, benzothienyl, benzoxazolyl, benzothiazolyl, benzisoxazolyl, benzisothiazolyl, quinolinyl, isoquinolinyl, quinazolinyl, cinolinyl, quinoxalinyl, benzoxazinyl, benzothiazinyl, imidazopyridyl, pyrimidopyrazolyl, pyrimidoimidazolyl and the like. Heteroaryl may be optionally substituted or unsubstituted, and when substituted, the substituent can be at any available point of attachment.

As used herein, "pharmaceutically acceptable salt" refers to salts of the compounds of the present disclosure, such salts are safe and effective when used in mammals, and have due biological activity.

As used herein, "solvate" refers in the conventional sense to a complex formed by a combination of a solute (e.g., active compound, salt of an active compound) and a solvent (e.g., water). The solvent refers to a solvent known or readily determined by those skilled in the art. In the case of water, the solvate is often referred to as a hydrate, such as hemihydrate, monohydrate, dihydrate, trihydrate, or alternative amounts thereof, and the like.

The *in vivo* effects of the compound represented by formula (I) may be exerted in part by one or more metabolites formed in humans or animals following administration of the compound represented by of formula (I). As noted above, the *in vivo* effects of the compound represented by formula (I) may also be exerted via the metabolism of the precursor compound ("prodrug"). As used herein, "prodrug" refers to a compound that is converted into the compound of the present disclosure due to reaction with enzymes, gastric acid, etc. under physiological conditions in the body, that is, a compound that is converted to the compound of the present disclosure through oxidation, reduction, hydrolysis and the like by enzymes and/or through a hydrolysis reaction by gastric acid and the like.

As used herein, "crystal" refers to a solid whose internal structure is formed by regularly repeating constituent atoms (or groups thereof) in three dimensions, which is different from an amorphous solid which does not have such a regular internal structure.

As used herein, "pharmaceutical composition" refers to a mixture comprising any one of the compounds of the present disclosure, including the corresponding isomers, prodrugs, solvates, pharmaceutically acceptable salts or chemically protected forms thereof, and one or more pharmaceutically acceptable carriers and/or one or more additional drugs. The purpose of a pharmaceutical composition is to facilitate the administration of a compound to an organism. The compositions are typically used in the manufacture of a medicament for the treatment and/or prevention of a disease mediated by one or more kinases.

As used herein, "pharmaceutically acceptable carrier" refers to a carrier that does not cause significant irritation to the organism and does not interfere with the biological activity and properties of the administered compound, including all solvents, diluents or other excipients, dispersing agents, surface active agents, isotonic agents, thickeners or emulsifiers, preservatives, solid binders, lubricants, etc., unless any conventional carrier medium is incompatible with the compounds of the present disclosure. Some examples of pharmaceutically acceptable carriers include, but are not limited to, carbohydrates such as lactose, glucose, and sucrose; starches such as corn starch and potato starch; cellulose and derivatives thereof, such as sodium carboxymethyl cellulose, and cellulose and cellulose acetate; malt, gelatin, etc.

As used herein, "excipient" refers to an inert substance added to a pharmaceutical composition to further facilitate administration of the compound. Excipients may include calcium carbonate, calcium phosphate, various sugars and types of starch, cellulose derivatives, gelatin, vegetable oils, polyethylene glycols.

As used herein, "SHP2" can be wild-type SHP2 or any mutant or variant of SHP2 that contains one or more mutations (e.g., conservative substitutions).

### DETAILED DESCRIPTION

Hereinafter, the present disclosure will be further described in detail below in conjunction with examples, but not limited to these examples. Unless otherwise specified, the materials used in the following examples are all commercially available.

### Examples

### Example 1 :

### (S)-6-amino-2-(1-amino-1,3-dihydrospiro[indene-2,4'-piperidin]-1'-yl)-3-methyl-5-((2-(tr ifluoromethyl)pyridin-3-yl)thio)pyrimidin-4(3H)-one

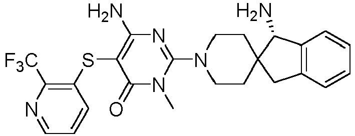

### Step 1 Synthesis of tert-butyl 1-oxo-1,3-dihydrospiro[tert-indene-2,4'-piperidine]-1'-carboxylate

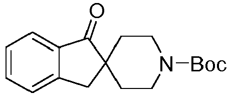

Into a 1L flask, tert-butyl N,N-bis(2-chloroethyl)carbamate (40g, 0.165mol), 1-indanone (21.84g, 0.165mol) and anhydrous N,N-dimethylformamide (400mL) were added. Under the protection of nitrogen gas, sodium hydride (16.52g, 0.41mol) was added in batches in an ice bath. The mixture was stirred in the ice bath for 1 hour, then at room temperature for 1 hour, and finally at 60°C for 8 hours. After completion of the reaction, water (20mL) was added dropwise into the reaction system in the ice bath to quench the reaction. The solvent was then removed by distillation under reduced pressure. To the residue, ethyl acetate (400ml) was added. The ethyl acetate phase was washed with water (200mL) and then saturated brine (200mL). Then the ethyl acetate phase was dried over anhydrous sodium sulfate, removed of solvent and purified with column chromatography, to obatain 13.87g of target product. ESI-MS m/z 324.2 (M+Na)⁺.

### Step 2 Synthesis of tert-butyl (R, Z)-1-((tert-butylsulfinyl)imino)-1,3-dihydrospiro[indene-2,4'-piperidine]-1'-carboxylate

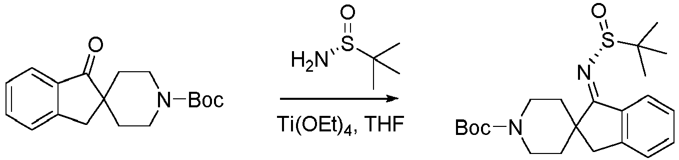

Into a 1L flask, tert-butyl 1-oxo-1,3-dihydrospiro[tert-indene-2,4'-piperidine]-1'-carboxylate (13.87g, 46.08mmol), (R)-(+)-tert-butylsulfinamide (45g, 369mmol), tetraethyl titanate (115ml, 553mmol) and anhydrous tetrahydrofuran (300mL) were added. After introducing protective nitrogen gas into the reaction flask, the mixture was stirred at 90°C for 24 hours. Afer being cooled to the room temperature, the reaction solution was poured into water (500mL). A large amount of white solid was precipitated. The insolubles were filtered off, and the filter cake was washed with ethyl acetate (100mL, three times). The filtrates were combined, and standed for layer seperation, to seperate out the organic phase. The organic phase was washed with water (200mL) and then saturated brine (200mL). Then the organic phase was dried over anhydrous sodium sulfate, removed of solvent and purified with column chromatography, to obtain 7g of the title compound. ESI-MS m/z 405.2 [M+H]⁺.

### Step 3 Synthesis of tert-butyl (S)-1-(((R)-tert-butylsulfinyl)amino)-1,3-dihydrospiro[tert-indene-2,4'-piperidine]-1'-car boxylate

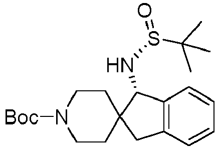

Into a 500ml flask, tert-butyl (R,Z)-1-((tert-butylsulfinyl)imino)-1,3-dihydrospiro[indene-2,4'-piperidine]-1'-carboxylate (7g, 17.3mmol) and anhydrous tetrahydrofuran (140mL) were added. After the introduction of protective nitrogen gas, a tetrahydrofuran solution of lithium borohydride (2M, 10.4mL) was added dropwise under an ice bath. After the addition, the mixture was stirred for 3 hours in an ice bath. After completion of the reaction, water (5mL) was added into the reaction system under an ice bath to quench the reaction, and tetrahydrofuran was distilled off under high pressure. After that, ethyl acetate (200mL) was added into the residual liquid. The ethyl acetate phase was washed with water (100mL, two times) and then saturated brine (100mL). Then the ethyl acetate phase was dried over anhydrous sodium sulfate, removed of solvent by distillation under high pressure, and purified with column chromatography to obtain 3g of the title compound. ESI-MS m/z 407.2 [M+H]⁺.

### Step 4 Synthesis of (R)-N-((S)-1,3-dihydrospiro[indene-2,4'-piperidine]-1-yl)-2-methylpropane-2-sulfinamid e

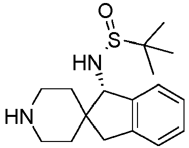

Into a 150mL flask, tert-butyl (S)-1-(((R)-tert-butylsulfinyl)amino)-1,3-dihydrospiro[tert-indene-2,4' -piperidine]-1'-carboxylate (3g, 7.39mmol) and dichloromethane (60mL) were added. At room temperature, the reaction system was added with trifluoroacetic acid (8mL, 110.8mmol) and reacted for 2 hours with stirring. The reaction system was added with a saturated sodium bicarbonate aqueous solution to adjust the pH to a weak alkaline. After the reaction system was layer separated, a dichloromethane phase was separated out, the water phase was extracted with dichloromethane (100mL), and the dichloromethane phase was combined. The combined dichloromethane phase was dried over anhydrous sodium sulfate and removed of solvent, to obtain 1.42g of the title compound. ESI-MS m/z 307.2 [M+H]⁺.

### Step5 Synthesis of (R)-N-((S)-1'-(4-amino-1-methyl-6-oxo-1,6-dihydropyrimidin-2-yl)-1,3-dihydrospiro[ind ene-2,4'-piperidin]-1-yl)-2-methylpropane-2-sulfinamide

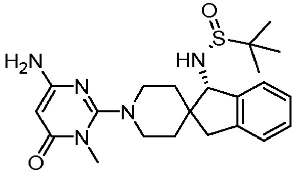

6-Amino-3-methylpyrimidine-2,4(1H,3H)-dione (280mg, 2mmol), a Carter condensing agent BOP (1.770g, 4mmol), 1,8-diazabicycloundec-7-ene (3.040g, 20mmol), (R)-N-((S)-1,3-dihydrospiro[indene-2,4'-piperidine]-1-yl)-2-methylpropane-2-sulfinamide (0.610g, 2mmol) and anhydrous N, N-dimethylformamide (10.0mL) were added into a single neck flask, to perform a reaction under the protection of argon gas at room temperature. After completion of the reaction, the mixture was extracted with water and ethyl acetate, removed of solvent, and purified with column chromatography, to obtain 790mg of the title compound. ESI-MS m/z 430.2 [M+H]⁺.

### Step 6 Synthesis of (R)-N-((S)-1'-(4-amino-5-iodo-1-methyl-6-oxo-1,6-dihydropyrimidin-2-yl)-1,3-dihydrospi ro[indene-2,4'-piperidine]-1-yl)-2-methylpropane-2-sulfinamide

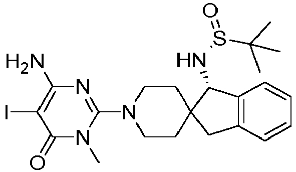

(R)-N-((S)-1'-(4-amino-1-methyl-6-oxo-1,6-dihydropyrimidin-2-yl)-1,3-dihydrospir o[indene-2,4'-piperidin]-1-yl)-2-methylpropane-2-sulfinamide (130mg, 0.3mmol), N-iodosuccinimide (75mg, 0.33mmol) and anhydrous N, N-dimethylformamide (1.0mL) were added a single neck flask, and stirred under the protection of argon gas at room temperature. After completion of the reaction, the solvent was removed, and 240 mg of the title compound was obatained. ESI-MS m/z 556.2 [M+H]⁺

### Step7 Synthesis of 2-ethylhexyl 2-(3-((2-(trifluoromethyl)pyridin-3-yl)thio)propanoate

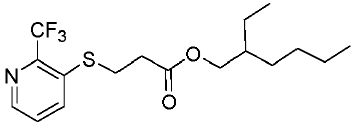

3-Bromo-2-trifluoromethylpyridine (5.000g, 22.10mmol), tris(dibenzylideneacetone)dipalladium (1.012g, 1.11mmol), 4,5-bis(diphenylphosphino)-9,9-dimethylxanthene (1.279g, 2.21mmol), 2-ethylhexyl 3-mercaptopropionate (5.308g, 24.31mmol), N,N-diisopropylethylamine (5.713g, 44.20mmol) and anhydrous 1.4-dioxane (50.0ml) were added a 250ml single neck flask, replaced with argon three times, and heated up to 110°C. The mixture was reacted overnight until the the reaction was completed as detected by LC-MS. The mixture was suction filtrated, removed of solvent by distillation under high pressure, and purified with column chromatography, to obtain 6.269g of the title compound at a yield of 78.15%. ESI-MS m/z 364.2 [M+H]⁺.

### Step 8 Synthesis of 2-(trifluoromethyl)pyridine-3-thiol

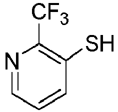

Into a 100ml flask, 2-ethylhexyl 2-(3-((2-(trifluoromethyl)pyridin-3-yl)thio)propanoate (3.074g, 8.47mmol) and anhydrous tetrahydrofuran (30.0ml) were added. The mixture was cooled at -78°C and replaced with nitrogen gas three time, and then added dropwise with a tetrahydrofuran solution of potassium tert-butoxide (1M, 25.41ml). After the addition, the reaction system was stirred for 1 hour in an ice bath, until the reaction was completed as detected by LC-MS. The mixture was removed of solvent by distillation under high pressure, dissolved into water (30ml), transferred into a separatory funnel, and added with diethyl ether (30 ml). The mixture was shaken and left to stand for layer separation and liquid separation. The water phase was added with a saturated citric acid solution (10ml) to adjust pH to a weak acidity, and extracted with ethyl acetate (30ml) for 3 times. Organic phases were combined, extracted with saturated brine (50ml), and dried over anhydrous sodium sulfate, removed of solvent by distillation under high pressure, and purified with column chromatography, to obtain 1.500g of the title compound at a yield of 98.68%. ESI-MS m/z 180.1 [M+H]⁺.

### Step 9 Synthesis of (R)-N-((S)-1'-(4-amino-1-methyl-6-oxo-5-((2-(trifluoromethyl)pyridin-3-yl)thio)-1,6-dihy dropyridin-2-yl)-1,3-dihydrospiro[indene-2,4'-piperidine]-1-yl)-2-methylpropane-2-sulfi namide

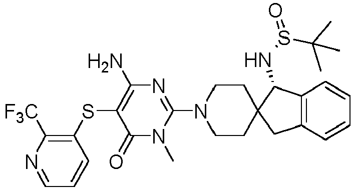

(R)-N-((S))-1'-(4-amino-5-iodo-1-methyl-6-oxo-1,6-dihydropyrimidin-2-yl)-1,3-dihy drospiro[indene-2,4'-piperidine]-1-yl)-2-methylpropane-2-sulfinamide (240mg, 0.43mmol), 2-(trifluoromethyl)pyridine-3-thiol (81mg, 0.45mmol), cuprous iodide (16mg, 0.086mmol), tetramethyldiethylamine (20mg, 0.172mmol), potassium phosphate(274mg, 1.29mmol) and anhydrous 1.4-dioxane(10mL) were added into a single neck flask, heated up to 100°C under an oxygen gas atmosphere, and stirred. After completion of the reaction, the resultant was removed of solvent and purifed with column chromatography to obtain 170mg of the title compound. MS m/z 607.3 [M+H]⁺.

### Step 10 Synthesis of (S)-6-amino-2-(1-amino-1,3-dihydrospiro[indene-2,4'-piperidin]-1'-yl)-3-methyl-5-((2-(tr ifluoromethyl)pyridin-3-yl)thio)pyrimidin-4(3H)-one

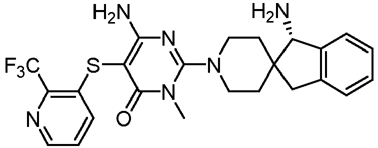

(R)-N-((S)-1'-(4-amino-1-methyl-6-oxo-5-((2-(trifluoromethyl)pyridin-3-yl)thio)-1,6 -dihydropyridin-2-yl)-1,3-dihydrospiro[indene-2,4'-piperidine]-1-yl)-2-methylpropane-2-sulfi namide (170mg, 0.28mmol), hydrogen chloride solution in 1.4-dioxane (1mL) and anhydrous methanol (1mL) were added into a single neck flask, and stirred under the protection of argon gasroom temperature. After completion of the reaction, the resultant was removed of solvent and added with ethyl acetate (20mL). The solution was basified to pH 10 with saturated sodium carbonate solution. The organic phase was separated. The aqueous phase was extracted with ethyl acetate and dried over anhydrous sodium sulfate, removed of solvent and purified with column chromatography, to obtain 12mg of the title compound. ESI-MS m/z 503.2[M+H]⁺. ¹H NMR (400 MHz, CD₃OD) δ 8.25-8.24(m, 1H), 7.44-7.42 (m, 1H), 7.34-7.27 (m, 2H), 7.11(m, 3H), 3.88(s, 1H), 3.56-3.53(m, 2H), 3.34(s, 3H), 3.18-3.04(m, 3H), 2.70-2.67 (m, 1H), 1.93-1.78 (m, 2H), 1.53-1.50 (m, 1H), 1.30-1.34(m, 1H).

### Example 2 : (S)-6-amino-2-(5-amino-5,7-dihydrospiro[cyclopentadieno[b]pyridine-6,4'-piperidine]-1' -yl)-3-methyl-5-((2--(trifluoromethyl)pyridin-3-yl)thio)pyrimidin-4(3H)-one

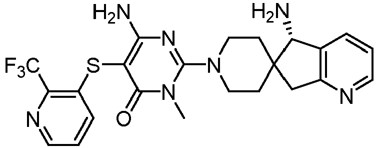

The title compound was prepared by using the same method as that in Example 1, except that the starting material 1-indanone was replaced with 6,7-dihydro-5H-cyclopentadieno [b]pyridin-5-one. ESI-MS m/z 504.2[M+H]⁺. ¹H NMR (400 MHz, CD₃OD) δ 8.6-8.7 (m, 2H), 8.45-8.40 (m, 1H), 8.10-7.96 (m, 1H), 7.60-7.52 (m, 1H), 7.41-7.38 (m, 1H), 4.5(s, 1H), 3.72-3.50 (m, 2H), 3.4-3.0 (m, 4H), 3.13-3.01 (m, 3H), 1.95-1.80 (m, 2H), 1.71-1.50 (m, 2H).

### Example 3 : (S)-6-amino-2-(1-amino-1,3-dihydrospiro[indene-2,4'-piperidine]-1'-yl)-3-isopropyl-5-((2 -(trifluoromethyl)pyridin-3-yl)thio)pyrimidin-4(3H)-one

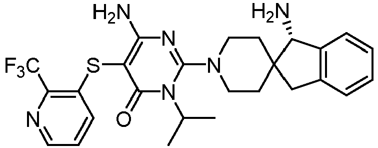

The title compound was prepared by using the same method as that in Example 1, except that intermediate 6-amino-3-methylpyrimidine-2,4(1H,3H)-dione was replaced with 6-amino-3-isopropylpyrimidine-2,4(1H,3H)-dione. ESI-MS m/z 531.2[M+H]⁺. ¹H NMR (400 MHz, CD₃OD) δ 8.24(d, 1H), 7.40 - 7.28 (m, 3H), 7.20 - 7.10 (m, 3H), 4.49-4.41 (m, 1H), 4.05 (s, 1H), 3.58 - 3.46 (m, 2H), 3.13 (t, 2H), 3.04(d, 1H), 2.81 (d, 1H), 1.91 - 1.79 (m, 2H), 1.56 - 1.42 (m, 8H).

### Example 4 : (S)-6-amino-2-(1-amino-1,3-dihydrospiro[indene-2,4'-piperidine]-1'-yl)-3-methyl-5-((6-a mino-2-chloropyridin-3-yl)thio)pyrimidin-4(3H)-one

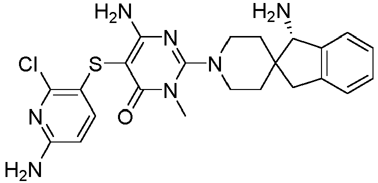

The title compound was prepared by using the same method as that in Example 1, except that intermediate 3-bromo-2-trifluoromethylpyridine was replaced with 5-bromo-6-chloropyridin-2-amine. ESI-MS m/z 483.8[M+H]⁺. ¹H NMR (400 MHz, CD₃OD) δ 7.33 (s, 1H), 7.18 (s, 1H), 6.97-6.95 (d, *J=* 8.0 Hz, 2H), 6.35-6.33 *(d, J =* 8.0 Hz, 1H), 6.20 (s, 1H), 3.88 (s, 1H), 3.28 (s, 3H), 3.03-2.99 (m, 3H), 2.67-2.60 (m, 1H), 1.99-1.78 (m, 3H), 1.54-1.51 (d, *J=* 8.0 Hz, 1H), 1.24(s, 2H).

### Example 5 : (S)-6-amino-2-(1-amino-1,3-dihydrospiro[indene-2,4'-piperidine]-1'-yl)-3-methyl-5-((3-c yano -2-fluorophenyl)thio)pyrimidin-4(3H)-one

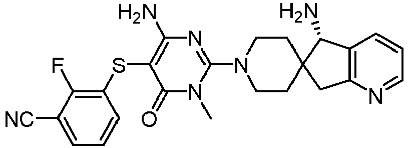

The title compound was prepared by using the same method as that of Example 2, except that 3-bromo-2-trifluoromethylpyridine was replaced with 3-bromo-2-fluorobenzonitrile. ESI-MS m/z 478.2[M+H]⁺. ¹H NMR (400 MHz, CD₃OD) *δ* 8.31 (s, 1H), 7.68-7.61 (m, 2H), 7.29-7.25 (m, 1H), 7.19-7.10 (m, 1H), 3.92 (s, 1H), 3.56-3.33 (m, 2H), 3.29 (s, 3H), 3.07-3.05 (d, *J* = 8.0 Hz, 2H), 2.75-2.71 (d, *J* = 16.0 Hz, 2H), 1.92-1.83 (m, 3H), 1.58-1.54(d, *J=* 16.0 Hz, 1H), 1.19-1.15 (d, *J=* 16.0 Hz, 1H).

### Example 6 : (S)-6-amino-2-(1-amino-1,3-dihydrospiro[indene-2,4'-piperidine]-1'-yl)-3-methyl-5-((2-methylpyridin-3-yl)thio)pyrimidin-4(3H)-one

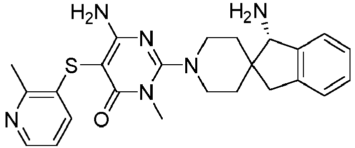

The title compound was prepared by using the same method as that in Example 1, except that 3-bromo-2-trifluoromethylpyridine in the example was replaced with 3-bromo-2-methylpyridine. ESI-MS m/z 448.9[M+H]⁺. ¹H NMR (400 MHz, CD₃OD) *δ* 8.13 (s, 1H), 7.36-7.31 (m, 1H), 7.18-7.16 (d, *J* = 8.0 Hz, 3H), 7.09-7.101 (m, 2H), 3.87 (s, 1H), 3.56-3.48 (m, 2H), 3.30 (s, 3H), 3.08-2.94(m, 3H), 2.63-2.59 (d, *J =* 16.0 Hz, 1H), 2.50 (s, 3H), 1.92-1.76 (m, 2H), 1.55-1.52 (d, *J=* 12.0 Hz, 1H), 1.10 (s, 1H).

### Example 7 : (S)-6-amino-2-(1-amino-1,3-dihydrospiro[indene-2,4'-piperidine]-1'-yl)-3-methyl-5-((4-(t rifluoromethyl)pyrimidin-5-yl)thio)pyrimidin-4(3H)-one

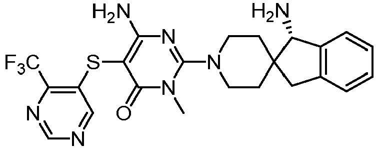

The title compound was prepared by using the same method as that in Example 1, except that 3-bromo-2-trifluoromethylpyridine in Example 1 was replaced with 5-bromo-4-(trifluoromethyl)pyrimidine. ESI-MS m/z 504.1[M+H]⁺. ¹H NMR (400 MHz, CD₃OD) δ 9.09 (s, 1H), 8.35-8.45 (m, 1H), 7.29-7.38 (m, 1H), 7.20 (m, 3H), 3.94(s, 1H), 3.45-3.60 (m, 2H), 3.20-3.35 (m, 3H), 3.11-3.02 (m, 3H), 2.68-2.64(d, *J =* 16.0 Hz, 1H), 1.92-1.77 (m, 2H), 1.55-1.52 (m, *J=* 12.0 Hz, 1H), 1.22-1.19 (d, *J=* 12.0 Hz, 1H).

### Example 8 : (S)-6-amino-2-(1-amino-1,3-dihydrospiro[indene-2,4'-piperidine]-1'-yl)-3-methyl-5-((2-(t rifluoromethyl)pyridin-3-yl)thio)pyrimidin-4(3H)-one

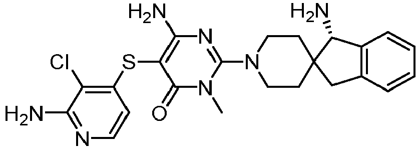

The title compound was prepared by using the same method as that in Example 1, except that 3-bromo-2-trifluoromethylpyridine was replaced with 3-chloro-4-iodopyridin-2-amine. ESI-MS m/z 484.2[M+H]+. ¹H NMR (400 MHz, CD₃OD) *δ* 7.60-7.65 (m, 1H), 7.35-7.30 (m, 1H), 7.25-7.15 (m, 2H), 6.20-6.10 (m, 1H), 5.80-6.00 (m, 1H), 3.88 (s, 1H), 3.28 (s, 3H), 3.10-3.00 (m, 3H), 2.60-2.65 (m, 1H), 1.85-1.75 (m, 2H), 1.51-1.54(m, 2H), 1.24-1.20 (m, 2H).

### Example 9 : (S)-3-((4-amino-2-(1-amino-1,3-dihydrospiro[indene-2,4'-piperidine]-1'-yl)-1-methyl-6-o xo-1,6-dihydropyrimidin-5-yl)thio)-2-chlorobenzonitrile

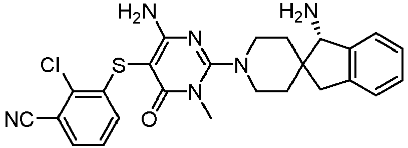

The title compound was prepared by using the same method as that in Example 1, except that 3-bromo-2-trifluoromethylpyridine was replaced with 3-bromo-2-chlorobenzonitrile. ESI-MS m/z 493.2[M+H]⁺. ¹H NMR (400 MHz, CD₃OD) δ 7.60-7.65 (m, 1H), 7.41-7.30 (m, 2H), 7.25-7.15 (m, 2H), 7.07-7.00 (m, 2H), 3.85 (s, 1H), 3.30 (s, 3H), 3.10-3.00 (m, 3H), 2.60-2.65 (m, 1H), 1.85-1.75 (m, 2H), 1.50-1.60 (m, 2H), 1.15-1.30 (m, 2H).

### Example 10 : (S)-3-((4-amino-2-(1-amino-1,3-dihydrospiro[indene-2,4'-piperidin]-1'-yl)-1-methyl-6-ox o-1,6-dihydropyrimidin-5-yl)thio)-N-methylbenzenesulfonamide

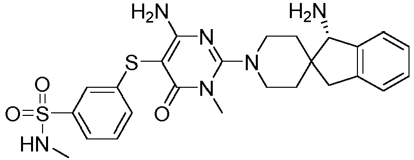

The title compound was prepared by using the same method as that in Example 1, except that 3-bromo-2-trifluoromethylpyridine was replaced with 3-bromo-N-methylbenzenesulfonamide. ESI-MS m/z 527.2[M+H]⁺. ¹H NMR (400 MHz, CD₃OD) δ 7.61-7.10 (m, 5H), 7.15-7.00 (m, 2H), 7.0-6.5 (m, 1H), 3.88 (s, 1H), 3.40 (s,3H), 3.31 (s, 3H), 3.15-3.00 (m, 3H), 2.55-2.65 (m, 1H), 1.85-1.65 (m, 2H), 1.45-1.56 (m, 2H), 1.20-1.30 (m, 2H).

### Example 11 : (S)-6-amino-2-(1-amino-1,3-dihydrospiro[indene-2,4'-piperidin]-1'-yl)-5-(((2-amino-5-ch loropyridin-4-yl)thio)-3-methylpyrimidin-4(3H)-one

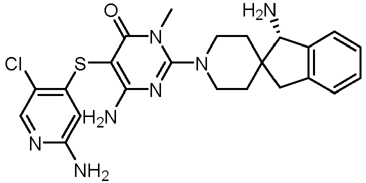

The title compound was prepared by using the same method as that in Example 1, except that 3-bromo-2-trifluoromethylpyridine was replaced with 4-bromo-5-chloropyridin-2-amine. ESI-MS m/z 484.2[M+H]⁺. ¹H NMR (400 MHz, CD₃OD) *δ* 7.80-7.70 (m, 1H), 7.40-7.30 (m, 1H), 7.25-7.15 (m, 2H), 6.20-6.10 (m, 1H), 5.85-6.03 (m, 1H), 3.88 (s, 1H), 3.28 (s, 3H), 3.10-3.00 (m, 3H), 2.60-2.65 (m, 1H), 1.85-1.75 (m, 2H), 1.51-1.54(m, 2H), 1.24-1.20 (m, 2H).

### Example 12 : Synthesis of (S)-6-amino-2-(1-amino-1,3-dihydrospiro[indene-2,4'-piperidin]-1'-yl)-5-((3-(dimethylph osphoryl)phenyl]thio)-3-methylpyrimidin-4(3H)-one

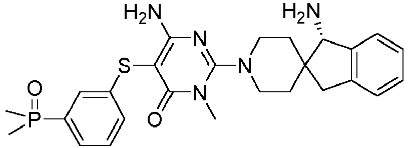

The title compound was prepared by using the same method as that in Example 1, except that 3-bromo-2-trifluoromethylpyridine was replaced with (3-bromophenyl)dimethylphosphine oxide. ESI-MS m/z 510.2[M+H]⁺. ¹H NMR (400 MHz, CD₃OD) δ 7.61-7.30 (m, 5H), 7.15-7.00 (m, 3H), 3.88 (s, 1H), 3.40 (s,3H), 3.31 (s, 3H), 3.15-3.00 (m, 3H), 2.55-2.65 (m, 1H), 2.10-1.85 (m, 2H), 1.45-1.56 (m, 2H), 1.35(s, 6H), 1.00-0.89 (m, 2H).

### Example 13 : Synthesis of (S)-6-amino-2-(1-amino-1,3-dihydrospiro[indene-2,4'-piperidine]-1'-yl)-3-methyl-5-((2-( methylsulfonyl)phenyl)thio)pyrimidin-4(3H)-one

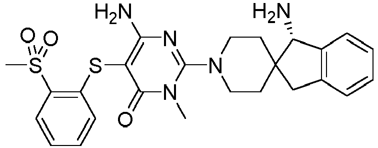

The title compound was prepared by using the same method as that in Example 1, except that 3-bromo-2-trifluoromethylpyridine was replaced with 1-bromo-2-(methylsulfonyl)benzene. ESI-MS m/z 512.2[M+H]⁺. ¹H NMR (400 MHz, CD₃OD) δ 7.65-7.60 (m, 1H), 7.60-7.53 (m, 1H), 7.40-7.30 (m, 3H), 7.10-7.20 (m, 3H), 4.00 (s, 1H), 3.38 (s, 3H), 3.10-3.00 (m, 3H), 2.95 (s, 3H), 2.75-2.65 (m, 1H), 2.10-1.85 (m, 2H), 1.60-1.45 (m, 2H), 1.30-1.10(m, 2H).

### Example 14 : (S)-6-amino-2-(1-amino-1,3-dihydrospiro[indene-2,4'-piperidin]-1'-yl)-3-methyl-5-((3-(m ethylsulfonyl) phenyl)thio)pyrimidin-4(3H)-one

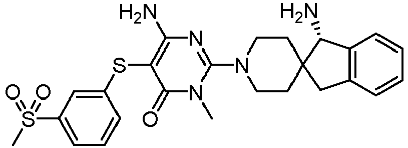

The title compound was prepared by using the same method as that in Example 1, except that 3-bromo-2-trifluoromethylpyridine was replaced with 1-bromo-3-(methylsulfonyl)benzene. ESI-MS m/z 512.2[M+H]⁺. ¹H NMR (400 MHz, CD₃OD) δ 8.00-7.90 (m, 1H), 7.38-7.24(m, 3H), 7.24-7.12 (m, 4H), 4.00 (s, 1H), 3.38 (s, 3H), 3.35 (s, 3H), 3.10-2.95 (m, 3H), 2.70-2.61 (m, 1H), 2.10-1.85 (m, 2H), 1.60-1.45 (m, 2H), 1.30-1.10(m, 2H).

### Example 15 : (S)-2-((4-amino-2-(1-amino-1,3-dihydrospiro[indene-2,4'-piperidine]-1'-yl)-1-methyl-6-o xo-1,6-dihydropyrimidin-5-yl)thio)-N-methylbenzenesulfonamide

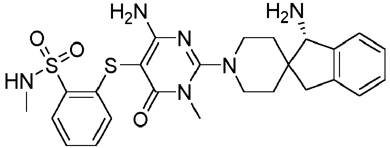

The title compound was prepared by using the same method as that in Example 1, except that 3-bromo-2-trifluoromethylpyridine was replaced with 2-bromo-N-methylbenzenesulfonamide. ESI-MS m/z 527.2[M+H]+. ¹H NMR (400 MHz, CD₃OD) δ 8.38-8.50 (m, 2H), 8.05-7.85 (m, 1H), 7.31-7.70 (m, 5H), 4.00 (s, 1H), 3.35 (s, 3H), 3.10-2.95 (m, 3H), 2.70-2.61 (m, 1H), 2.52(s, 3H), 2.10-1.85 (m, 2H), 1.60-1.45 (m, 2H), 1.30-1.10(m, 2H).

### Example 16 : (S)-3-((4-amino-2-(5-amino-5,7-dihydrospiro[cyclopenta[b]pyridine-6,4'-piperidine]-1'-y 1)-1-methyl-6-oxo-1,6-dihydropyrimidin-5-yl)thio)-2-chlorobenzonitrile

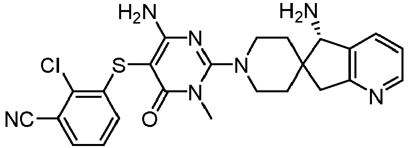

The title compound was prepared by using the same method as that of Example 2, except that 3-bromo-2-trifluoromethylpyridine was replaced with 3-bromo-2-chlorobenzonitrile. ESI-MS m/z 494.1[M+H]⁺. ¹H NMR (400 MHz, CD₃OD) δ 8.50 (s, 1H), 8.00-7.90 (m, 1H), 7.71-7.65 (m, 1H), 7.41-7.35(m, 1H), 7.35-7.30(m, 1H), 7.00-7.10(m, 1H), 4.45 (s, 1H), 3.71-3.60(m, 2H), 3.35 (s, 3H), 3.25-2.96 (m, 4H), 2.00-1.82(m, 2H), 1.62-1.51(m, 2H).

### Example 17 : (S)-N-(4-((4-amino-2-(1-amino-1,3-dihydrospiro[indene-2,4'-piperidine]-1'-yl)-1-methyl-6-oxo-1,6-dihydropyrimidin-5-yl)thio)-3-chloropyridin-2-yl)cyclopropanecarboxamide

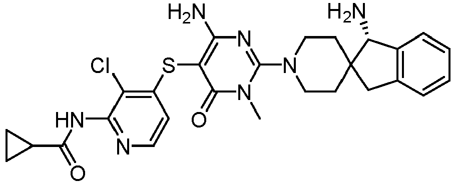

The title compound was prepared by using the same method as that in Example 1, except that 3-bromo-2-trifluoromethylpyridine was replaced with N-(4-bromo-3-chloropyridin-2-yl) cyclopropanecarboxamide. ESI-MS m/z 552.2[M+H]⁺. ¹H NMR (400 MHz, d6-DMSO) δ 10.45 - 10.28 (s, 1H), 8.16 - 8.03 (m, 1H), 7.41-7.76 (m, 2H), 7.41 - 7.12 (m, 4H), 6.97 - 6.71 (m, 2H), 6.71-6.65 (m, 1H), 3.95 - 3.84(s, 1H), 3.52(s, 3H), 3.08 - 2.93 (m, 4H), 2.65-2.51 (m,3H), 2.23 - 2.09 (m, 2H), 1.98 - 1.73 (m, 3H), 1.61 - 1.46 (m, 1H), 1.28 - 1.08 (m, 2H).

### Example 18 : (S)-6-amino-2-(1-amino-1,3-dihydrospiro[indene-2,4'-piperidine]-1'-yl)-5-((2-(difluorom ethyl)pyridine-4-yl)thio)-3-methylpyrimidin-4(3H)-one

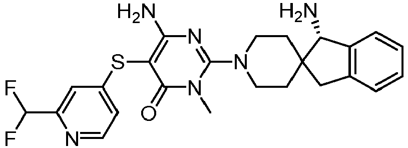

The title compound was prepared by using the same method as that in Example 1, except that 3-bromo-2-trifluoromethylpyridine was replaced with 4-bromo-2-(difluoromethyl)pyridine. ESI-MS m/z 484.9 [M+H]+. ¹H NMR (400 MHz, *d*6-DMSO) δ 8.42 (dd, *J =* 16.6, 1H), 7.32 (m, 2H), 7.17 (m, 4H), 6.65-7.04(m, 3H), 3.91 - 3.81 (s, 1H), 3.54(dd, *J* = 29.3, 14.1 Hz, 2H), 3.31 (s, 3H), 2.96-3.16 (m, 2H), 2.66 - 2.57 (m, 1H), 1.96 - 1.85 (m, 1H), 1.85 - 1.74(m, 1H), 1.55 (d, *J* = 12.8 Hz, 1H), 1.24(m, 1H), 1.15 (d, *J* = 12.8 Hz, 1H).

According to the synethsis method of Example 1 of the present disclosure, the compound of examples 19-41 was prepared by using different commercially available raw materials. The characterization parameters of theses compounds are set forth in Table 1.

**Table 1**

| Ex. | Structure of compound | ESI-MS m/z [M+H]⁺ | Ex. | Structure of compound | ESI-MS m/z [M+H]⁺ |
|---|---|---|---|---|---|
| 19 | 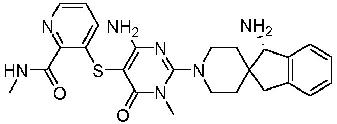 | 492.2 | 32 | 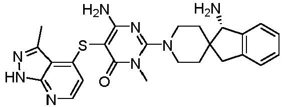 | 489.2 |
| 20 | 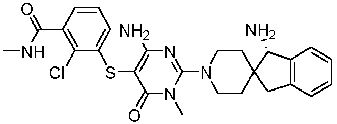 | 525.2 | 33 | 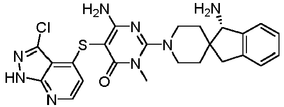 | 509.2 |
| 21 | 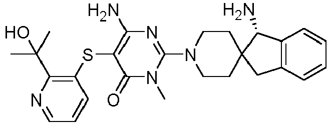 | 493.2 | 34 | 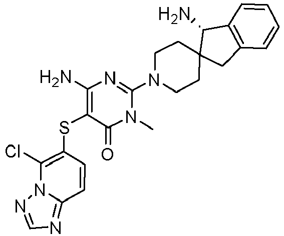 | 509.2 |
| 22 | 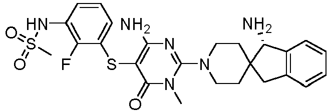 | 545.1 | 35 | 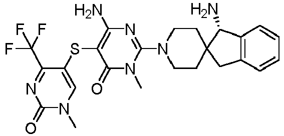 | 534.2 |
| 23 | 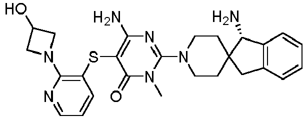 | 506.2 | 36 | 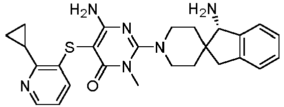 | 475.3 |
| 24 | 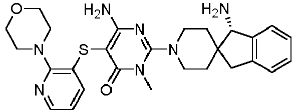 | 520.2 | 37 | 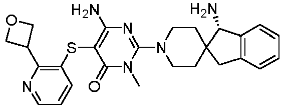 | 491.2 |
| 25 | 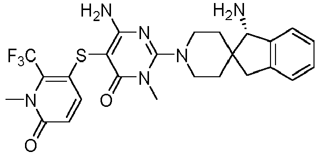 | 533.2 | 38 | 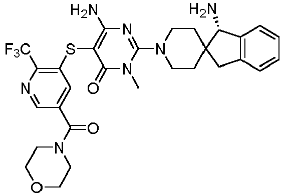 | 616.3 |
| 26 | 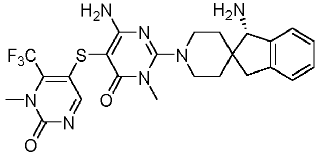 | 534.2 | 39 | 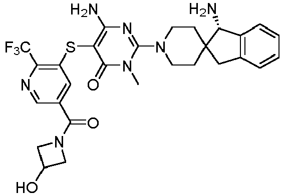 | 602.2 |
| 27 | 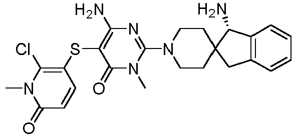 | 499.1 | 40 | 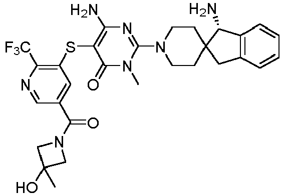 | 616.3 |
| 28 | 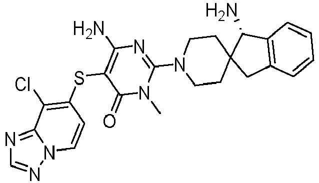 | 509.2 | 41 | 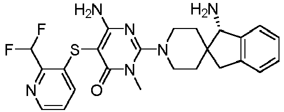 | 485.2 |
| 29 | 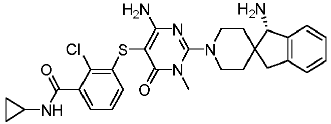 | 551.2 | 42 | 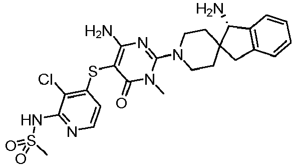 | 562.1 |
| 30 | 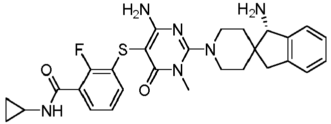 | 535.3 | 43 | 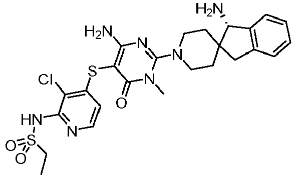 | 576.1 |
| 31 | 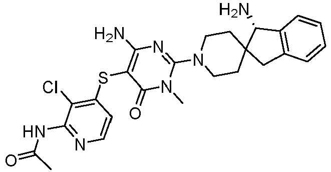 | 526.1 | 44 | 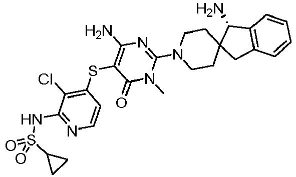 | 588.1 |

### Example 45 : (R)-1'-(8-(((2-amino-3-chloropyridin-4-yl)thio)imidazo[1,2-c[pyrimidin-5-yl)-3H-spiro[1-benzofuran-2,4-piperidine]-3-amine

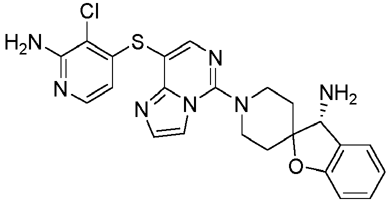

### Step 1: Synthesis of 2-(2-fluorophenyl)-1,3-dithiane

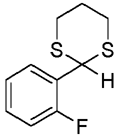

In a 1000mL three-necked flask, 2-fluorobenzaldehyde (20.0g, 161.15mmol) was dissolved in dichloromethane (340mL). At room temperature and under protection of nitrogen gas, the mixture was added with propanethiol (16mL, 159.54mmol) and iodine (1.2g, 4.83mmol) to perform a reaction at room temperature for18 h. After completion of the reaction, the reaction solution was poured into sodium thiosulfate aqueous solution (180mL, 0.4 M), and then poured into sodium hydroxide aqueous solution (150mL, 40%). The mixture was stirred for 10 min, extracted with dichloromethane (500M1) for three times, washed with saturated brine once, dried over anhydrous sodium sulfate, concentrated and then seperated with column chromatography, to obtain 19.3g of a white solid with a yield of 56%. ESI-MS m/z: 215.1[M+H]⁺.

### Step 2: Synthesis of tert-butyl 4-(2-(2-fluorophenyl)-1,3-dithio-2-yl)-4-hydroxypiperidine-1-carboxylate

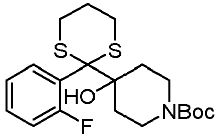

In a 1L three-neck flask, 2-(2-fluorophenyl)-1,3-dithiane (19.3g, 90.25mmol) was dissolved in anhydrous tetrahydrofuran (250mL). Under protection of nitrogen gas, the reaction system was cooled to -20°C, and slowly added dropwise with lithium diisopropylamide (45mL, 90.25mmol). After the addition, the reaction was continued for 30 min. Then, the reaction system was cooled to -78°C. Tert-butyl 4-oxopiperidine-1-carboxylate dissolved in 180mL of anhydrous tetrahydrofuran was added dropwise into the reaction system. After completion of the reaction, saturated ammonium chloride aqueous solution was added to quench the reaction. The resultant was exracted with ethyl acetate (300mL) for 3 times. Organic phases were combined, washed with saturated brine once. The resluting organic phase was dried, concentrated and then seperated by column chromatography, to obtain a white-like solid 27.5g with a yield of 74%, ESI-MS m/z: 414.0 [M+H]⁺.

### Step 3: Synthesis of tert-butyl 4-(2-fluorobenzoyl)-4-hydroxypiperidine-1-carboxylate

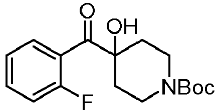

In a 100mL single neck flask, tert-butyl 4-(2-(2-fluorophenyl)-1,3-dithio-2-yl)-4-hydroxypiperidine-1-carboxylate (3.9g, 9.43mmol) were dissolved in dichloromethane (60mL), and then pyridine (1.1mL, 14.05mmol), water (10mL), pyridinium tribromide (4.5g, 1.49mmol) and tetrabutylammonium bromide (0.3g, 0.94mmol) were added. The mixture was stirred at room temperature for 24 h. After completion of the reaction, the reaction solution was poured into 250mL of water, stirred for 10 min at room temperature, exracted with dichloromethane (200mL) for 3 times. The organic phase was backwashed once with saturated sodium chloride aqueous solution. The resulting organic phase was dried, concentrated and then seperated by column chromatography, to obtain 2.91g of a yellow crude product with a yield of 90%. ESI-MS m/z: 324.2 [M+H]⁺.

### Step 4: Synthesis of tert-butyl 3-oxo-3H-spiro[benzofuran-2,4'-piperidine]-1'-carboxylate

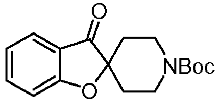

In a 500ml single neck flask, tert-butyl 4-(2-fluorobenzoyl)-4-hydroxypiperidine-1-carboxylate (20.4g, 63.08mmol) was dissolved in anhydrous tetrahydrofuran (200mL), and potassium tert-butoxide (10.6g, 94.7mmol) was added. Under protection of nitrogen gas, the mixture was heated up to 70°C to perform a reaction for 2h. After completion of the reaction, the reaction solution was poured into 800mL of water, stirred for 10 min at room temperature, and exracted with ethyl acetate (500mL) for 3 times. The resulting organic phase was washed with saturated brine once, dried over anhydrous sodium sulfate, concentrated, and seperated by column chromatography, to obtain 10.7g of a white solid with a yield of 70%. ESI-MS m/z: 304.1 [M+H]⁺.

### Step 5: Synthesis of tert-butyl (R,Z)-3-((tert-butylsulfinyl)imino)-3H-spiro[benzofuran-2,4'-piperidine]-1'-carboxylate

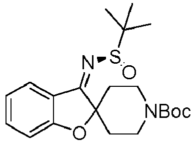

In a 500mL single neck flask, tert-butyl 3-oxo-3H-spiro[benzofuran-2,4'-piperidine]-1'-carboxylate (10.7g, 35.27mmol) was dissolved in anhydrous 2-methyltetrahydrofuran (100mL), and R-tert-butylsulfinamide (12.82g, 105.8mmol) and titanium tetraethoxide (37mL, 176.35mmol) were added. Under protection of nitrogen gas, the mixture was heated up to 90°C to perform a reaction overnight. After completion of the reaction, the reaction solution was poured into water (800mL) and then poured into ethyl acetate (800mL). The mixture was stirred at room temperature for 15min, and filtrated with diatomite. The filtrate was washed with saturated sodium chloride aqueous solution once, dired, concentrated, and seperated with column chromatography, to obtain 9.17g of title product with a yield of 97%. ESI-MS m/z: 407.1 [M+H]⁺.

### Step 6: Synthesis of tert-butyl (R)-3-(((R)-(tert-butylsulfinyl)amino]-3H-spiro[benzofuran-2,4'-piperidine]-1'-carboxyl ate

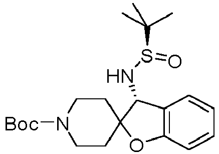

In a 500ml three neck flask, tert-butyl (R,Z)-3-((tert-butylsulfinyl)imino)-3H-spiro[benzofuran-2,4'-piperidine]-1'-carboxylate (9.17g, 22.56mmol) was dissolved in anhydrous tetrahydrofuran (100mL). The mixture was cooled to 0°C, and added with lithium borohydride (2.0M, 18mL, 35.43mmol), to perform a reaction at 0°C for 1h. After completion of the reaction, the reaction solution was added slowly with saturated ammonium chloride aqueous solution. The mixture was extracted with 300mL of ethyl acetate for 3 times, washed with saturated sodium chloride aqueous solution

once, dried, and concentrated, and seprated by column chromatography, to obtain 6.1g of title product with a total yield of 70%. ESI-MS m/z: 409.1 [M+H]⁺.

### Step 7: Synthesis of (R)-2-methyl-N-((R)-3H-spiro[benzofuran-2,4'-piperidine]-3-yl)propane-2-sulfinamide

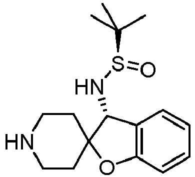

In a 100mL single neck flask, tert-butyl (R)-3-(((R)-(tert-butylsulfinyl)amino]-3H-spiro[benzofuran-2,4'-piperidine]-1'-carboxylate (0.25g, 0.61mmol) was dissolved in anhydrous dichloromethane (10mL), and trifluoroacetic acid (0.5mL) was added. to perform a reaction at room temperature for 1 h. After completion of the reaction, saturated sodium bicarbonate solution was added until pH was 7-8. The resultant was extracted with 60mL of ethyl acetate. The organic phase was washed with saturated brine, and concentrated to obtain the title product. ESI-MS m/z: 309.1 [M+H]⁺.

### Step 8: Synthesis of 5-bromo-2-chloro-N-(2,2-dimethoxyethyl)pyrimidin-4-amine

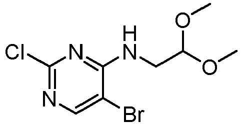

5-Bromo-2,4-dichloropyrimidine (15g, 66mmol) and 150mL of ethanol were added into a 250mL three neck flask and cooled to 0°C. Under the protection of argon gas. The mixture was added with 2,2-dimethoxyethane-1-amine (13.94g, 132.8mmol), and stirred for 10 min, and added with triethylamine (13.4g, 132.8mmol) to perform a reaction overnight at room temperature. After completion of the reaction, the resultant was concentrated to remove ethanol, and added with 300mL of ethyl acetate. The mixture was washed with water twice, washed with saturated sodium chloride solution, concentrated and dried, to obtain a white solid 20.1g with a yield of 64%. ESI-MS m/z: 296.2 [M+H]+.

**Step 9: Preparation of 8-bromoimidazo[1,2-c]pyrimidin-5-ol**

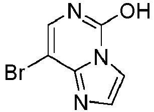

5-Bromo-2-chloro-N-(2,2-dimethoxyethyl) pyrimidin-4-amine (5g, 16.86mmol) was added into a 250ml single neck flask, and concentrated sulfuric acid (40ml) was slowly added under an ice bath. After the addition, the mixture was reacted at 65°C for 2h. After the reaction was completed as detected by LC-MS and TLC, the reaction solution was poured into ice water, adjusted to a pH of about 6 with saturated sodium bicarbonate solution, and extracted with dichloromethane/ethanol (10:1) 500mL for 3 times. The organic phase was washed with saturated brine once, dried over anhydrous sodium sulfate, and concentrated to obtain 1.5g of a yellow solid product with a yield of 41%. MS (ESI) m/z: 214.2/216.2[M+H]⁺.

### Step 10: Preparation of 8-bromo-5-chloroimidazo[1,2-c]pyrimidine

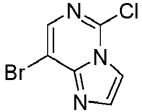

8-Bromoimidazo[1,2-c]pyrimidin-5-ol (750mg, 3.50mmol) was added into a 100ml single neck flask, and then phosphorus oxychloride (13ml, 140mmol) and N,N-diisopropylethylamine (0.5ml) were added. The mixture was reacted at 110°C for 3h under protection of nitrogen gas. After completion of the reaction, the reaction solution was concentrated, adjusted to a pH of about 6 with saturated sodium bicarbonate solution, and exracted with ethyl acetate for 3 times. Organic phases were combined, washed with saturated brine once, dried over anhydrous sodium sulfate and concentrated to obtain 0.5g of a yellow-brown solid product with a yield of 61%, MS (ESI) m/z: 232.2[M+H]⁺.

### Step 11 : Preparation of (R)-N-((R)-1'-(8-bromoimidazo[1,2-c]pyrimidin-5-yl)-3H-spiro[benzofuran-2,4'-piperidi ne]-3-yl))-2-methylpropane-2-sulfinamide

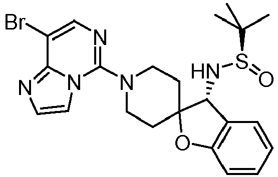

In a 100mL single neck flask, 8-bromo-5-chloroimidazo[1,2-c]pyrimidine (150mg, 0.645mmol) was dissolved in anhydrous N,N-dimethylformamide (15ml), and (R)-2-methyl-N-((R)-3H-spiro[benzofuran-2,4'-piperidine]-3-yl)propane-2-sulfinamide (199mg, 0.645mmol) and N,N-diisopropylethylamine (0.533ml, 3.23mmol) were added. The mixture was reacted at 110°C for 1h. After the reaction was completed as detected by LC-MS and TLC, 100mL of ethyl acetate was added. The water phase was exracted with for 3 times. The organic phase was washed with saturated brine for three times, dried over anhydrous sodium sulfate, concentrated, and subjected to column chromatography, to obtain 50mg of a product with a yield of 15%, ESI-MS m/z: 504.2/506.2 [M+H]⁺.

### Step 12: 2-ethylhexyl 2-(2-(3-amino-3-chloropyridin-4-yl)thio)propanoate

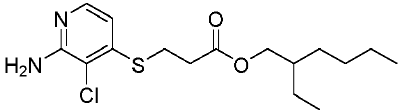

3-Chloro-4-iodopyridin-2-amine (5g, 19.65mmol), 2-ethylhexyl 3-mercaptopropionate (5.4ml, 23.58mmol), tris(dibenzylideneacetone)dipalladium (1.1g, 1.18mmol), 4,5-bis(diphenylphosphino)-9,9-dimethylxanthene (1.1g, 1.97mmol), N,N-diisopropylethylamine (6.5ml, 39.3mmol) were dissolved in 50ml of dioxane. The mixture was reacted under protection of nitrogen gas at 110°C for 2h. After completion of the reaction, the resultant was filtered with diatomite. The filtrate was concentrated, and purified with a column (ethyl acetate/petroleum ether 30%-50%) to obtain 6.64g of a white-like solid with a yield of 98%. MS (ESI) m/z: 345.0[M+H]+.

### Step 13: Sodium salt of 2-amino-3-chloropyridine-4-thiol

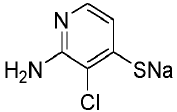

2-Ethylhexyl 2-(2-(3-amino-3-chloropyridin-4-yl)thio)propanoate (0.5g, 1.45mmol) was dissolved in 10ml of anhydrous tetrahydrofuran, and added dropwise with sodium ethoxide (20%wt. 0.5ml, 1.52mmol) under protection of nitrogen gas at room temperature. After 40min of reaction, 15ml of dichloromethane was added to the reaction system. A large amount of precipitation was precipitated. The resultant was ultrasonicated for 5min and filtered. The filter cake was washed with dichloromethane, and then dried, to obtain 0.26g of a white-like solid with a yield of 80%. MS (ESI) m/z: 161.1 [M+H]+.

### Step 14 : Preparation of (R)-N-((R)-1'-(8-((2-amino-3-chloropyridin-4-yl)-thio)imidazo[1,2-c[pyrimidin-5-yl)-3H-spiro[benzofuran-2,4'-piperidine]-3-yl)-2-methylpropane-2-sulfinamide

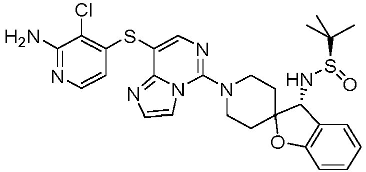

In a 100ml single neck flask, (R)-N-((R)-1'-(8-bromoimidazo[1,2-c]pyrimidin-5-yl)-3H-spiro[benzofuran-2,4'-piperidine]-3-yl))-2-methylpropane-2-sulfinamide (50mg, 0.099mmol) was dissolved in 1,4-dioxane (10ml), and then 2-amino-3-chloropyridine-4-thiol (30mg, 0.198mmol), 4,5-bis(diphenylphosphino)-9,9-dimethylxanthene (30mg, 0.059mmol), tris(dibenzylideneacetone)dipalladium (30mg, 0.029mmol) and N,N-diisopropylethylamine (0.5ml, 0.297mmol) were added. The mixture was reacted at 100°C for 1h. After the reaction was completed as detected by LC-MS and TLC, the reaction solution was concentrated, and purified by column chromatography, to obtain 50mg of a product with a yield of 86%. ESI-MS m/z: 584.1 [M+H]+.

### Step 15 : Preparation of (R)-1'-(8-(((2-amino-3-chloropyridin-4-yl)thio)imidazo[1,2-c]pyrimidin-5-yl)-3H-spiro[1-benzofuran-2,4-piperidine]-3-amine

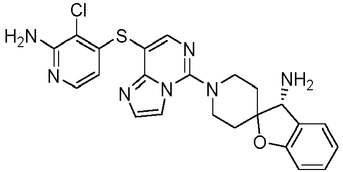

In a 100mL reaction flask, (R)-N-((R)-1'-(8-((2-amino-3-chloropyridin-4-yl)-thio)imidazo[1,2-c]pyrimidin-5-yl)-3H-spir o[benzofuran-2,4'-piperidine]-3-yl)-2-methylpropane-2-sulfinamide (50mg, 0.085mmol) was dissolved in 1,4-dioxane (15ml), and then hydrochloric acid/dioxane solution (3ml) was slowly added dropwise. After the addition, the mixture was reacted for 1 hour. After the reaction was completed as detected by LC-MS and TLC, the reaction solution was concentrated and seperated by preparative HPLC, to obtain 9mg of a white solid product with a yield of 21%. ¹H NMR (400 MHz, DMSO) δ 8.05 (s, 1H), 7.92 (s, 1H), 7.64-7.50 (m, 2H), 7.43 (d, J = 7.2 Hz, 1H), 7.29-7.13 (m, 2H), 6.94(t, J = 7.3 Hz, 1H), 6.87 (d, J = 7.9 Hz, 1H), 6.33 (s, 2H), 5.83 (d, J = 5.2 Hz, 1H), 5.33 (s, 1H), 4.33 (s, 1H), 4.05 (d, J = 13.3 Hz, 1H), 3.96 (d, J = 13.2 Hz, 1H), 3.53 (d, J = 12.6 Hz, 2H), 1.40-1.30 (m, 2H), 0.85 (d, J = 6.8 Hz, 2H). ESI-MS m/z: 480.2 [M+H]+.

### Example 46 : (S)-1'-(8-((2-amino-3-chloropyridin-4-yl)thio)imidazo[1,2-c]pyrimidin-5-yl)-5,7-dihydros piro[cyclopenta[b]pyridine-6,4'-piperidine]-5-amine

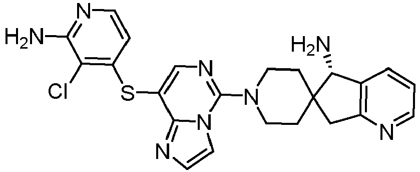

### Step 1: Preparation of 2,2'-(benzylazadiyl)bis(ethane-1-ol)

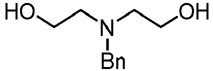

Diethanolamine (10g, 95mmol) was dissolved in 150mL of acetonitrile, and then benzyl bromide (19.5g, 114mmol) and potassium carbonate (25.8g, 190mmol) were added. The mixture was reacted at 90°C overnight, until the raw material was reactecd completely as detected by TLC. The resultant was concentrated, extracted with water and ethyl acetate, and washed with saturated sodium chloride solution. The organic layer were collected and dried with anhydrous sodium sulfate, and purified by column chromatography, to obtain 15.8g of light yellow oil with a yield of 85%. LC-MS m/z: 196 [M+H]+.

### Step 2: Preparation of N-benzyl-2-bromo-N-(2-bromoethyl)ethan-1-amine

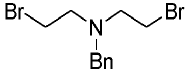

2,2'-(benzylazadiyl)bis(ethan-1-ol) (0.8g, 4.1mmol) was dissolved in 10mL of toluene, and then phosphorus tribromide (2.2 g) was added dropwise. The mixture was reacted at 100°C under reflux overnight. After the reaction was completed as detected by TLC, the resultant was concentrated to remove toluene, adjusted to a pH of 9 with saturated sodium bicarbonate solution, and extracted with ethyl acetate, to obtain an organic phase. The organic phase was washed with water and saturated brine, dried over anhydrous sodium sulfate, and concentrated under reduced pressure, to obtain a crude product. The crude product was purified by column to obtain the title product. LC-MS m/z: 320/322 [M+H]+.

### Step 3: Preparation of 1'-benzylspiro[cyclopenta[b]pyridine-6,4'-piperidine]-5-(7H)-1-one

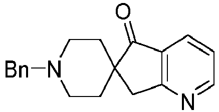

Sodium hydride (1.2g, 30mmol) was suspended in 10mL of anhydrous N,N-dimethylformamide, and then a solution of 6,7-dihydro-5H-cyclopenta[b]pyridin-5-one (2g, 15mmol) in 20mL of N,N-dimethylformamide was added dropwise at 0°C. After the addition, the mixture was raised to room temperature to perform a reaction for 1.5h. Then, a solution of N-benzyl-2-bromo-N-(2-bromoethyl)ethan-1-amine (4.8g, 15mmol) in 20mL of N,N-dimethylformamide was added dropwise again. After the addition, the mixture was continued to react at room temperature for 1.5 h, until the reaction was completed as detected by TLC. The reaction was quenched by addition of water. The resultant was extracted with dichloromethane, spin-dried, and extracted with water and ethyl acetate. The organic layer were collected, and dried with anhydrous sodium sulfate, and purified by column chromatography, to obtain 1.7g of a purple-black solid with a yield of 38.5 %. LC-MS m/z: 293.2 [M+1]⁺.

### Step 4: Preparation of tert-butyl 5-oxo-5,7-dihydrospiro[cyclopenta[b]pyridine-6,4'-piperidine]-1'-carboxylate

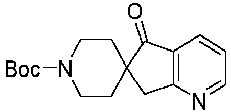

The intermediate1'-benzylspiro[cyclopenta[b]pyridine-6,4'-piperidine]-5-(7H)-1-one (1.73g, 5.9mmol) was dissolved in 20mL of 1,2-dichloroethane, and then 1-chloroethyl chloroformate (3.2mL, 29.6mmol) was added. The mixture was raised to 60°C to perform a reaction overnight. The mixture was removed of 1,2-dichloroethane through rotary evaporation and the residue was dissolved in methanol (20mL). The mixture was raised to 80°C to perform reation for 3h under reflux. The mixture was removed of methanol through rotary evaporation and the residue was dissolved in 20mL of dichloromethane, and added with N,N-diisopropylethylamine (2.9mL,17.5mmol) and di-tert-butyl dicarbonate (2.7mL, 11.7mmol). The mixture was reacted at room temperature for 1 h, until the reaction was completed as detected by TLC. The resultant was extracted with water and dichloromethane. The organic layer was seperated out, dried with anhydrous sodium sulfate and spin-dried, and purified by column chromatography, to obtain 0.7g of light yellow oil with a yield of 39.1%. LC-MS m/z: 303.2 [M+H]+.

### Step 5: Preparation of tert-butyl (R,Z)-5-((tert-butylsulfinyl)imino)-5,7-dihydrospiro[cyclopenta[b]pyridine-6,4'-piperidi ne]-1'-carboxytate

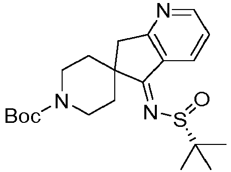

The intermediate tert-butyl 5-oxo-5,7-dihydrospiro[cyclopenta[b]pyridine-6,4'-piperidine]-1'-carboxylate (0.6g, 1.98mmol) was dissolved in 2-methyltetrahydrofuran (10mL), and then tetraethyl titanate (2.26g, 9.93mmol) and R-(+)-tert-butylsulfinamide (0.72g, 5.96mmol) were added. The mixture was refluxe at 90°C overnight. After the reaction was completed as detected by TLC, the resultant was extracted with water and ethyl acetate, and filtered with diatomite to remove insolubles. The organic was washed with saline solution. The organic layer were collected and dried with anhydrous sodium sulfate, and purified with column chromatography, to obtain 0.42g of a light yellow solid with a yield of 52.2%. LC-MS m/z: 405.6 [M+H]⁺.

### Step 6: Preparation of tert-butyl (S)-5-(((R)-(tert-butylsulfinyl)amino)-5,7-dihydrospiro[cyclopenta[b]pyridine-6,4'-piperi dine]-1'-carboxylate

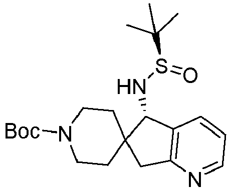

The intermediate tert-butyl (R,Z)-5-((tert-butylsulfinyl)imino)-5,7-dihydrospiro [cyclopenta[b]pyridine-6,4'-piperidine]-1'-carboxylate (0.42g, 1.03mmol) was dissolved in 10mL of anhydrous tetrahydrofuran, and then lithium borohydride solution (0.8mL, 1.6mmol) was added dropwise at 0°C. The mixture was continued to react at 0°C for 0.5 h. After the reaction was completed as detected by TLC, the reaction was qunched through addition of saturated ammonium chloride solution. The resultant was extracted with ethyl acetate, concentrated, and then purified by column chromatography, to obtain 0.15g of a light yellow solid with a yield of 35.5%. LC-MS m/z: 408.2 [M+H]⁺.

### Step 7: Preparation of (R)-N-((S)-5,7-dihydrospiro[cyclopenta[b]pyridine-6,4'-piperidine]-5-yl)-2-methylpropa ne-2-sulfinamide

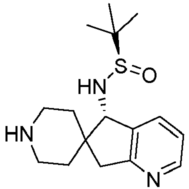

The intermediate tert-butyl (S)-5-(((R)-(tert-butylsulfinyl)amino)-5,7-dihydrospiro [cyclopenta[b]pyridine-6,4'-piperidine]-1'-carboxylate (150mg, 0.36mmol) was dissolved in 5mL of dichloromethane, and then 1mL of trifluoroacetic acid was added. The mixture was reacted at room temperature for 0.5h with stirring. After the reaction was completed as detected by TLC, the resultant was neutralized with saturated sodium bicarbonate, extracted with ethyl acetate, dried over anhydrous sodium sulfate, and spin-dried. The resulting product was used in the next reaction directly. LC-MS m/z: 308.2 [M+H]⁺

### Step 8: Preparation of (R)-N-((S)-1'-(8-bromoimidazo[1,2-c[pyrimidin-5-yl)-5,7-dihydrospiro[cyclopenta[b[pyri dine-6,4'-piperidin]-5-yl)-2-methylpropane-2-sulfinamide

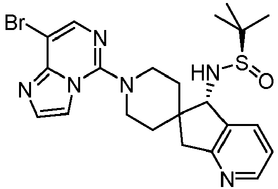

This intermediate was synthesized in the same way as that in Step 11 of Example 45, except that the raw material (S)-2-methyl-N-((R)-3H-spiro[benzofuran-2,4'-piperidine] -3-yl)propane-2-sulfinamide was replaced with (R)-N-((S)-5,7-dihydrospiro[cyclopenta [b]pyridine-6,4'-piperidine]-5-yl)-2-methylpropane-2-sulfinamide. LC-MS m/z: 503.2/505.2 [M+H]⁺.

### Step 9: Preparation of (R)-N-((S)-1'-(8-bromoimidazo[l,2-c]pyrimidin-5-yl)-5,7-dihydrospiro[cyclopenta[b]pyri dine-6,4'-piperidine]-5-yl)-2-methylpropane-2-sulfinamide

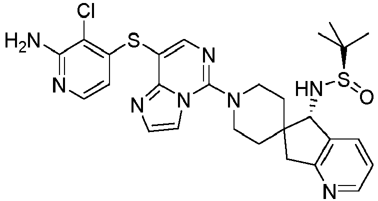

The target molecule was synthesized by using (R)-N-((S)-1'-(8-bromoimidazo[1,2-c]pyrimidin-5-yl)-5,7-dihydrospiro[cyclopenta[b]pyridine -6,4'-piperidine]-5-yl)-2-methylpropane-2-sulfinamide and 2-amino-4-bromo-3-chloropyridine as raw materials according to the method of Example 45. LC-MS m/z: 583.2 [M+H]⁺.

### Step 10: Synthesis of (S)-1'-(8-((2-amino-3-chloropyridin-4-yl)thio)imidazo[1,2-c[pyrimidin-5-yl)-5,7-dihydros piro[cyclopenta[b]pyridine-6,4'-piperidine]-5-amine

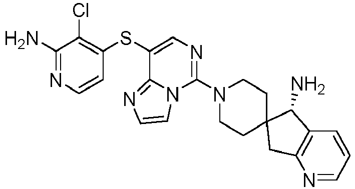

The title compound was synthesied by using (R)-N-((S)-1'-(8-bromoimidazo[1,2-c]pyrimidin-5-yl)-5,7-dihydrospiro[cyclopenta[b]pyridine -6,4'-piperidine]-5-yl)-2-methylpropane-2-sulfinamide as raw materials according to Step 15 of Example 45. ¹H NMR (400 MHz, DMSO) δ 8.34(d, *J* = 3.8 Hz, 1H), 8.03 (s, 1H), 7.84(s, 1H), 7.70 (d, *J* = 7.4 Hz, 1H), 7.62 - 7.49 (m, 2H), 7.20 (s, 1H), 6.32 (s, 2H), 5.80 (d, *J* = 5.3 Hz, 1H), 3.99 (s, 3H), 3.14(d, *J* = 16.3 Hz, 2H), 2.81 (d, *J* = 16.3 Hz, 1H), 2.13 - 1.82 (m, 3H), 1.65 (d, *J=* 13.0 Hz, 1H), 1.41 - 1.13 (m, 3H). LC-MS m/z: 479.2[M+H]⁺.

### Example 47: (R)-1'-(8-(((2-amino-3-fluoropyridin-4-yl)thio)imidazo[1,2-c]pyrimidin-5-yl)-3H-spiro[be nzofuran-2,4'-piperidin]]-3-amine

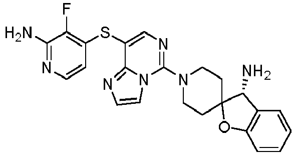

The title compound was prepared according to the method of Example 45, except that 2-amino-4-bromo-3-chloropyridine was replaced with 2-amino-4-bromo-3-fluoropyridine.

¹H NMR (400 MHz, DMSO) δ 8.19 (s, 1H), 8.03 (s, 1H), 7.90 (s, 1H), 7.60 (s, 1H), 7.43 (dd, J = 18.3, 6.2 Hz, 2H), 7.26 - 7.17 (m, 1H), 6.96 - 6.80 (m, 2H), 6.70 - 6.56 (m, 1H), 6.23 (s, 1H), 5.91 (s, 1H), 5.32 (s, 1H), 3.50 (s, 4H), 2.06 - 1.78 (m, 4H). 1.0 (d, J = 6.8 Hz, 1H). ESI-MS m/z: 464.2 [M+H]⁺.

### Example 48 : (R)-1'-(8-(2,3-dichlorophenyl)-7-methylimidazo[1,2-c]pyrimidin-5-yl)-3H-spiro[benzofur an-2,4'-piperidine]-3-amine

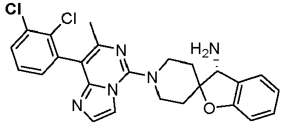

### Step 1: Synthesis of 8-bromo-5-chloro-7-methylimidazo[1,2-c]pyrimidine

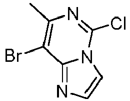

The title compound 8-bromo-5-chloro-7-methylimidazo[1,2-c]pyrimidine was synthsized according to the method in steps 8-10 of Example 45, except that 5-bromo-2,4-dichloropyrimidine was replaced with 5-bromo-2,4-dichloro-6-methylpyrimidine. ESI-MS m/z: 246.2 [M+H]⁺.

### Step 2: Synthesis of (R)-N-((R)-1'-(8-bromo-7-methylimidazo[1,2-c]pyrimidin-5-yl)-3H-spiro[benzofuran-2,4 '-piperidine]-3-yl)-2-methylpropane-2-sulfinamide

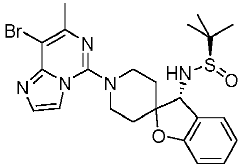

The title product was synthsized according to the method in step 11 of Example 45, except that 8-bromo-5-chloroimidazo[1,2-c]pyrimidine was replaced with 8-bromo-5-chloro-7-methylimidazo[1,2-c]pyrimidine. ESI-MS m/z: 518.2/520.2 [M+H]⁺.

### Step 3: Synthesis of (R)-N-((R)-1'-(8-(2,3-dichlorophenyl)-7-methylimidazo[1,2-c]pyrimidin-5-yl)-3H-spiro[b enzofuran-2,4'-piperidine]-3-yl)-2-methylpropane-2-sulfinamide

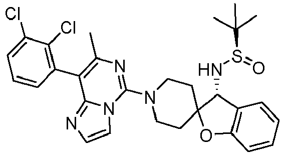

Into a 100ml single neck flask, (R)-N-((R)-1'-(8-bromo-7-methylimidazo[1,2-c]pyrimidin-5-yl)-3H-spiro[benzofuran-2,4'-pi peridine]-3-yl))-2-methylpropane-2-sulfinamide (233mg, 0.45 1mmol), dichlorobenzeneboronic acid (94mg, 0.496mmol), potassium carbonate (174mg, 1.35mmol), N,N-dimethylacetylamide(10ml) and water (0.5mL) were added, and then tetrakis(triphenylphosphine)palladium (70mg, 0.06mmol) was added. The mixture was reacted for at 110°C 3h under the protection of argon gas. After completion of the reaction, the mixture was extracted with 30mL of water and 60mL of ethyl acetate. Organic phases were combined, washed with water and saturated sodium chloride solution, and concentrated, and purified by column chromatography, to obtain 100mg of the target product with a yield of 38%. ESI-MS m/z: 584.2 [M+H]⁺.

### Step 4: Synthesis of (R)-1'-(8-(2,3-dichlorophenyl)-7-methylimidazo[1,2-c]pyrimidin-5-yl)-3-spiro[benzofur an-2,4'-piperidine]-3-amine

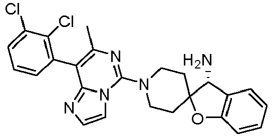

In a 100mL reaction flask, (R)-N-((R)-1'-(8-(2,3-dichlorophenyl)-7-methylimidazo[1,2-c]pyrimidin-5-yl)-3H-spiro[benz ofuran-2,4'-piperidine]-3-yl))-2-methylpropane-2-sulfinamide (100mg, 0.172mmol) was dissolved in anhydrous dioxane (8mL), and added with hydrochloric acid/dioxane solution (4mL). After completion of the reaction, the resultant was concentrated, to obtain 21mg of title product with a yield of 25.6%. 1H NMR (400 MHz, DMSO) δ 7.79 (s, 1H), 7.74-7.73 (d, J = 7.7 Hz, 1H), 7.57-7.44(m, 2H), 7.41-7.36 (dd, J = 14.5, 7.4 Hz, 2H), 7.17-7.15 (t, J = 7.5 Hz, 1H), 6.91-6.87 (t, J = 7.3 Hz, 1H), 6.81-6.79 (d, J = 7.9 Hz, 1H), 4.18 (s, 1H), 3.89-3.78 (dd, J = 29.6, 13.0 Hz, 2H), 3.44(s, 2H), 2.20 (d, J = 13.1 Hz, 1H), 2.14(s, 3H), 2.08(s, 1H) 2.02-1.92 (dd, J = 25.3, 11.8 Hz, 3H), 1.86-1.83 (d, J = 13.6 Hz, 1H)..ESI-MS m/z: 480.2 [M+H]+.

### Example 49 : (R)-1'-(8-(2-chloro-3-methoxyphenyl)-7-methylimidazo[1,2-c]pyrimidin-5-yl)-3H-spiro[b enzofuran-2,4'-piperidine]-3-amine

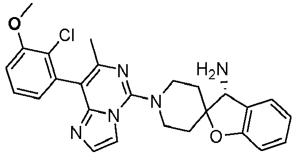

The title compound was prepared by using the same method as that of Example 48, except that the intermediate 2,3-dichlorobenzeneboronic acid was replaced with 2-chloro-3-methoxyphenylboronic acid. 1H NMR (400 MHz, DMSO) δ 7.76 (s, 1H), 7.45 (s, 1H), 7.43 - 7.31 (m, 2H), 7.26 - 7.10 (m, 2H), 6.96 (d, J = 7.3 Hz, 1H), 6.88 (t, J = 7.2 Hz, 1H), 6.79 (d, J = 7.8 Hz, 1H), 4.16 (s, 1H), 3.92 (s, 3H), 3.80 (dd, J = 28.6, 12.5 Hz, 2H), 2.15 (d, J = 22.1 Hz, 5H), 2.07 - 1.75 (m, 4H), 1.23 (s, 2H). ESI-MS m/z: 476.2 [M+H]+.

### Example 50 : (R)-1'-(8-(3-chloro-2-((cyclopropylamino)pyridin-4-yl)imidazo[1,2-c]pyrimidin-5-yl)-3H-spiro[spiro[benzofuran-2,4'-piperidine]-3-amine

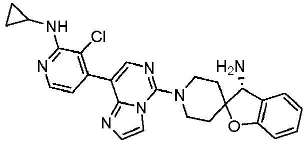

### Step 1: Preparation of (R)-N-((R)-1'-(8-(3-chloro-2-((cyclopropylamino)pyridin-4-yl)imidazo[1,2-c]pyrimidin-5-yl)-3H-spiro[benzofuran-2,4'-piperidine]-3-yl)-2-methylpropane-2-sulfinamide

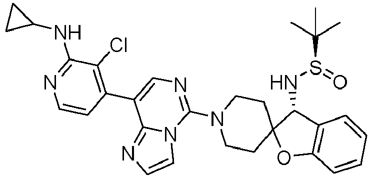

In a 100ml single neck flask, the product in step 11 of Example 45 (R)-N-((R)-1'-(8-bromoimidazo[1,2-c]pyrimidin-5-yl)-3H-spiro[benzofuran-2,4'-piperidine]-3-yl))-2-methylpropane-2-sulfinamide (200mg, 0.396mmol) was dissolved in 1,4-dioxane (20ml), and then 3-chloro-2-(cyclopropyl-2-azayl)-4-(4,4,5,5-tetramethyl-1,3,2-dioxaboran-2-yl)pyridine (128mg, 0.436mmol), tetrakis(triphenylphosphine)palladium (23mg, 0.019mmol), and potassium carbonate (164mg, 1.19mmol) were added. The mixture was reacted at 100°C overnight. After the reaction was completed as detected by LC-MS and TLC, the resultant was subjected to suction filtration to remove insolubles, and ethyl acetate and water were added. The water phase was extracted for 3 times, and the the organic phase was washed with saturated brine for 3 times. The resulting organic phase was dried over anhydrous sodium sulfate, concentrated, and separated by column chromatography, to obtain 90mg of a product with a yield of 38%. ESI-MS m/z: 592.2 [M+H]⁺.

### Step 2: Preparation of (R)-1'-(8-(3-chloro-2-((cyclopropylamino)pyridin-4-yl)imidazo[1,2-c]pyrimidin-5-yl)-3H-spiro[benzofuran-2,4'-piperidine]-3-amine

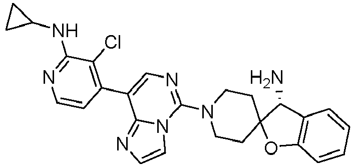

In a 100ml single neck flask, (R)-N-((R)-1'-(8-(3-chloro-2-((cyclopropylamino)pyridin-4-yl)imidazo[1,2-c]pyrimidin-5-yl)-3H-spiro[benzofuran-2,4'-piperidine]-3-yl)-2-methylpropane-2-sulfinamide (90mg, 0.152mmol) was dissolved in 1,4-dioxane (20ml), and hydrochloric acid/dioxane (2ml) was slowly added dropwise. After the addtion, the mixture was reacted for 1 hour. After the reaction was completed as detected by LC-MS and TLC, the reaction solution was concentrated and separated by preparative HPLC, to obtain 10mg of a white solid product with a yield of 13%. ¹H NMR (400 MHz, DMSO) δ 8.11 (d, *J =* 4.9 Hz, 1H), 7.87 (s, 1H), 7.76 (d, *J* = 9.2 Hz, 1H), 7.62 (s, 1H), 7.44(d, *J =* 7.1 Hz, 1H), 7.29 - 7.15 (m, 2H), 6.99 - 6.90 (m, 1H), 6.87 (d, *J* = 8.0 Hz, 1H), 6.75 (d, *J =* 4.9 Hz, 1H), 6.67 (s, 1H), 4.35 (s, 1H), 3.95 (d, *J* = 12.3 Hz, 1H), 3.86 (d, *J =* 12.7 Hz, 1H), 2.79 (s, 1H), 2.29 - 2.18 (m, 2H), 2.02 (dd, *J =* 28.9, 11.4 Hz, 3H), 1.24(s, 2H), 0.72 (d, *J =* 5.0 Hz, 2H), 0.57 (s, 2H). ESI-MS m/z: 488.2 [M+H]⁺.

### Example 51 : (R)-6-(3-amino-3H-spiro[benzofuran-2,4'-piperidine]-1'-yl)-3-(3-chloro-2-((cyclopropyla mino)pyridin-4-yl)-5-methyl-2,5-dihydro-4H-pyrazolo[3,4-d]pyrimidin-4-one

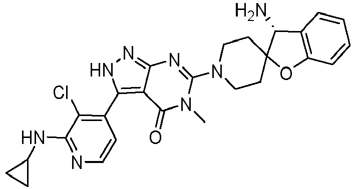

### Step 1: 6-hydrazino-3-methylpyrimidine-2,4(1H,3H)-dione

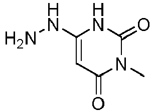

In a 500mL three-necked flask, 6-chloro-3-methylpyrimidine-2,4(1H,3H)-dione (10.0g, 62.3mmol) was dissolved in ethanol (200mL), and then hydrazine hydrate (9.17mL, 187.0mmol) was added under protection of nitrogen gas at room temperature. The mixture was heated to react at 80°C for 1h, and then reacted at room temperature for another 1h. After the reaction was completed as detected by LC-MS and TLC, the reaction solution was filtered. The filter cake was washed with 100mL of ethanol, dried to obtain 13.2 g of crude product with a yield of 100%. ESI-MS m/z: 157.1[M+H]⁺.

### Step 2: (E)-6-((4-methoxybenzyl)diazenyl)-3-methylpyrimidine-2,4(1H,3H)-dione

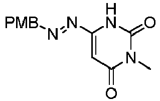

In a 250mL three-necked flask, 6-hydrazino-3-methylpyrimidine-2,4(1H,3H)-dione (9.7g, 62.30mmol) was dissolved in methanol (50mL). After replacement by nitrogen gas, 4-methoxybenzaldehyde (9.1mL, 74.76mmol) was added. The mixed solution was reacted at room temperature for 30 min, and then the reaction solution was diluted with methanol (45mL), to continue the reaction at room temperature for 30 min. After the reaction was completed as detected by LC-MS and TLC, the reaction solution was filtered. The filter cake was washed with 100mL of methanol and dried, to obtain 16.7 g of a light yellow solid with a yield of 98%. ESI-MS m/z: 275.1[M+H]⁺.

### Step 3: 3-(2,3-dichloropyridin-4-yl)-2-(4-methoxybenzyl)-5-methyl-2,7-dihydro-4H-pyrazolo[3,4 -d]pyrimidin-4,6(5H)-dione

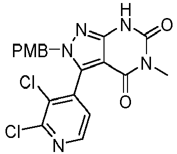

In a 100mL single neck flask, (*E*)-6-((4-methoxybenzyl)diazenyl)-3-methylpyrimidine-2,4(1H,3H)-dione (0.47g, 1.71mmol) was dissolved in N,N-dimethylformamide/isopropanol (2:1, 9mL), and then piperidine (0.15mL, 1.67mmol) and 2,3-dichloropyridine-4-carbaldehyde (0.3g, 1.71mmol) were added. The mixture was reacted at 85°C for 1.5 h. After the reaction was completed as detected by LC-MS and TLC, the reaction solution was poured into 5mL of water, added with 5mL of ethyl acetate. The mixture was stirred for 10 min at room temperature, and then exracted with ethyl acetate for 3 times. The organic phase was washed with saturated sodium chloride aqueous solution once, dired, concentrated, and separated by column chromatography, to obtain 0.459 g of a yellow solid with a yield of 62%. ESI-MS m/z: 432.2 [M+H]⁺.

### Step 4: 3-(3-chloro-2-(cyclopropylamino)pyridin-4-yl)-2-(4-methoxybenzyl)-5-methyl-2,7-dihydr o-4H-pyrazolo[3,4-d]pyrimidin-4,6(5H)-dione

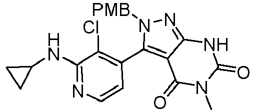

3-(2,3-Dichloropyridin-4-yl)-2-(4-methoxybenzyl)-5-methyl-2,7-dihydro-4H-pyrazo lo[3,4-d]pyrimidin-4,6(5H)-dione (0.459g, 1.06mmol) was dissolved in cyclopropylamine (4mL). After replacement by nitrogen gas, the mixture was reacted in microwave at 150°C for 10 min. After completion of the reaction, the resultant was exracted with 50mL of ethyl acetate for 3 times, washed with saturated brine once, dried over anhydrous sodium sulfate, and concentrated, and purified by column chromatography, to obtain 0.394 g of a brown solid with a yield of 82%. ESI-MS m/z: 453.2 [M+H]⁺.

### Step 5: (R)-N-((R)-1'-(3-(3-chloro-2-((cyclopropylamino)pyridin-4-yl)-2-(4-methoxybenzyl)-5-me thyl-4-oxo-4,5-dihydro-2H-pyrazolo[3,4-d]pyrimidin-6-yl)-3H-spiro[benzofuran-2,4'-pip eridine]-3-yl)-2-methylpropane-2-sulfinamide

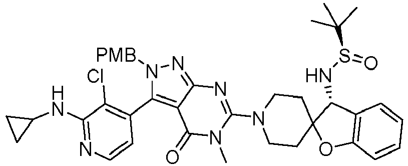

In a 100mL single neck flask, 3-(3-chloro-2-((cyclopropylamino)pyridin-4-yl)-2-(4-methoxybenzyl)-5-methyl-2,7-dihydro-4 H-pyrazolo[3,4-d]pyrimidine-4-,6(5H)-dione (0.394g, 0.869mmol) was dissolved in anhydrous N, N-dimethylformamide (3ml), and then the product in step 7 of Example 1 (R)-2-methyl-N-((R)-3H-spiro[benzofuran-2,4'-piperidine]-3-yl)propane-2-sulfinamide (0.295g, 0.957mmol), Castros condensing agent BOP (0.769g, 1.738mmol), and 1,8-diazabicycloundec-7-ene (0.6mL, 4.345mmol) were added. The mixture was replaced with nitrogen gas and then reacted at room temperature overnight. After completion of the reaction, saturated ammonium chloride aqueous solution was added to the reaction solution to quench the reaction. The resulting reaction solution was exracted with 60mL dichloromethan for 3 times. The organic phase was washed with saturated sodium chloride aqueous solution once, dried, oncentrated, and then seperated by column chromatography, to obtain 0.345 g of a product with a yield of 53%. ESI-MS m/z: 743.1 [M+H]⁺.

### Step 6: Synthesis of (R)-6-(3-amino-3H-spiro[benzofuran-2,4'-piperidin]-1'-yl)-3-(3-chloro-2-((cyclopropyla mino)pyridin-4-yl)-5-methyl-2,5-dihydro-4H-pyrazolo[3,4-d]pyrimidin-4-one

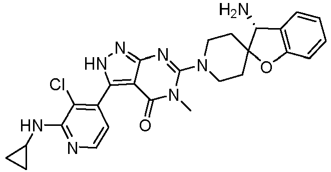

In a 100mL single neck flask, (R)-N-((R)-1'-(3-(3-chloro-2-((cyclopropylamino)pyridin-4-yl)-2-(4-methoxybenzyl)-5-methy 1-4-oxo-4,5-dihydro-2H-pyrazolo[3,4-d]pyrimidin-6-yl)-3H-spiro[benzofuran-2,4'-piperidine] -3-yl)-2-methylpropane-2-sulfinamide (0.28g, 0.377mmol) was dissolved in anhydrous dichloromethane solution, and then trifluoroacetic acid (1.1mL) and para toluene sulfonic acid (0.03mL) were added dropwise. The mixture was reacted at room temperature for 10 min. After the reaction was completed as detected by LC-MS and TLC, the reaction solution was concentrated directly. ESI-MS m/z: 623.1 [M+H]⁺. The concentrated reaction solution was dissolved in dioxane, and then added dropwise with 2mL of hydrochloric acid-dioxane solution. The mixture was reacted at room temperature for 30 min. After the reaction was completed as detected by LC-MS and TLC, the reaction solution was directly concentrated and seperated, to obtain 10 mg of white powder with a yield of 3.8%. ¹H NMR (400 MHz, DMSO) δ 11.81(s,IH), 8.32(d, J = 5.1 Hz , 1H), 7.83(d, J = 4.9 Hz, 1H), 7.80(d, J = 8.7 Hz ,.1H), 7.01(m ,1H), 6.93-9.92(m, 2H), 3.46(s, 2H), 3.36(s, 3H), 3.15(s, 1H), 3.13(s, 1H), 2.89(m, 2H), 2.83(m, 2H), 2.28(s, 1H), 2.25(m, 2H), 1.93(m, 2H), 0.91(m, 2H), 0.64(m, 2H). ESI-MS m/z: 519.2 [M+H]⁺.

### Example 52 : (S)-6-(5-amino-5,7-dihydrospiro[cyclopenta[b]pyridine-6,4'-piperidin]-1'-yl)-3-(3-chloro -2-((cyclopropylamino)pyridin-4-yl)-5-methyl-2,5-dihydro-4H-pyrazolo[3,4-d]pyrimidin-4-one

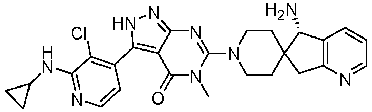

### Step 1: Synthesis of (R)-N-((S)-1'-(3-(3-chloro-2-((cyclopropylamino)pyridin-4-yl)-2-(4-methoxybenzyl)-5-me thyl-4-oxo-4,5-dihydro-2H-pyrazolo[3,4-d]pyrimidin-6-yl)-5,7-dihydrospiro[cyclopenta[ b]pyridine-6,4'-piperidin]-5-yl)-2-methylpropane-2-sulfinamide

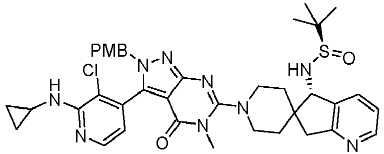

In a 100mL single neck flask, 3-(3-chloro-2-((cyclopropylamino)pyridin-4-yl)-2-(4-methoxybenzyl)-5-methyl-2,7-dihydro-4 H-pyrazolo[3,4-d]pyrimidine-4,6(5H)-dione(0.106g, 0.234mmol) was dissolved in anhydrous DMF, and then (R)-N-((S)-5,7-dihydrospiro[cyclopenta[b]pyridine-6,4'-piperidine]-5-yl)-2-methyl propane-2-sulfinamide(0.079g, 0.257mmol), Castros condensing agent BOP (0.21g, 0.468mmol), and 1,8-diazabicycloundec-7-ene (0.16mL, 1.17mmol) were added in sequence. The mixture was replaced and protected with nitrogen gas and reacted overnight at room temperature. After completion of the reaction, saturated ammonium chloride aqueous solution was added to quench the reaction. The resultant was exracted with 60mL of dichloromethane for 3 times, washed with saturated sodium chloride aqueous solution once, dried, concentrated, and subjected to column chromatography to obtain 44 mg of a weak polar isomer and 61 mg of a strong polar isomer with a yield of 61%. ESI-MS m/z: 742.1 [M+H]⁺.

### Step 6: Synthesis of (S)-6-(5-amino-5,7-dihydrospiro[cyclopenta[b]pyridine-6,4'-piperidine]-1'-yl)-3-(3-chlor o-2-(cyclopropylamino)pyridin-4-yl)-5-methyl-2,5-dihydro-4H-pyrazolo[3,4-d]pyrimidin -4-one

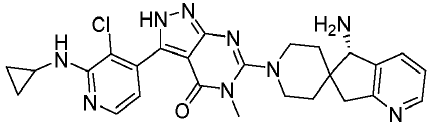

In a 100mL single neck flask, (R)-N-((S)-1'-(3-(3-chloro-2-((cyclopropylamino)pyridin-4-yl)-2-(4-methoxybenzyl)-5-methy 1-4-oxo-4,5-dihydro-2H-pyrazolo[3,4-d]pyrimidin-6-yl)-5,7-dihydrospiro[cyclopenta[b]pyridi ne-6,4'-piperidine]-5-yl)-2-methylpropane-2-sulfinamide(0.044g, 0.059mmol) was dissolved in 5mL of anhydrous dichloromethane solution, and then trifluoroacetic acid (0.18mL) and para toluene sulfonic acid (0.005mL) were added dropwise. The mixture was reacted at room temperature for 10 min. After completion of the reaction, the reaction solution was concentrated. ESI-MS m/z: 622.1 [M+H]+. The concentrated reaction solution was dissolved in dioxane, and then 2mL of hydrochloric acid-dioxane solution was added dropwise. The mixture was reacted at room temperature for 30 min. After completion of the reaction, the reaction solution was directly concentrated and purified to obtain 5mg of the title product with a yield of 17%. 1H NMR (400 MHz, DMSO) 8 11.85(s,1H), 8.47(d, J= 5.3Hz , 1H), 7.99(d, *J* = 5.1Hz, 1H), 7.78(d, *J* = 8.9 Hz ,.1H), 7.55(m ,1H), 6.97(t, 1H), 3.73(s, 1H), 3.37(s, 3H), 3.14(m, 3H), 3.01(m, 4H), 2.41(s, 1H), 2.36(s, 1H), 2.23(m, 2H),2.02(m, 3H), 1.93(m, 2H), 1.56(m, 2H). ESI-MS m/z: 518.2 [M+H]⁺.

### Example 53 : (R)-6-amino-2-(3-amino-3H-spiro[benzofuran-2,4'-piperidine]-1'-yl)-3-methyl-5-((2-(trif luoromethyl)pyridin-3-yl)thio)pyrimidin-4(3H)-one

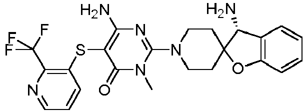

### Step 1: 6-amino-5-bromo-3-methylpyrimidin-2,4(1H,3H)-dione

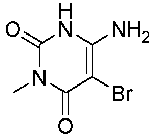

In a 250mL three-necked flask, 6-amino-3-methylpyrimidine-2,4(1H,3H)-dione (1.0g, 7.08mmol) was dissolved in N,N-dimethylformamide (50mL). The mixture was replaced with nitrogen gas, and then N-bromosuccinimide (1.39g, 7.79mmol) was added at room temperature. The mixture was reacted at room temperature overnight. After the reaction was completed by detection, the reaction solution was filtered. The filter cake was washed with petroleum ether (50mL) and dried, to obtain 0.9 g of a white solid with a yield of 25%. ESI-MS m/z: 220.1[M+H]⁺.

### Step 2: Synthesis of (R)-N-((R)-1'-(4-amino-5-bromo-1-methyl-6-oxo-1,6-dihydropyrimidin-2-yl)-3H-spiro[b enzofuran-2,4'-piperidine]-3-yl)-2-methylpropane-2-sulfinamide

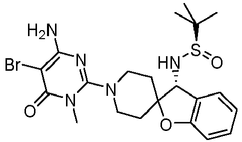

In a 100mL three-necked flask, 6-amino-5-bromo-3-methylpyrimidine-2,4 (1H, 3H)-dione (0.43g, 1.96mmol) was dissolved in N,N-dimethylformamide (5mL), and then (R)-2-methyl-N-((R)-3H-spiro[benzofuran-2,4'-piperidine]-3-yl)propane-2-sulfinamide (0.6g, 1.96mmol), Castros condensing agent BOP (1.74g, 3.92mmol) and 1,8-diazabicyclo[5.4.0]undec-7-ene (1.5mL, 9.8mmol) were added in sequence. The mixture was replaced with nitrogen gas and reacted at room temperature for 2.5 days. After the reaction was completed by detection, the resultant was exracted with ethyl acetate (100mL) for 3 times, washed with saturated sodium chloride aqueous solution once, dried, concentrated and purified, to obtain 0.4 g of a crude product with a yield of 60%. ESI-MS m/z: 510.2/512.2[M+H]⁺.

### Step 3: Synthesis of (R)-N-((R)-1'-(4-amino-1-methyl-6-oxo-5-((2-(trifluoromethyl)pyridin-3-yl)thio)-1,6-dihy dropyrimidin-2-yl)-3H-spiro[benzofuran-2,4'-piperidine]-3-yl)-2-methylpropane-2-sulfi namide

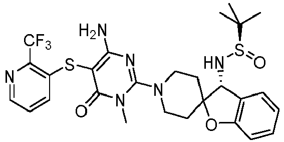

In a 100mL single neck flask, (R)-N-((R)-1'-(4-amino-5-bromo-1-methyl-6-oxo-1,6-dihydropyrimidin-2-yl)-3H-spiro[benzo furan-2,4'-piperidine]-3-yl)-2-methylpropane-2-sulfinamide (0.4g, 0.78mmol) was dissolved in 1,4-dioxane (10mL), and then 2-(trifluoromethyl)pyridine-3-thiol (0.4g, 0.78mmol), cuprous iodide (0.03g, 0.16mmol), tetramethylethylenediamine (0.05mL, 0.31mmol), potassium phosphate (0.5g, 2.34mmol) and phenanthroline (0.06g, 0.31mmol) were added. The mixture was reacted at 100°C for 1.5h. After the reaction was completed by detection, the resultant was exracted with ethyl acetate (100mL) for 3 times, washed with saturated sodium chloride aqueous solution once, dried, concentrated, and subjected to column chromatography, to obtain 0.181g of the title product with a yield of 29%. ESI-MS m/z: 609.1 [M+H]⁺.

### Step 4: Synthesis of (R)-6-amino-2-(3-amino-3H-spiro[benzofuran-2,4'-piperidine]-1'-yl)-3-methyl-5-((2-(trif luoromethyl)pyridin-3-yl)thio)pyrimidin-4(3H)-one

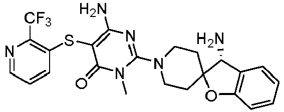

N-(1'-(4-amino-1-methyl-6-oxo-5-((2-(trifluoromethyl)pyridin-3-yl)thio)-1,6-dihydr opyrimidin-2-yl)-3H-spiro[benzofuran-2,4'-piperidine]-3-yl)-2-methylpropane-2-sulfinamide (0.181g, 0.30mmol) was dissolved in dioxane (2mL). The mixture was replaced by nitrogen gas, and then added dropwise with hydrochloric acid-dioxane solution (2mL). After the reaction was completed as detected by LC-MS and TLC, the resultant was directly concentrated and separated by preparative chromatography, to obtain 15 mg of the title product (white). ¹H NMR (400 MHz, DMSO) δ 8.41 (d, *J* = 3.3 Hz, 1H), 7.56-7.48 (m, 1H), 7.42 (*d, J* = 8.1 Hz, 1H), 7.36 (*d, J*=7.1 Hz, 1H), 7.21-7.13 (m, 1H), 6.89 (*t, J* =7.2 Hz, 1H), 6.79 (*d, J* =7.9 Hz, 1H), 5.32(s, 2H), 4.19 (s, 1H), 3.34(s, 3H), 2.12-1.68 (m, 8H), 1.2(s, 2H). ESI-MS m/z: 505.2 [M+H]⁺.

### Example 54 : (S)-5-((5-(1-amino-1,3-dihydrospiro[indene-2,4'-piperidine]-1'-yl)imidazo[1,2-c]pyrimidi n-8-yl)thio)-6-chloro-1-methylpyridin-2(1H)-one

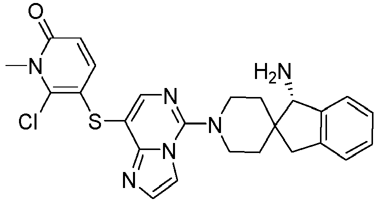

### Step 1: Synthesis of tert-butyl 1-oxo-1,3-dihydrospiro[indene-2,4'-piperidine]-1'-carboxylate

Under protection of nitrogen gas, sodium hydride (4.12g, 103.25mmol) was dissolved in anhydrous tetrahydrofuran (100mL), and then slowly added dropwise with a solution of 2,3-dihydro-1H-inden-1-one (5.45g, 41.30mmol) in tetrahydrofuran at 0°C. The mixture was stirred at room temperature for 0.5 hours. After that, a solution of tert-butyl bis(2-chloroethyl)carbamate (10.0g, 41.30mmol) in tetrahydrofuran was slowly added at 0°C dropwise. After the addition, the mixture was reacted at 60°C overnight. After the reaction was completed as detected by LC-MS and TLC, ethyl acetate and water were added. The water phase was extracted for 3 times. The organic phase was dried over anhydrous sodium sulfate, concentrated, and separated by column chromatography, to obtain the title compound (3.50g, yield: 31.80%). ESI-MS m/z: 246.20 [M+H]⁺

### Step 2: Synthesis of tert-butyl (R, Z)-1-((tert-butylsulfinyl)imino)-1,3-dihydrospiro[indene-2,4'-piperidine]-1'-carboxylate

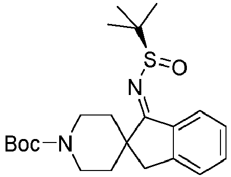

Under protection of nitrogen gas, tert-butyl 1-oxo-1,3-dihydrospiro[indene-2,4'-piperidine]-1'-carboxylate (0.50g, 1.66mmol), (R)-2-methylpropane-2-sulfinamide (0.80g, 6.64mmol) and tetraethyl titanate (3.03g, 2813.mmol) were dissolved in anhydrous tetrahydrofuran (50mL). The mixture was reacted at 90°C overnight. After the reaction was completed as detected by LC-MS and TLC, the reaction was quenched with saturated sodium bicarbonate. The resultant was filtered with diatomite and added with ethyl acetate and water. The water phase was extracted for 3 times. The organic phase was dried with anhydrous sodium sulfate, concentrated, and subjected to column chromatography, to obtain the title product (0.30g, yield: 44.78%). ESI-MS m/z: 305.20 [M+H]⁺

### Step 3: Synthesis of tert-butyl (S)-1-(((R)-tert-butylsulfinyl)amino)-1,3-dihydrospiro[indene-2,4'-piperidine]-1'-carbox ylate

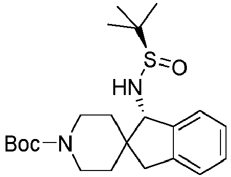

Tert-butyl (R,Z)-1-((tert-butylsulfinyl)imino)-1,3-dihydrospiro[indene-2,4'-piperidine]-1'-carboxylate (1.0g, 2.47mmol) was dissolved in anhydrous tetrahydrofuran (20mL). Under protection of nitrogen gas, lithium borohydride solution (2.0M, 3.70mmol) was slowly added dropwise at 0°C. The mixture was stired at room temperature. After the reaction was completed as detected by TLC and LCMS, the reaction was quenched with saturated ammonium chloride at 0°C, and then added with tthyl acetate and water. The water phase was extracted with 3 times. The organic phase was dried with anhydrous sodium sulfate, concentrated, and seperated by column chromatography, to obtain the title product (0.7g, yield: 44.78%). ESI-MS m/z: 307.20 [M+H]⁺

### Step 4: Synthesis of (R)-N-((S)-1,3-dihydrospiro[indene-2,4'-piperidine]-1-yl)-2-methylpropane-2-sulfinamid e

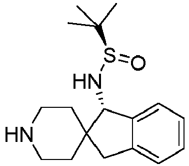

Tert-butyl (S)-1-(((R)-tert-butylsulfinyl)amino)-1,3-dihydrospiro[indene-2,4'-piperidine] -1'-carboxylate (0.20g, 0.49mmol) was dissolved in dichloromethane (5mL), and then added with trifluoroacetic acid (2.5mL). After the addition, 60°C stirred at room temperature. After the reaction was completed as detected by TLC, the resultant was adjusted to a pH of 7-9 with sodium bicarbonate, extracted with ethyl acetate, washed with water and saturated sodium chloride, dried and concentrated, to obtain the title product, which was directly used in the next reaction (0.35 g, yield: 100%). ESI-MS m/z: 307.1 [M+H]+

### Step 5: Synthesis of (S)-5-((5-(1-amino-1,3-dihydrospiro[indene-2,4'-piperidine]-1'-yl)imidazo[1,2-c]pyrimidi n-8-yl)thio)-6-chloro-1-methylpyridin-2 (1H)-one

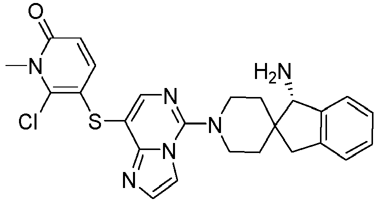

The title compound was prepared by using the same method as that of Example 45, except that 4-bromo-3-chloropyridin-2-amine was replaced with 5-bromo-6-chloro-1-methylpyridin-2 (1H)-one.

1H NMR (400 MHz, CDCl3) δ 7.66 (s, 1H), 7.64 - 7.55 (m, 2H), 7.47 (s, 1H), 7.35 (s, 1H), 7.22 (s, 2H), 6.47 (d, J = 9.4 Hz, 1H), 5.30 (d, J = 40.2 Hz, 2H), 4.06 (s, 1H), 3.79 (d, J = 13.5 Hz, 2H), 3.72 (s, 3H), 3.22 (dd, J = 24.2, 12.2 Hz, 2H), 3.11 (d, J = 15.6 Hz, 1H), 2.76 (d, J = 15.5 Hz, 1H), 2.12 - 1.90 (m, 3H), 1.47 (d, J = 12.8 Hz, 2H). ESI-MS m/z: 493.20 [M+H]+

According to the synthesis method of Example 45 or 48, the compounds of examples 55-74 was synthesized by using different commercially available raw materials. The characterization parameters of these compounds are set forth in Table 2:

**Table 2**

| Ex. | structure of compound | ESI-MS m/z [M+H]⁺ | Ex. | structure of compound | ESI-MS m/z [M+H]⁺ |
|---|---|---|---|---|---|
| 55 | 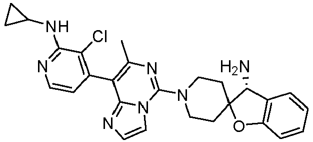 | 502.2 | 65 | 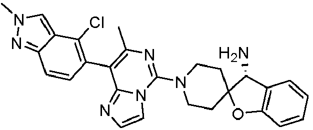 | 500.2 |
| 56 | 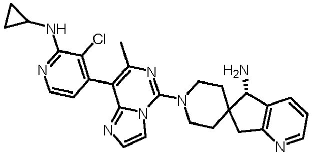 | 501.3 | 66 | 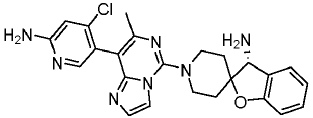 | 462.3 |
| 57 | 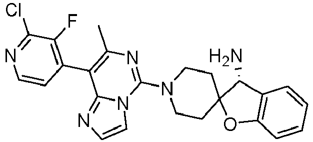 | 465.4 | 67 | 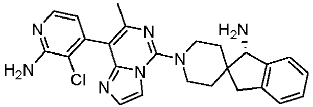 | 460.1 |
| 58 | 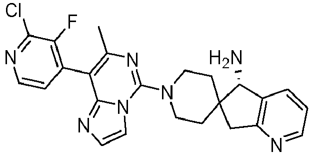 | 464.2 | 68 | 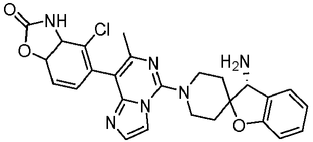 | 505.2 |
| 59 | 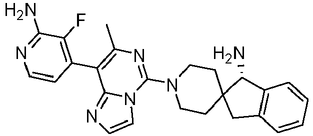 | 444.2 | 69 | 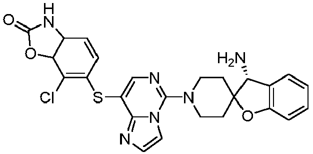 | 523.3 |
| 60 | 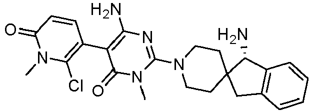 | 467.1 | 70 | 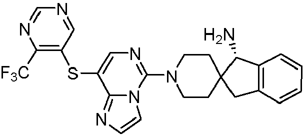 | 498.3 |
| 61 | 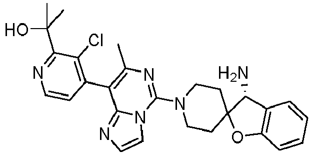 | 505.2 | 71 | 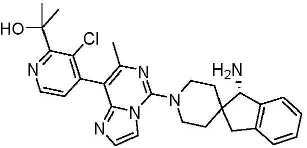 | 503.1 |
| 62 | 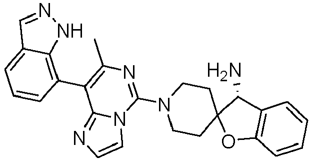 | 452.1 | 72 | 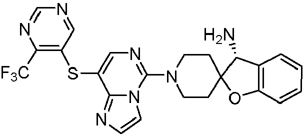 | 500.1 |
| 63 | 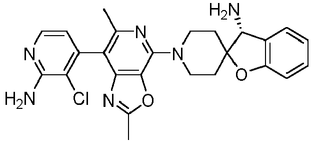 | 477.2 | 73 | 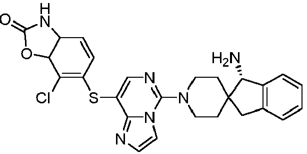 | 521.4 |
| 64 | 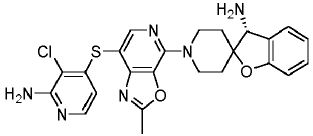 | 495.1 | 74 | 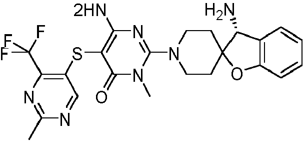 | 520.2 |

### Example 75 : (R)-2-(5-(3-amino-3H-spiro[benzofuran-2,4'-piperidine]-1'-yl)-7-methylimidazo[1,2-c]py rimidine-8-yl)-3-fluorophenol

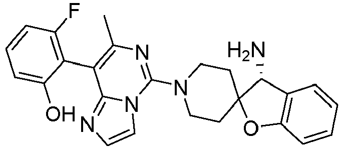

The title compound was prepared by using the same method as that of Example 48, except that the intermediate of 2, 3-dichlorobenzeneboronic acid was replaced with 2-fluoro-6-hydroxyphenylboronic acid. ¹H NMR (400 MHz, DMSO) δ 9.74(s, 1H), 7.74(s, 1H), 7.67 - 7.53 (m, 5H), 7.45 (d, *J =* 11.8 Hz, 1H), 7.25 (dd, *J* = 17.3, 8.3 Hz, 1H), 6.97 - 6.84(m, 1H), 6.83 - 6.70 (m, 1H), 3.84(dd, *J =* 50.0, 21.6 Hz, 2H), 3.57 (s, 3H), 2.20 (d, *J* = 23.4 Hz, 2H), 2.02 (dd, *J =* 25.3, 17.6 Hz, 2H), 1.24(s, 4H). ESI-MS m/z: 446.1 [M+H]⁺

### Example 76 : (R)-6-amino-2-(3-amino-3H-spiro[benzofuran-2,4'-piperidine]-1'-yl)-3-methyl-5-((4-(trif luoromethyl)pyrimidin-5-yl)thiopyrimidin-4(3H)-one

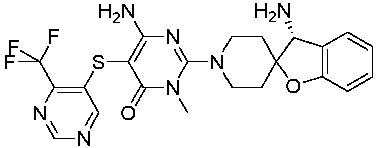

The title compound was prepared by using the same method as that of Example 53, except that the intermediate 3-bromo-2-(trifluoromethyl)pyridine was replaced with 5-bromo-4-(trifluoromethyl)pyrimidine. ¹H NMR (400 MHz, DMSO) δ 9.09 (s, 1H), 8.43 (s, 1H), 7.34(d, J = 7.0 Hz, 1H), 7.15 (t, J = 7.5 Hz, 1H), 7.01 (s, 2H), 6.91 - 6.84(m, 1H), 6.77 (d, J = 8.1 Hz, 1H), 4.43(s, 2H), 4.11 (s, 1H), 3.30 (s, 3H), 3.23 (d, J = 11.3 Hz, 4H), 1.99 - 1.66 (m, 4H). ESI-MS m/z: 506.2 [M+H]⁺

### Example 77 : (S)-5-((1H pyrazolin [3,4-b]pyridine-4-yl)thio)-6-amino-2-(1-amino-1,3-dihydrospiro[indene-2,4'-pip eridine]-1'-yl)-3-methylpyrimidin-4(3H)-one

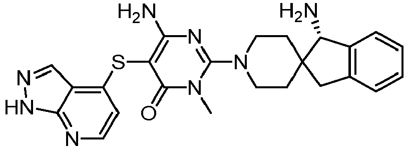

The title compound was prepared by using the same method as that in Example 1, except that 3-bromo-2-trifluoromethylpyridine was replaced with bromo-1H-pyrazoline[3,4-b]pyridine. ESI-MS m/z 475.2 [M+H]+. ¹H NMR (400 MHz, *d*6-DMSO) *δ* 13.80 - 13.50 (m, 1H), 8.28 - 8.18 (m, 1H), 8.13 - 8.06 (m, 1H), 7.41 - 7.31 (m, 1H), 7.15-7.25 (m, 3H), 6.90-6.70(s, 3H), 6.64 - 6.47 (m, 2H), 4.01 - 3.90 (m, 1H), 3.51 - 3.41 (s, 3H), 3.10 - 3.01 (m, 2H), 2.93-2.85(m, 1H), 2.60-2.69 (m, 1H), 1.95 - 1.72 (m, 2H), 1.60 - 1.42 (m, 2H), 1.27 - 1.14(m, 2H).

According to the synthesis method of Example 1 of the present disclosure, the compounds of examples 78-81 was synthesized by using different commercially available raw materials. The characterization parameters of these compounds are shown in Table 3:

**Table 3**

| Ex. | structure of compound | ESI-MS m/z [M+H]⁺ | Ex. | structure of compound | ESI-MS m/z [M+H]⁺ |
|---|---|---|---|---|---|
| 78 | 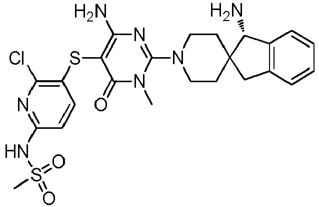 | 562.1 | 80 | 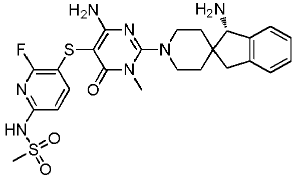 | 546.2 |
| 79 | 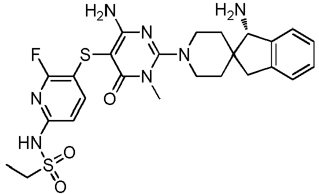 | 560.2 | 81 | 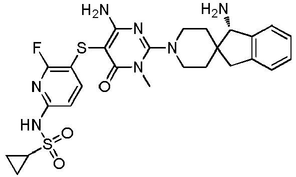 | 572.2 |

### Comparative Example 1

With reference to the method for compound 45 disclosed in WO2018/172984 (PCT/IB2018/051973), the compound represented by the following formula (Compound A) was prepared and identified by hydrogen and mass spectrometry.

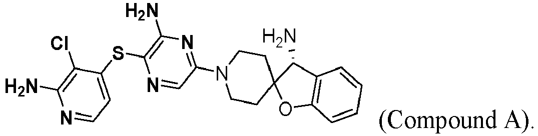

### Experimental Example 1 Cell proliferation inhibition test

### 1. Experimental materials

Test compounds: The compounds prepared in examples and comparative example of the present disclosure was tested. Each compound was formulated with DMSO into 20 mM. The concentrations of the compounds acting on NCI-H358 cells were 100 µM, 25 µM, 6.25 µM, 1.56 µM, 0.391 µM, 0.098 µM, 0.024 µM, 0.006 µM, 0.0015 µM, and 0.00038 µM successively.

Human non-small cell lung cancer cells NCI-H358 were purchased from the American Type Culture Collection (ATCC).

Reagents: The CCK-8 proliferation inhibition detection kit was purchased from Jiangsu KeyGEN BioTECH Co., Ltd. The CKX41 inverted microscope was purchased from Olympus, Japan. The multi-mode microplate reader was purchased from Molecular Devices, USA. The cell culture incubator was purchased from Thermo, USA.

### 2. Experimental methods

### 2.1 Cell culture

Cell recovery: ANCI-H358 cell cryogenic vial was taken out from a liquid nitrogen tank, placed in water bath at 37°C, and shaked gently to allow it to thaw. The thawed cryogenic vial was disinfected with alcohol cotton, and opened to aspirate the cell fluid into a centrifuge tube. To the centrifuge tube, 1mL of serum-containing complete medium was added and well-mixed. The centrifuge tube was placed into a centrifuge, for centrifugation at 1000 rpm for 5 min. Then the supernatant was discarded, and complete medium was added to completely dissipate and resuspend the cells through repeated pipetting. The cells were seeded in petri dishes at appropriate concentrations and cultured in a CO₂ incubator at 37°C, with 5% CO₂, 95% humidified air.

Cell passage: After the cells grew to about 80-90% confluence, the original culture medium (1640 culture medium + 10% FBS + 1% penicillin-streptomycin + 1 mM sodium pyruvate) was discarded, and 1 mL of PBS was added to wash away the residual medium and then discarded. 1mL of trypsin digestion solution was added to digest for 1-2 min. When it was observed under the microscope that the pseudopodia of the cells shrinked and became round but the cells did not fall off into pieces, the trypsin was discarded and the digestion was terminated with 1-2mL of complete medium. The cell suspension was collected by gently pipetring, and centrifuged at 1000 rpm for 5 min. After discarding the supernatant, the cells was resuspended in complete medium, seeded in petri dishes at the required density, and then cultured in a CO₂ incubator at 37°C, with 5% CO₂, 95% humidified air. Depending on cell growth, the culture medium was changed or passaged every 2-3 days.

### 2.2 Experimental steps:

After NCI-H358 cell passage, the cells were resuspended in fresh culture medium (1640 culture medium + 3% FBS + 1% penicillin-streptomycin + 1 mM sodium pyruvate), and counted. The cells were seeded into a 96-well culture plate at a density of 1.5×10⁴ cells/mL, with each well added 100 µL thereto (ie, 1.5×10³ cells/well). After 24h, 100 µL of fresh culture medium containing different drug concentrations (2×) was added to the old culture medium. The final concentrations of the compounds were 100 µM, 25 µM, 6.25 µM, 1.56 µM, 0.391 µM, 0.098 µM, 0.024 µM, 0.006 µM, 0.0015 µM, and 0.00038 µM, with each concentration group set two replicate wells. The cells were culculated in the incubator again for 168 h. The culture medium in wells was discarded as thoroughly as possible, and 100 µL of culture medium containing CCK-8 (CCK-8 : culture medium= 1 : 10) was added. After a certain time of culture in the incubator, the 96-well plate was taken out of the incubator, and allowed to equilibrate at room temperature for 5 min. The absorbance (OD value) at 450 nm was detected in a multi-mode plate reader, and the cell proliferationin hibition rate was calculated. The calculation formula was Inhibition (%) = 100-(OD experiment _{well-}OD _{blank well}) / (ODₛₒₗᵥₑₙₜ control _{well}-OD _{blank well}) × 100. According to different drug concentrations and their corresponding inhibition rates, IC₅₀ curves were plotted using GraghPad 5.0 software, to analyze the data and to obtain final IC₅₀. The experimental results are set forth in Table 4.

**Table 4**

| Ex. | NIH358 cell proliferation inhibition IC₅₀ (nM) | Ex. | NIH358 cell proliferation inhibition IC₅₀ (nM) | Ex. | NIH358 cell proliferation inhibition IC₅₀ (nM) |
|---|---|---|---|---|---|
| 1 | 1.6 | 45 | 3.9 | 51 | 6.0 |
| 4 | 5.6 | 46 | 8.5 | 53 | 4.9 |
| 6 | 2.8 | 47 | 16.7 | compound A | 18.3 |
| 8 | 3.3 | 48 | 12 | | |
| 9 | 1.5 | 49 | 13.3 | | |

As can be seen from the above experiments, the compounds of the present disclosure exhibited good inhibitory activity on NCI-H358 cells, and are very promising to be a therapeutic agent for non-small cell lung cancer.

### Experimental example 2 Pharmacokinetic experiment

### 1. Experimental materials

Compounds: The compounds of the present disclosure prepared in the above examples were tested. The drug vehicle was Captisol/50 mM sodium acetate with pH 4.6 (10%/90%, w/v%). Oral drugs were formulated into a 0.5 mg/mL clear solution. Intravenous drug were formulated into a 0.1 mg/mL clear solution.

Animals: Male BALB/c mice, SPF grade, were purchased from Shanghai SIPPR-Bk Lab Animal Co., Ltd, with a wight of 18-20g, and adapted for 2-3 days before experiments.

Instrument: API4500-type Triple Quadrupole LC/MS System, from AB, United States, equipped with electrospray ion source (ESI) and dual pumps LC-30AD; SIL-30AC autosampler; CTO-30AC column oven; DGU-20A3R degasser; AnalystQSA01.01 chromatography workstation; Milli-Q ultrapure water device (Millipore Inc); QilinbeierVortex-5 shaker; HITACHI CF16RX II desktop high-speed freezing centrifuge.

### 2. Experimental method

(1) There were 3 mice per group. The compounds of the examples of the present disclosure were given by intragastric administration (I.G.) at10mg/kg, or the compounds of the examples of the present disclosure were given by intravenous (IV) administration at 1mg/kg.
(2) At 5 min, 15 min, 30 min, 1h, 2h, 6h, 10h, and 24 h after the intragastric administration or intravenous administration, blood was collected from the orbital venous plexus in heparinized EP tubes (0.6 ML), and centrifuged at 8000rpm/min for 5min. The upper plasma was taken, frozen at -20 °C and analyzed by LC-MS/MS.
(3) Plasma drug concentration-time curves were poltted according to blood drug concentration data obtained from the above-mentioned step. Pharmacokinetic parameters were calculated by using WinNonlin software. The experimental results are shown in Table 5.

**Table 5**

| **routes of administration** | **dose (mg/kg)** | **index** | **Example 45** | **Example 48** | **Example 53** |
|---|---|---|---|---|---|
| I.G. | 10 | T_{1/2} (h) | 7.3 | 15.6 | 3.0 |
| | | Cₘₐₓ(ng/mL) | 1586.7 | 1893.3 | 1393.3 |
| | | AUC₍₀₋ₜ₎ (h×ng/mL) | 19360.2 | 24157.4 | 6522.8 |
| | | AUC_{(0-∞)} (h×ng/mL) | 21634.7 | 36969.5 | 7406.1 |
| | | F (%) | 78.3 | 81.2 | 74.3 |

The pharmacokinetics of Compound A was determined using the method of Experimental Example 2. The experimental results showed that the bioavailability (F) of Compound A was 51.2%, which was significantly lower than that of the compounds of the present disclosure.

The experimental results show that the compounds of the present disclosure have good half-life T_{1/2}, area under the curve AUC and bioavailability F, as well as good oral absorption and exposure, so they are suitable for finished medicine.

Although the present disclosure has been described in detail above, it will be understood by these skilled in the art that various modifications and variations can be made in the present invention without departing from the spirit and scope of the invention. The scope of the present invention is not limited to the detailed description above, but should be attributed to the claims.

## Claims

1. A compound represented by general formula (I), or an isomer, a pharmaceutically acceptable salt, a solvate, a crystal or a prodrug thereof, 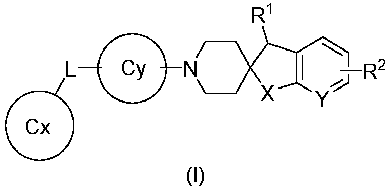 wherein,
L is absent or selected from -NH-, -S- and -O-;
X is selected from CH₂, NH, O and S;
Y is selected from CH and N;
R¹ and R² are independently selected from hydrogen, halogen, hydroxyl, alkyl, haloalkyl, hydroxyalkyl, alkoxy, haloalkoxy, hydroxyalkoxy, nitro, carboxyl, cyano, amino, monoalkylamino, alkylacylamino, alkylacyl, aminoacyl, alkylaminoacyl, bisalkylamino and cycloalkyl;
ring Cx is selected from aryl, heteroaryl, cycloalkyl and heterocyclyl, which are optionally substituted with one or more R³, wherein R³ is selected from halogen, hydroxy, alkyl, haloalkyl, hydroxyalkyl, alkoxy, haloalkoxy, hydroxyalkoxy, nitro, carboxyl, cyano, amino, monoalkylamino, alkylacylamino, alkylacyl, alkylsulfonyl, aminoacyl, alkylaminoacyl, bisalkylamino, alkenyl, alkynyl, cycloalkyl, heterocyclyl, substituted heterocyclyl, cycloalkylamino, cycloalkylacyl, heterocyclylacyl, substituted heterocyclylacyl, cycloalkylaminoacyl, cycloalkylacylamino, alkylsulfonyl, alkylaminosulfonyl, alkylsulfonamido, cycloalkylsulfonamido, bisalkylphosphoryl and oxo group; and
ring Cy is selected from 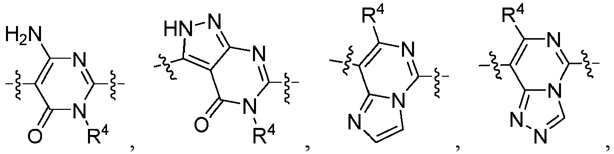 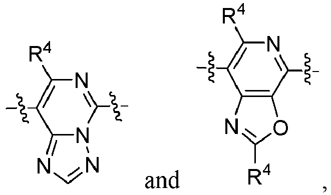 wherein R⁴ is selected from hydrogen, halogen, hydroxy, alkyl, haloalkyl, hydroxyalkyl, alkoxy, haloalkoxy, hydroxyalkoxy, nitro, carboxyl, cyano, amino, monoalkylamino, alkylacylamino, alkylacyl, aminoacyl, alkylaminoacyl, bisalkylamino and cycloalkyl.

2. The compound or an isomer, a pharmaceutically acceptable salt, a solvate, a crystal or a prodrug thereof according to claim 1, wherein Cx is selected from C₆₋₁₂ aryl, C₅₋₁₂ heteroaryl, C₃₋₆ cycloalkyl and C₃₋₆ heterocyclyl, which are substituted with one or more R², wherein R² is selected from halogen, hydroxy, alkyl, haloalkyl, hydroxyalkyl, alkoxy, haloalkoxy, hydroxyalkoxy, nitro, carboxyl, cyano, amino, monoalkylamino, alkylacylamino, alkylacyl, alkylsulfonyl, aminoacyl, alkylaminoacyl, bisalkylamino, alkenyl, alkynyl, cycloalkyl, heterocyclyl, substituted heterocyclyl, cycloalkylamino, cycloalkylacyl, heterocyclylacyl, substituted heterocyclylacyl, cycloalkylaminoacyl, cycloalkylacylamino, alkylsulfonyl, alkylsulfonamido, bisalkylphosphoryl and oxo group.

3. The compound or an isomer, a pharmaceutically acceptable salt, a solvate, a crystal or a prodrug thereof according to claim 1 or 2, wherein
R³ is selected from halogen, hydroxy, C₁₋₆ alkyl, halogenated C₁₋₆ alkyl, hydroxy C₁₋₆ alkyl, C₁₋₆ alkoxy, halogenated C₁₋₆ alkoxy, hydroxy C₁₋₆ alkoxyl, nitro, carboxyl, cyano, amino, mono-C₁₋₆ alkylamino, C₁₋₆ alkylacylamino, C₁₋₆ alkylacyl, C₁₋₆ alkylsulfonyl, aminoacyl, C₁₋₆ alkylaminoacyl, bis-C₁₋₆ alkylamino, C₂₋₁₀ alkenyl, C₂₋₁₀ alkynyl, C₃₋₈ cycloalkyl, C₃₋₈ heterocyclyl, substituted C₃₋₈ heterocyclyl, C₃₋₈ cycloalkylamino, C₃₋₈ cycloalkylacyl, C₃₋₈ heterocyclylacyl, substituted C₃₋₈ heterocyclylacyl, C₃₋₈ cycloalkylaminoacyl, C₃₋₈ cycloalkylacylamino, C₁₋₆ alkylsulfonyl, C₁₋₆ alkylaminosulfonyl, C₁₋₆ alkylsulfonamido, C₃₋₈ cycloalkylsulfonamido, bisC₁₋₆ alkylphosphoryl and oxo group.

4. The compound or an isomer, a pharmaceutically acceptable salt, a solvate, a crystal or a prodrug thereof according to any one of claims 1-3, wherein general formula (I) has a structure represented by general formula (Ia), 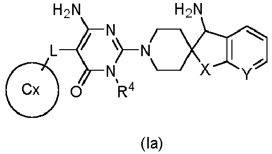 wherein Cx, L, X, Y and R⁴ have the definitions set forth in claims 1-3.

5. The compound or an isomer, a pharmaceutically acceptable salt, a solvate, a crystal or a prodrug thereof according to any one of claims 1-3, wherein general formula (I) has a structure represented by general formula (Ib), 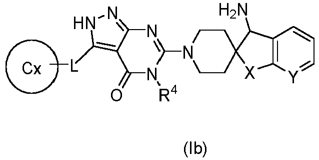 wherein Cx, L, X, Y and R⁴ have the definitions set forth in claims 1-3.

6. The compound or an isomer, a pharmaceutically acceptable salt, a solvate, a crystal or a prodrug thereof according to any one of claims 1-3, wherein general formula (I) has a structure represented by general formula (Ic), 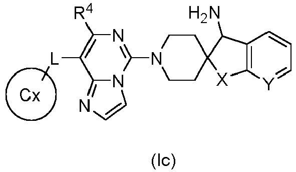 wherein Cx, L, X, Y and R⁴ have the definitions set forth in claims 1-3.

7. The compound or an isomer, a pharmaceutically acceptable salt, a solvate, a crystal or a prodrug thereof according to claim 6, wherein Cx is selected from 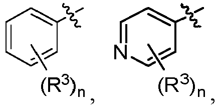 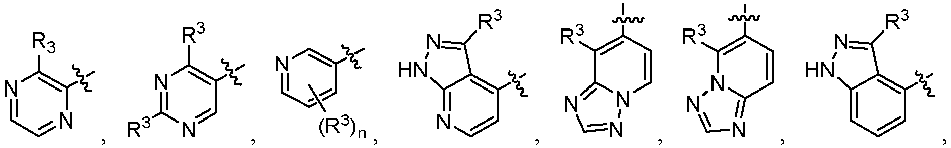 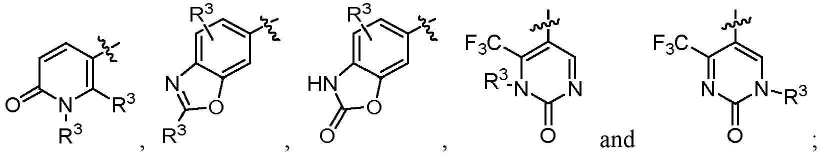 wherein n is 1, 2 or 3, and R³ has the definition set forth in claim 3.

8. The compound or an isomer, a pharmaceutically acceptable salt, a solvate, a crystal or a prodrug thereof according to claim 1, wherein the compound is selected from the group consisting of:

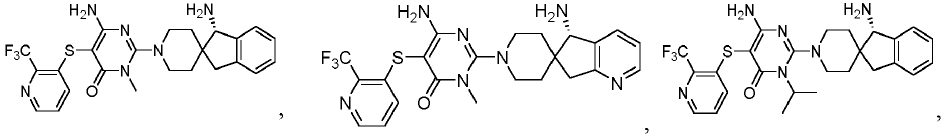

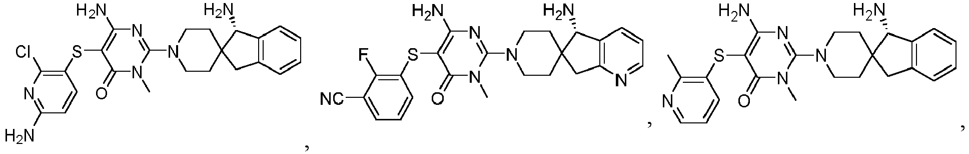

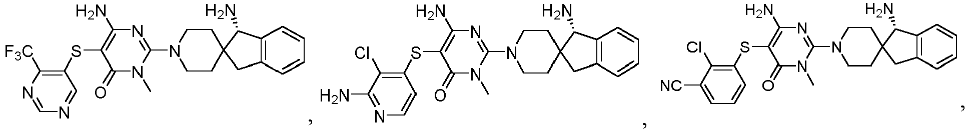

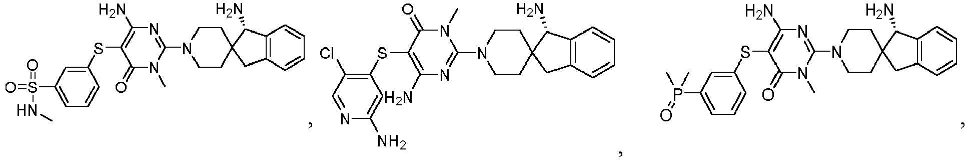

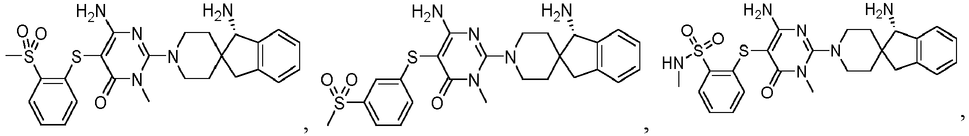

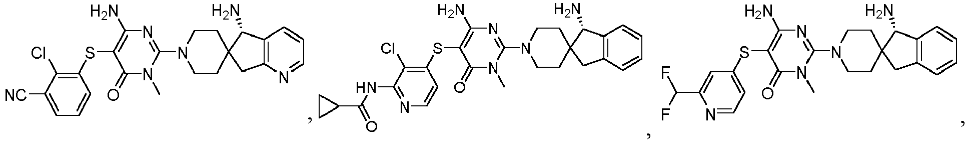

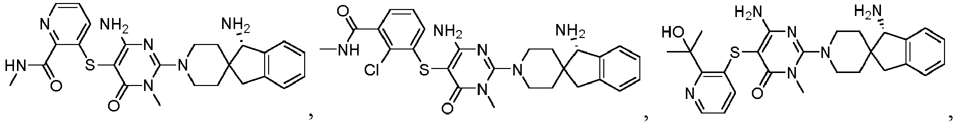

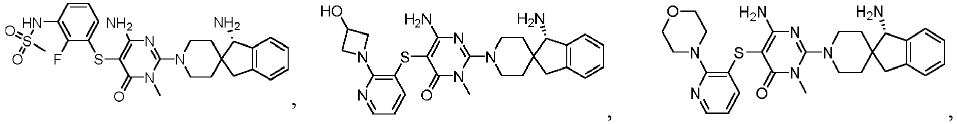

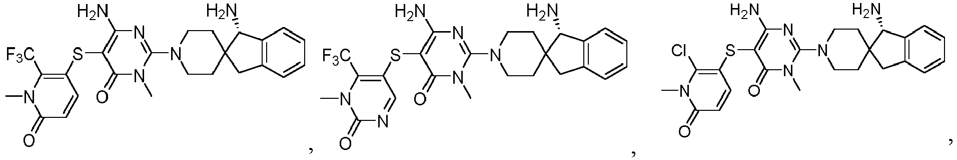

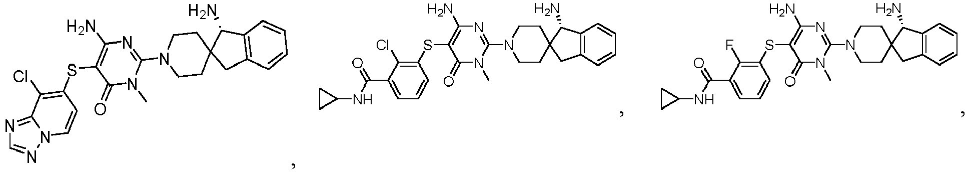

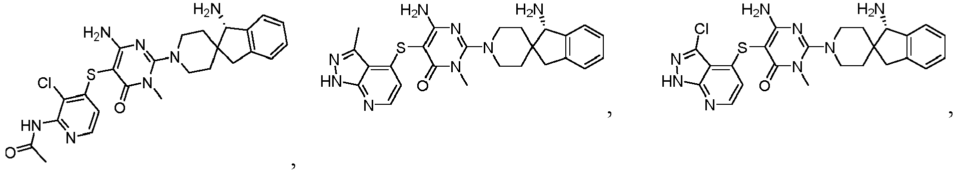

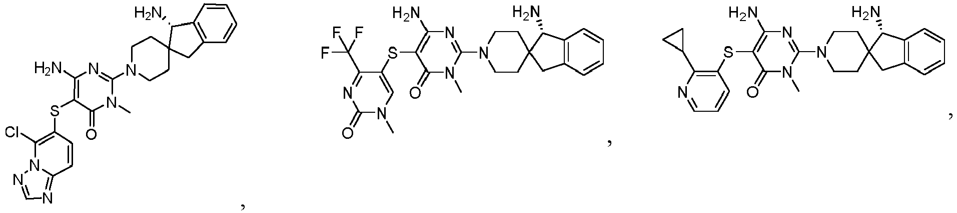

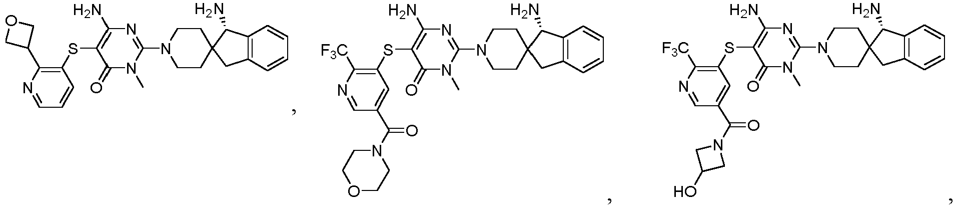

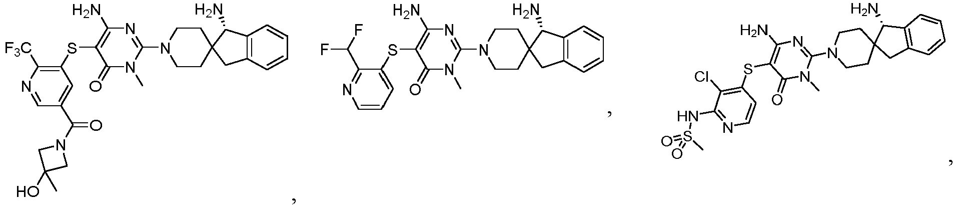

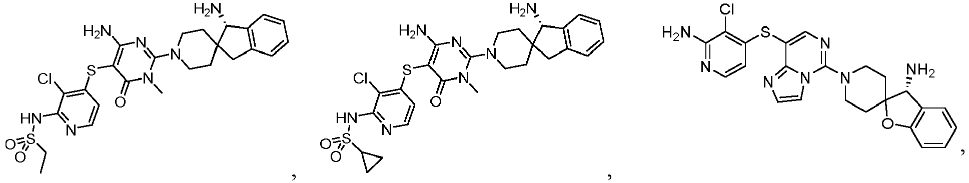

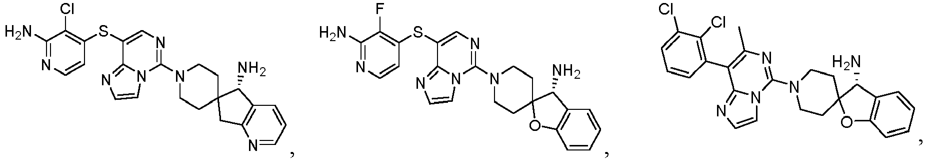

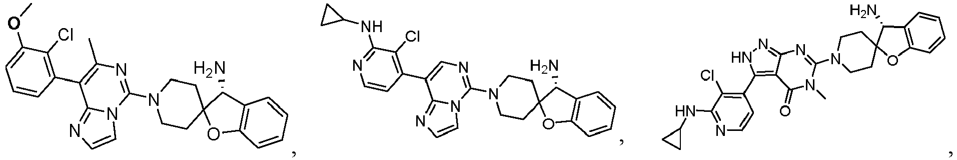

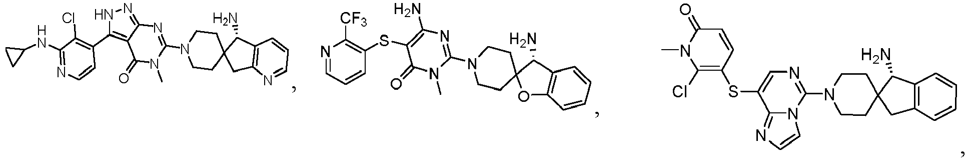

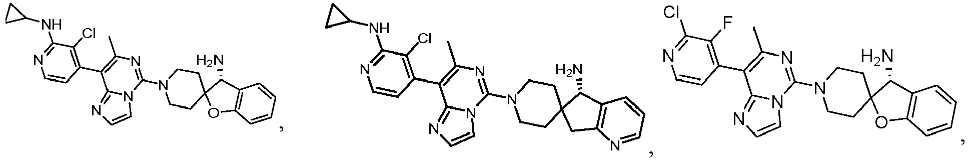

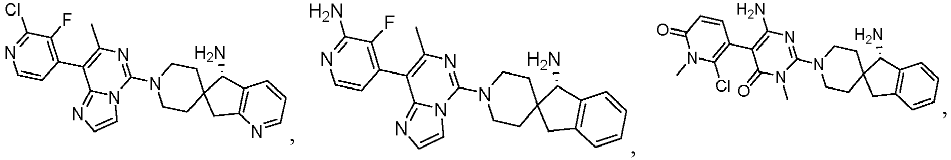

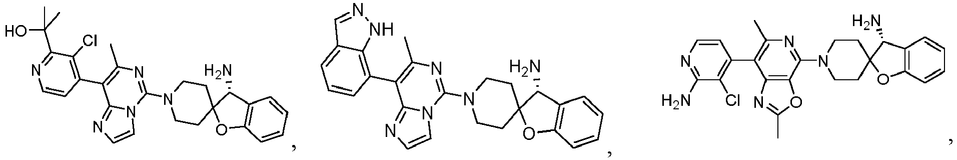

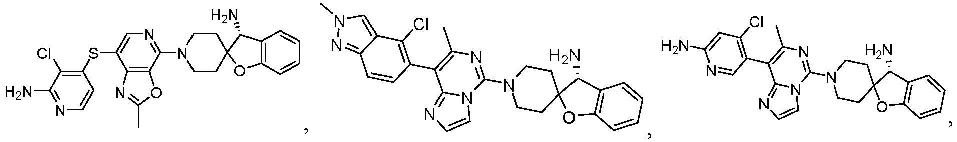

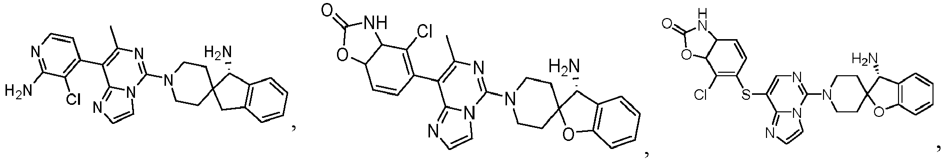

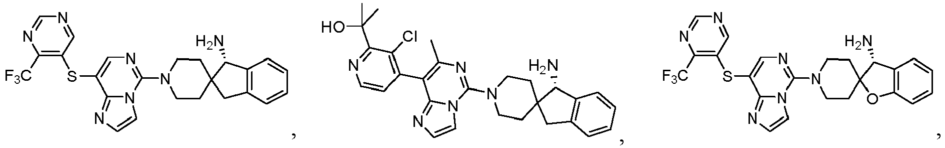

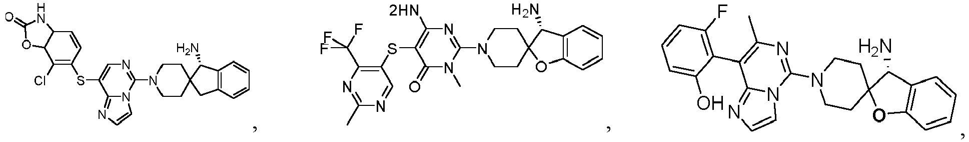

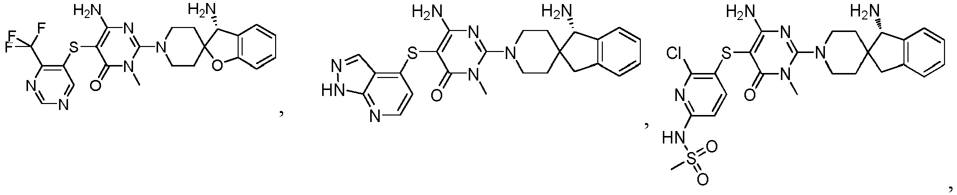

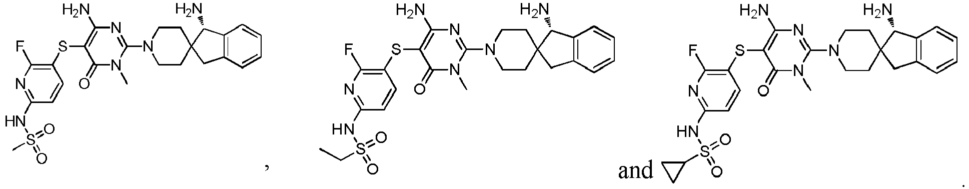

9. A pharmaceutical composition comprising the compound or an isomer, a pharmaceutically acceptable salt, a solvate, a crystal or a prodrug thereof according to any one of claims 1-8, and a pharmaceutically acceptable carrier.

10. Use of the compound or an isomer, a pharmaceutically acceptable salt, a solvate, a crystal or a prodrug thereof according to any one of claims 1-8, or the pharmaceutical composition according to claim 9 in the manufacture of a medicament for treating a SHP2-mediated disease.
